(19) Europäisches Patentamt
European Patent Office
Office européen des brevets

(11) EP 3 317 320 B1

(12) FASCICULE DE BREVET EUROPEEN

(45) Date de publication et mention de la délivrance du brevet:
**08.06.2022 Bulletin 2022/23**

(21) Numéro de dépôt: **16750916.5**

(22) Date de dépôt: **01.07.2016**

(51) Classification Internationale des Brevets (IPC):
***C08G 65/00*** *(2006.01)* ***C08G 65/333*** *(2006.01)*
***C08G 65/337*** *(2006.01)* ***C08L 71/00*** *(2006.01)*

(52) Classification Coopérative des Brevets (CPC):
(C-Sets disponibles)
**C08G 65/007; C08G 65/333; C08G 65/33365; C08G 65/337; C08K 5/0025; C08K 5/28; C08L 71/00;** C08G 2650/20; C08G 2650/60 (Cont.)

(86) Numéro de dépôt international:
**PCT/FR2016/051676**

(87) Numéro de publication internationale:
**WO 2017/001806 (05.01.2017 Gazette 2017/01)**

(54) **MATÉRIAUX RÉTICULÉS THERMODURCISSABLES À BASSE TG ET THERMOSTABLES À BASE D'UNITÉS FLUOROÉTHERS**

THERMOSTABILE FLUORETHER HITZEHÄRTBAREN VERNETZTEN MATERIALIEN MIT NIEDRIGER TG

FLUOROETHER BASED CURED THERMOSETTING MATERIALS OF LOW TG AND THERMAL STABILITY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.07.2015 FR 1556208**

(43) Date de publication de la demande:
**09.05.2018 Bulletin 2018/19**

(73) Titulaires:
- **Safran Electrical & Power**
  **31702 Blagnac Cedex (FR)**
- **Universite De Montpellier (Universite Montpellier 2 Sciences Et Techniques)**
  **34090 Montpellier (FR)**
- **Centre National de la Recherche Scientifique (CNRS)**
  **75016 Paris (FR)**

(72) Inventeurs:
- **CHARLAS, Mathieu**
  **31700 Blagnac (FR)**
- **MARTINEAU, Donatien Henri Edouard**
  **31700 Blagnac (FR)**
- **LOPEZ, Gérald Pierre Maurice**
  **34400 Lunel-viel (FR)**
- **HABAS, Jean-Pierre**
  **34080 Montpellier (FR)**
- **AMEDURI, Bruno**
  **34000 Montpellier (FR)**

(74) Mandataire: **Regimbeau**
**20, rue de Chazelles**
**75847 Paris Cedex 17 (FR)**

(56) Documents cités:
**WO-A1-2010/115855**
**WO-A2-2014/055406**
**US-A1- 2010 324 234**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 317 320 B1

- **YING-WEI YANG ET AL: ""Clicked" fluoropolymer elastomers as robust materials for potential microfluidic device applications", JOURNAL OF MATERIALS CHEMISTRY, vol. 22, no. 3, 17 novembre 2011 (2011-11-17), pages 1100-1106, XP055267631, GB ISSN: 0959-9428, DOI: 10.1039/C1JM14131G & Ying-Wei Yang ET AL: "Supporting Information for "Clicked" fluoropolymer elastomers as robust materials for potential microfluidic device applications", Journal of Materials Chemistry This journal is The Royal Society of Chemistry, 17 novembre 2011 (2011-11-17), XP055267680, Extrait de l'Internet: URL:http://www.rsc.org/suppdata/jm/c1/c1jm14131g/c1jm14131g.pdf [extrait le 2016-04-21]**
- **GÉRALD LOPEZ ET AL: "A Versatile Strategy to Synthesize Perfluoropolyether-Based Thermoplastic Fluoropolymers by Alkyne-Azide Step-Growth Polymerization", MACROMOLECULAR RAPID COMMUNICATIONS, vol. 37, no. 8, 23 février 2016 (2016-02-23), pages 711-717, XP055308095, ISSN: 1022-1336, DOI: 10.1002/marc.201500658**


(52) Classification Coopérative des Brevets (CPC): (Cont.)

C-Sets
**C08L 71/00**

## Description

### DOMAINE DE L'INVENTION

La présente invention concerne le domaine des matériaux thermodurcissables et/ou élastomères à base de polyéthers fluorés, leurs procédés de préparation ainsi que leurs utilisations.

### ETAT DE LA TECHNIQUE

De nombreuses applications relevant du secteur du génie électrique ou électronique nécessitent des matériaux capables de présenter une très grande flexibilité mécanique sur un large domaine de températures dans un environnement chimique agressif. Ces mêmes matériaux ont vocation à être déclinés en tant qu'encapsulant ou surmoulant dans des montages de microélectronique à destination du secteur des transports. Ils doivent présenter de bonnes performances d'isolation électrique et de préférence une densité contenue (typiquement une densité inférieure ou égale à 3).

L'exemple le plus concret décrivant l'intérêt de l'encapsulation est celui présenté par la technologie des modules de puissance qui connaît un essor important, notamment dans le secteur aéronautique en particulier au travers des programmes de recherche visant à définir un avion « plus électrique », mais également dans le secteur automobile, notamment pour des véhicules à moteurs électrique, hybride ou à pile à combustible. Or, les matériaux actuellement utilisés pour ces applications (silicone, polyuréthane, époxy...) ne sont pas suffisamment résistants à haute température (typiquement supérieure à 250 °C, voire supérieure à 300 °C), notamment en milieu sévère (humidité saturée, vapeur d'hydrocarbure ou de phosphate d'ester).

Une solution proposée dans l'art antérieur consiste alors à substituer les matériaux de type silicone, polyuréthane ou époxy par des matériaux à base de polyéthers fluorés présentant une basse température de transition vitreuse (Tg).

On peut notamment citer la demande de brevet WO 2014/055406, qui fait appel à la réaction d'un premier composé (co-agent) fonctionnalisé par trois groupes azotures ($N_3$), et d'un deuxième composé de type bis-propargyle ou bis-nitrile, et éventuellement d'un troisième composé fonctionnalisé par au moins deux groupes azotures ($N_3$) synthétisé à partir d'un polyisocyanate en réaction avec le 2-azidoéthanol. La dite réaction est qualifiée de réticulation car elle conduit à la production d'un réseau polymère tri-dimensionnel. Le seul composé bis-propargyle exemplifié comporte des groupes terminaux $-C(=O)NH-CH_2-C\equiv CH$, i.e. comprenant un groupe mono-propargylamide, peu résistant chimiquement, en particulier en conditions humides. Ainsi, l'introduction de ces motifs amide dans la structure des polymères finaux exclut toute utilisation en milieu sévère humide, notamment à une température supérieure à 250 °C.

Yang et al. (Journal of Materials Chemistry, vol. 22, n°3, Novembre 2011) divulgue des élastomères obtenus par une réaction de chimie click entre un oligomère a,ω-bis(propargylique) fluoré de formule (I) :

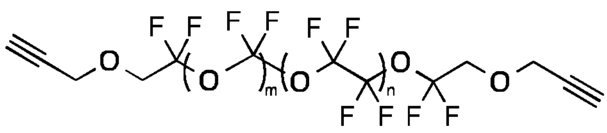

(I)

et l'agent de réticulation suivant :

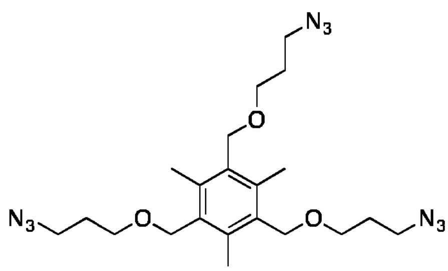

.

Cependant, ces matériaux présentent une température de transition vitreuse de -10°C.

Le document WO 2010/115855 ne divulgue pas de structure générale particulière pour le composé alcyne utilisé (voir notamment les paragraphes [0032] et [0033]). Il apparaît d'ailleurs à la lecture des paragraphes [0037]-[0041] (définition du substituant $R_H$) que l'alcyne utilisé est un mono-alcyne et non un diyine. Ce point est confirmé par les exemples 1 et 2 (étape 3), dans lesquels, seul le phénylacétylène est utilisé.

Dans le document US 2010/324234, le diyine utilisé est soit un fluoroéther comprenant deux groupes acétylènes (voir paragraphe [0014]), soit un fluoroéther aromatique. Dans les deux cas, le diyine est différent de celui utilisé dans la présente invention.

Il existe donc un besoin pour des matériaux capables de combiner propriétés d'isolation électrique, souplesse mécanique sur un large domaine de température, allant typiquement de -100 °C à +300 °C - possédant notamment au moins une valeur température de transition vitreuse inférieure ou égale à -70 °C - et résistance chimique accrue, notamment en milieu sévère et/ou humide.

## RESUME DE L'INVENTION

Le demandeur a résolu ce problème technique grâce à des matériaux comprenant le produit de la réaction de chimie click entre :

i) un oligomère fluoré a,ω-bis(propargylique) comprenant une chaîne fluoroéther dépourvue de groupement amide ;
ii) un agent de réticulation comprenant au moins trois groupes azotures -N3 (de préférence facilement synthétisable); et
iii) optionnellement un oligomère fluoré comprenant deux groupes terminaux azotures $-N_3$ (encore appelé oligomère fluoré a,ω-bis(azidé)).

Les matériaux selon l'invention sont donc des matériaux réticulés, résultant de la polymérisation d'oligomères bifonctionnels en présence d'un agent réticulant.

La flexibilité (caractère élastique) et la température de transition vitreuse des polymères selon l'invention peuvent être modulées à façon en fonction de deux paramètres :

- la proportion relative en agent de réticulation et en oligomère fluoré α,ω-bis(azidé), et
- la nature et la longueur de la chaîne fluorée des oligomères fluorés α,ω-bis(propargyliques) (i) et éventuellement α,ω-bis(azidés) (iii).

En d'autres termes, en fonction de ces deux paramètres, les polymères selon l'invention relèveront soit de la catégorie des polymères thermodurcissables, soit de celle des élastomères. Ainsi, les polymères selon l'invention peuvent présenter une température de transition vitreuse de l'ordre de -100 °C, en particulier comprise entre-150 °C et -30 °C. En outre, en fonction des paramètres rappelés ci-dessus, les polymères selon l'invention sont soit amorphes, soit cristallins.

Cette double polyvalence autorise le dimensionnement d'un catalogue de polymères capables de satisfaire des cahiers des charges variés tout en limitant les coûts de développement.

Avantageusement, les proportions des différents réactifs sont telles que le nombre total de groupements azotures apportés indifféremment par la chaîne fluorée de l'oligomère α,ω-bis(azidé) ou par l'agent de réticulation est égal au nombre de groupements propargyliques apportés par l'oligomère α,ω-bis(propargylique) fluoré.

En outre, indépendamment de ces caractéristiques, les polymères selon l'invention présentent une bonne inertie chimique du fait de la présence de groupements fluorés, et de l'absence de groupements amide dans la chaîne polymère.

Ainsi, un objet de la présente invention concerne une composition réticulable comprenant :

i) un oligomère α,ω-bis(propargylique) fluoré de formule (I) :

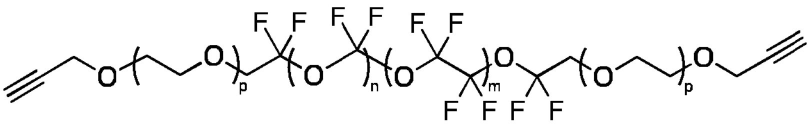

(I)

dans laquelle m est compris entre 1 et 100, par exemple entre 1 et 93,

n est compris entre 2 et 150, par exemple entre 1 et 128, et
p est compris entre 0 et 2, par exemple 0 ou 1.75,
n, m et p étant choisis de manière que l'oligomère α,ω-bis(propargylique) fluoré de formule (I) soit de masse molaire moyenne en nombre $M_n$ comprise entre 400 et 25000;

ii) un agent de réticulation comprenant trois groupes azotures $-N_3$; et
iii) optionnellement un oligomère fluoré comprenant deux groupes terminaux azotures $-N_3$, ou oligomère $\alpha,\omega$-bis(azidé) fluoré.

Un autre objet de l'invention vise un matériau comprenant le produit de la réaction par chimie click entre : i) un oligomère $\alpha,\omega$-bis(propargylique) fluoré de formule (I) :

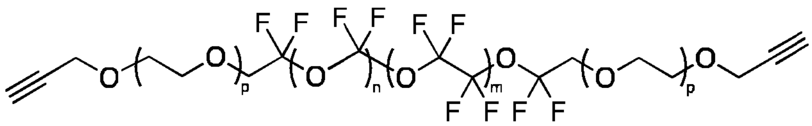

(I)

dans laquelle m est compris entre 1 et 100, par exemple entre 1 et 93,

n est compris entre 2 et 150, par exemple entre 1 et 128, et
p est compris entre 0 et 2, par exemple 0 ou 1.75,
n, m et p étant choisis de manière que l'oligomère $\alpha,\omega$-bis(propargylique) fluoré de formule (I) soit de masse molaire moyenne en nombre $M_n$ comprise entre 400 et 25000;

ii) un agent de réticulation comprenant au moins trois groupes azotures $-N_3$; et
iii) optionnellement un oligomère fluoré comprenant deux groupes terminaux azotures $-N_3$, ou oligomère $\alpha,\omega$-bis(azidé) fluoré.

Un autre objet de l'invention concerne un procédé de préparation du matériau selon l'invention.
Un autre objet de l'invention concerne l'utilisation du matériau selon l'invention en tant qu'isolant électrique.

**DEFINITIONS**

Sauf indication contraire, dans la présente description, les indices m, n, p, q, r, s et i sont des nombres réels positifs qui ne sont pas forcément entiers mais représentatifs de la structure moléculaire moyenne (valeurs statistiques).
Par « matériaux », on entend au sens de la présente invention un matériau réticulé, résultant de la polymérisation d'oligomères bifonctionnels en présence d'un agent réticulant. En d'autres termes, le matériau selon l'invention comprend ou est constitué du produit de la réaction par chimie « click » de la composition réticulable selon l'invention. Les matériaux selon la présente invention peuvent être assimilés à des matériaux thermodurcissables ou à des élastomères, notamment en fonction de la densité de maillage de leur réseau en 3 dimensions
Par « réaction de chimie click » on entend au sens de la présente invention une réaction de cycloaddition 1,3-dipolaire (ou réaction de Huisgen) entre un composé comprenant un groupe azotures et un composé comprenant un groupe alcyne, de préférence en présence d'un catalyseur au cuivre ou au ruthénium, pour fournir un groupe 1,2,3-triazole selon le schéma réactionnel suivant :

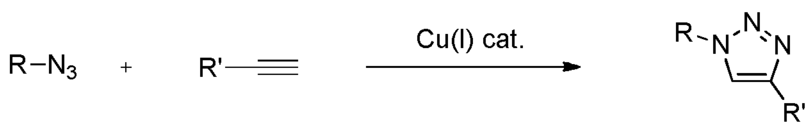


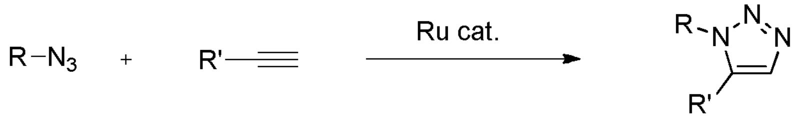


Lorsqu'un catalyseur au cuivre (sel de cuivre (I) ou sel de cuivre (II)) est utilisé, la réaction de chimie click mène majoritairement (voire exclusivement en fonction des substrats) à un 1,2,3-triazole 1,4-disubstitué.
Lorsqu'un catalyseur au ruthénium est utilisé, la réaction de chimie click mène majoritairement (voire exclusivement en fonction des substrats) à un 1,2,3-triazole 1,5-disubstitué.
Par « polymère thermodurcissable », on entend au sens de la présente invention un polymère ne pouvant être

mis en oeuvre qu'une seule fois et qui devient infusible et insoluble après polymérisation d'une composition généralement liquide à l'état initial et mono- ou multi-composants. Ladite polymérisation inclut également une « réticulation », reposant sur la production d'un réseau polymère tridimensionnel. Une fois durci, le polymère ne peut revenir à l'état liquide.

Par « élastomère », on entend au sens de la présente invention un polymère présentant des propriétés « élastiques », obtenues après réticulation. Il supporte de très grandes déformations avant rupture. Le terme de caoutchouc est un synonyme usuel d'élastomère.

La « température de transition vitreuse » ou « $T_g$ » est un paramètre bien connu de l'homme du métier. Il s'agit de la température critique à partir de laquelle la matière passe d'un état caoutchoutique à un état vitreux, solide (rigide) (et vice-versa). Elle peut être mesurée par calorimétrie différentielle à balayage (DSC) préférentiellement au travers d'une rampe en température élevée avantageusement supérieure à 5 °C/min. D'autres techniques connues de l'homme du métier permettent d'enregistrer une discontinuité de propriété physique lors du passage de la transition vitreuse. On citera par exemple, la rhéométrie dynamique, les mesures de spectroscopie diélectrique ou encore la dilatométrie.

La température de décomposition associée à une valeur de 10% de perte de poids $(T_d^{10\%})$ est également un paramètre bien connu de l'homme du métier, permettant de mesurer la stabilité thermique d'un polymère. Elle est notamment mesurée par analyse thermogravimétrique, notamment dans des conditions oxydantes (sous air) ou en milieu inerte (azote gazeux). Le choix de l'atmosphère d'analyse est conditionné par la volonté de discriminer les phénomènes de dégradation purement thermiques (atmosphère azote gazeux) de ceux relevant de processus thermo-oxydatif (atmosphère air)

Selon la présente invention, la masse molaire moyenne en nombre ($M_n$) est mesurée par spectroscopie RMN $^{19}F$. Ainsi, la masse molaire moyenne en nombre est de préférence mesurée selon la méthode décrite dans Karis et al (J. Fluorine Chem. 2002, 118, 81-94). Un exemple de mise en oeuvre de cette méthode est notamment décrit sur la figure 1 : le nombre m est calculé comme étant égal au rapport de 2 fois l'intégration du pic O-$CF_2$-O sur l'intégration du pic $CF_2CH_2OH$, tandis que le nombre n est calculé comme étant égal au rapport de 2 fois l'intégration du pic O-$CF_2CF_2$-O sur l'intégration du pic $CF_2CH_2OH$, l'intégration étant entendue comme l'aire mesurée sous le pic correspondant dans le spectre RMN $^{19}F$. La détermination de n et m permet de calculer ensuite la masse molaire moyenne en nombre ($M_n$).

Par « groupe amide » on entend au sens de la présente invention un groupe--R-C(=O)-NH- ou un groupe -NH-C(=O)-R-(C=O)NH-R', R et R' étant indépendamment choisis par exemple parmi un groupe alkyle, un groupe aryle ou hétéroaryle, ou une chaîne mixte fluor-carbone.

Par « milieu sévère », on entend au sens de la présente invention un milieu exposé (voire saturé) en vapeur d'hydrocarbure ou de phosphate d'ester, à une température typiquement comprise entre 200 et 350 °C, en particulier entre 250 °C et 350 °C. Un « milieu » sévère » peut également être un milieu présentant une humidité relative comprise entre 50% et 100%, à une température inférieure ou égale à 120 °C à pression atmosphérique normale (1 bar) voire supérieure (de préférence néanmoins inférieure ou égale à 20 bars).

Par « alkyle en $C_1$-$C_6$ » on entend au sens de la présente invention une chaîne hydrocarbonée saturée à 6 carbones, linéaire ou ramifiée. A titre d'exemple d'alkyle en $C_1$-$C_6$ ont peut citer notamment le méthyle, l'éthyle, le propyle, le n-butyle, le s-butyle, le tert-butyle, le pentyle, l'isopentyle, le n-hexyle. Au sens de la présente invention, un alkyle en $C_1$-$C_6$ englobe également les alklyles cycliques en $C_3$-$C_6$, tels que le cyclopropyle, le cyclobutyle, le cyclopentyle et le cyclohexyle. De préférence, l'alkyle en $C_1$-$C_6$ est un alkyle en $C_1$-$C_4$. Le méthyle et l'éthyle sont particulièrement préférés.

Par « alcényle en $C_2$-$C_6$ » on entend au sens de la présente invention une chaîne hydrocarbonée à 6 carbones, linéaire ou ramifiée, comprenant au moins une insaturation (double liaison C=C), mais non aromatique. A titre d'exemple d'alcényle en $C_2$-$C_6$ ont peut citer notamment l'éthylényle (ou vinyle), le 1-propènyle, l'allyle (2-propényle), le n-butényle. De préférence, l'alcényle en $C_2$-$C_6$ est un alcényle en $C_2$-$C_4$. Le vinyle est particulièrement préféré.

Au sens de la présente invention, le terme « aliphatique » vise aussi bien des alkyles que des alcényles.

Au sens de la présente invention, un groupe aromatique est un aryle ou un hétéroaryle.

Par « aryle », on entend, au sens de la présente invention, un groupement hydrocarboné aromatique, comportant de préférence de 6 à 10 atomes de carbone, et comprenant un ou plusieurs cycles accolés, comme par exemple un groupement phényle, indanyle ou naphtyle. Avantageusement, il s'agit du phényle.

Par « hétéroaryle » ou « hétéroaromatique », on entend, au sens de la présente invention, un groupe aromatique comprenant 5 à 10 atomes cycliques dont un ou plusieurs hétéroatomes, avantageusement 1 à 4 et encore plus avantageusement 1 ou 2, tels que par exemple des atomes de soufre, azote ou oxygène, les autres atomes cycliques étant des atomes de carbone. Des exemples de groupes hétéroaryle sont les groupes furyle, thiényle, pyrrolyle, pyridinyle, pyrimidinyle, pyrazolyle, imidazolyle, triazolyle, tétrazolyle ou encore indyle.

Par « composé propargylique », on entend au sens de la présente invention un composé comprenant le groupement chimique de formule -$CH_2$-C≡CH (radical propargyle).

Par « oligomère $\alpha,\omega$-bis(propargylique) » on entend au sens de la présente invention un oligomère comprenant

deux groupes terminaux propargyle ($-CH_2-C{\equiv}CH$). L'oligomère α,ω-bis(propargylique) selon l'invention ne comprend pas de groupe amide.

Par « agent de réticulation comprenant au moins trois groupes azotures $-N_3$ », on entend au sens de la présente invention une molécule comprenant au moins trois groupes azotures $-N_3$ et possédant une masse moléculaire comprise entre 200 et 1000 $g.mol^{-1}$. L'agent de réticulation selon l'invention ne comprend pas de groupe amide.

Par pentaérythritol triazide, on entend le composé de formule :

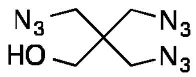.

Par « oligomère α,ω-bis(azidé) » on entend au sens de la présente invention un oligomère comprenant deux groupes terminaux azotures ($-N_3$). L'oligomère α,ω-bis(azidé) selon l'invention ne comprend pas de groupe amide.

Par « groupe partant », on entend, au sens de la présente invention, un groupement chimique qui peut être facilement déplacé par un nucléophile lors d'une réaction de substitution nucléophile, le nucléophile étant plus particulièrement un groupe azotures $-N_3$. Un tel groupe partant peut être plus particulièrement un atome d'halogène tel qu'un atome de chlore, de brome ou d'iode, ou un groupe sulfonate tel qu'un mésylate ($-OS(O_2)-CH_3$), un triflate ($-OS(O)_2-CF_3$) ou encore un tosylate ($-OS(O)_2-(p-Me-C_6H_4)$).

## DESCRIPTION DETAILLEE DE L'INVENTION

*Compositions réticulables*

La présente invention concerne en premier lieu une composition réticulable comprenant :

i) un oligomère α,ω-bis(propargylique) fluoré de formule (I) :

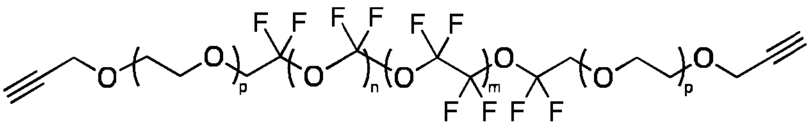

(I)

dans laquelle m est compris entre 1 et 100, par exemple entre 1 et 93,

n est compris entre 2 et 150, par exemple entre 1 et 128, et
p est compris entre 0 et 2, par exemple 0 ou 1.75,
n, m et p étant choisis de manière que l'oligomère α,ω-bis(propargylique) fluoré de formule (I) soit de masse molaire moyenne en nombre $M_n$ comprise entre 400 et 25000;

ii) un agent de réticulation comprenant au moins trois groupes azotures $-N_3$ ; et
iii) optionnellement un oligomère fluoré comprenant deux groupes terminaux azotures $-N_3$, ou oligomère α,ω-bis(azidé) fluoré.

Avantageusement, les compositions réticulables selon l'invention comprennent un nombre total de groupes propargyliques ($-CH_2-C{\equiv}CH$) égal au nombre total de groupes azotures $-N_3$. En d'autres termes, les proportions molaires respectives des oligomères (i), (ii) et (iii) sont telles que le nombre total de groupes propargyliques ($-CH_2-C{\equiv}CH$) est égal au nombre total de groupes azotures $-N_3$.

Par exemple, les proportions relatives d'agent de réticulation comprenant trois groupes azotures $-N_3$ (ii) et d'oligomère α,ω-bis(azidé) fluoré (iii) sont telles que 20% (en moles) des groupes azotures $-N_3$ sont apportés par l'agent de réticulation comprenant trois groupes azotures $-N_3$, par rapport au nombre total des groupes azotures dans la composition réticulable selon l'invention.

Dans un deuxième exemple, les proportions relatives d'agent de réticulation comprenant trois groupes azotures $-N_3$ (ii) et d'oligomère α,ω-bis(azidé) fluoré (iii) sont telles que 40% (en moles) des groupes azotures $-N_3$ sont apportés par l'agent de réticulation comprenant trois groupes azotures $-N_3$, par rapport au nombre total des groupes azotures dans la composition réticulable selon l'invention.

Dans un troisième exemple, les proportions relatives d'agent de réticulation comprenant trois groupes azotures $-N_3$ (ii) et d'oligomère α,ω-bis(azidé) fluoré (iii) sont telles que 60% (en moles) des groupes azotures $-N_3$ sont apportés

par l'agent de réticulation comprenant trois groupes azotures $-N_3$, par rapport au nombre total des groupes azotures dans la composition réticulable selon l'invention.

Dans un quatrième exemple, les proportions relatives d'agent de réticulation comprenant trois groupes azotures $-N_3$ (ii) et d'oligomère $\alpha,\omega$-bis(azidé) fluoré (iii) sont telles que 80% (en moles) des groupes azotures $-N_3$ sont apportés par l'agent de réticulation comprenant trois groupes azotures $-N_3$, par rapport au nombre total des groupes azotures dans la composition réticulable selon l'invention.

*(i) Oligomère $\alpha,\omega$-bis(propargylique) fluoré*

L'oligomère $\alpha,\omega$-bis(propargylique) fluoré de formule (I) est particulièrement avantageux en particulier parce qu'il est dépourvu de tout groupe amide, dont la sensibilité à l'humidité est avérée. Par ailleurs, le motif perfluoropolyéther lui confère la stabilité chimique nécessaire pour une utilisation prolongée en milieu sévère. Enfin, la présence d'atomes d'oxygène dans le squelette carboné confère au matériau obtenu après réticulation par chimie click une flexibilité avantageuse pour les applications envisagées (encapsulants, joints etc...), et quoi qu'il en soit supérieure à celle d'une chaîne comprenant uniquement des atomes de carbone et de fluor.

De préférence, l'oligomère $\alpha,\omega$-bis(propargylique) fluoré de formule (I) présente une masse molaire moyenne en nombre $M_n$ comprise entre 400 et 25000 $g.mol^{-1}$, de manière encore préférée entre 800 et 4000 $g.mol^{-1}$, de manière préférée entre toutes entre 1000 et 2000 $g.mol^{-1}$. Par exemple, l'oligomère $\alpha,\omega$-bis(propargylique) fluoré de formule (I) possède une masse molaire moyenne en nombre $M_n$ d'environ 1250-1900 $g.mol^{-1}$, notamment 1250-1300 $g.mol^{-1}$ ou 1750-1850 $g.mol^{-1}$.

Ainsi, avantageusement, m est compris entre 1 et 93 et n est compris entre 2 et 128.

Dans un mode de réalisation particulier, p est égal à 1.75. Dans ce cas, m est typiquement compris entre 5 et 7 (notamment entre 5.5 et 6.5), et n est typiquement compris entre 7 et 10 (notamment entre 8 et 9).

L'oligomère $\alpha,\omega$-bis(propargylique) fluoré de formule (I) est obtenu par réaction de substitution nucléophile $S_n2$ entre :

- le diol de formule (II):

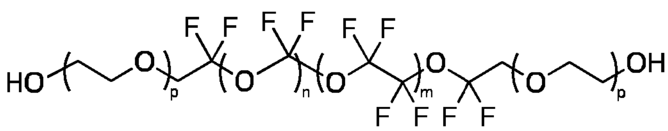

(II),

dans laquelle m, n et p sont tels que définis ci-dessus, et

- un composé propargylique de formule $LG-CH_2-C\equiv CH$, dans laquelle LG représente un groupe partant, par exemple choisi parmi un groupe sulfonate ($RSO_2O$-) et un halogène, en particulier un mésylate, un triflate, un tosylate, un atome de chlore, de brome ou d'iode. De préférence, le composé propargylique est de formule $X-CH_2-C\equiv CH$, X représentant un atome d'halogène, en particulier choisi parmi le chlore, le brome et l'iode, le brome étant préféré. Les conditions réactionnelles à mettre en oeuvre pour cette réaction sont classiques pour l'homme du métier, qui pourra en particulier s'inspirer de l'article de Yang et al (J. Mater. Chem. 2012, 22, 1100-1106).

Par exemple, dans le cas où p est égal à 0, l'oligomère $\alpha,\omega$-bis(propargylique) fluoré de formule (I) est obtenu par propargylation du poly(oxyde de tétrafluoroéthylène-*co*-oxyde de difluorométhylène)-$\alpha,\omega$-diol vendu sous le nom commercial Fomblin® Z-DOL (disponible notamment chez Solvay Specialty Polymers). Dans le cas où p est égal à 1.75, il est obtenu par propargylation du poly(oxyde de tétrafluoroéthylène-co-oxyde de difluorométhylène)-$\alpha,\omega$-diol vendu sous le nom commercial Fluorolink® E10H (disponible notamment chez Solvay Specialty Polymers).*(ii) Agent de réticulation comprenant au moins trois groupes azotures - $N_3$ (ou triazide)*

L'agent de réticulation selon l'invention ne comprend pas de groupe amide.

L'agent de réticulation selon l'invention comporte au moins trois groupes azotures $-N_3$. De préférence, il comporte trois groupes azotures $-N_3$.

En outre, il est préférable que les trois groupes azotures $-N_3$ soient portés par une molécule de faible masse molaire qui garantit, par sa taille réduite, une bonne mobilité moléculaire et donc une réactivité élevée. Dans le même temps, cette caractéristique dimensionnelle confère au mélange réactionnel conduisant au matériau selon l'invention une viscosité réduite. Ainsi, on préférera des agents de réticulation triazidés compacts.

En particulier, l'agent de réticulation peut être représenté par la formule (III) suivante :

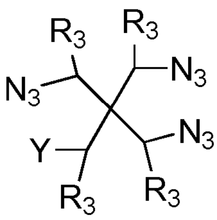

(III)

dans laquelle $R_3$ représente un atome d'hydrogène, un groupe aliphatique en $C_1$-$C_6$, ou un groupe aromatique, et Y représente un groupe choisi parmi H, OH, un groupe aromatique, un groupe aliphatique en $C_1C_6$, un groupe $O(CH_2)_sP(O)(OR_4)_2$ avec
s un entier compris entre 2 et 20, de préférence compris entre 2 et 6, de manière encore préférée égal à 2,
$R_4$ représentant H ou un groupe aliphatique en $C_1$-$C_6$, notamment un groupe méthyle, éthyle, isopropyle, de préférence méthyle.

Dans un mode de réalisation particulier, Y représente un groupe choisi parmi H, OH, un groupe aliphatique en $C_1$-$C_6$ et un groupe aromatique, de préférence OH.

Il est connu de l'homme du métier que les polymères contenant des atomes de phosphore présentent des propriétés spécifiques très intéressantes permettant diverses applications, telles que :

1. Action ignifugeante (il est connu que les dérivés phosphorés sont d'excellents retardateurs de flammes),
2. Substrat pour revêtements anti-corrosion et anti-salissure (« antifouling »),
3. Promoteur d'adhésion.

Ainsi, dans un autre mode de réalisation, Y représente un groupe $(CH_2)_sP(O)(OR_4)_2$ avec s et $R_4$ tels que définis ci-dessus. De préférence, s est alors égal à 2 et $R_4$ représente de préférence $P(O)(OH)_2$ ou $P(O)(OCH_3)_2$. Dans ce mode de réalisation, lorsque s est égal à 2, l'agent de réticulation est obtenu par une réaction de type oxa-Michael entre le composé de formule (III) avec Y représentant OH et un composé de formule $CH_2$=CH-$P(O)(OR_4)_2$ avec $R_4$ tel que défini ci-dessus. Lorsque s est différent de 2, l'agent de réticulation est obtenu par une réaction d'addition radicalaire, en présence d'un amorceur radicalaire, entre un hydrogénophosphonate de dialkyle de formule H-$P(O)(OR_4)_2$ avec $R_4$ tel que défini ci-dessus et un composé de formule (III) dans laquelle Y représente un groupe $O(CH_2)_{s-2}CH=CH_2$ (obtenu par réaction entre un composé de formule (III) avec Y représentant OH et un composé de formule LG-$(CH_2)_{S-2}CH=CH_2$, LG représentant un groupe partant par exemple choisi parmi un groupe sulfonate ($RSO_2O$-) et un halogène, en particulier un mésylate, un triflate, un tosylate, un atome de chlore, de brome ou d'iode.

De préférence, $R_3$ représente un atome d'hydrogène, un groupe alkyle en $C_1$-$C_6$ ou un aryle en $C_6$-$C_{10}$, et/ou Y représente un groupe choisi parmi H, OH et un groupe alkyle en $C_1$-$C_6$ ou un groupe $O(CH_2)_sP(O)(OR_4)_2$ avec s et $R_4$ tel que défini ci-dessus, de préférence OH.

De manière encore préférée, $R_3$ représente un atome d'hydrogène et Y représente OH, $OCH_2CH_2P(O)(OH)_2$ ou $OCH_2CH_2P(O)(OCH_3)_2$. En particulier, l'agent de réticulation peut être le pentaérythritol triazide.

*(iii) Oligomère fluoré $\alpha,\omega$)-bis(azidé)*

De préférence, l'oligomère fluoré $\alpha,\omega$-bis(azidé) est linéaire, et avantageusement représenté par la formule (IV) :

$$N_3\text{-}CHR_1(CHR_2)_q\text{-}R_F\text{-}(CHR_2)_qCHR_1\text{-}N_3 \qquad (IV)$$

dans laquelle le radical $R_F$ représente une chaîne fluorée,
q est 0 ou 1, et
$R_1$ et $R_2$ sont indépendamment choisis parmi un atome d'hydrogène, un alkyle en $C_1$-$C_6$ et un alcényle en $C_2$-$C_6$.

De préférence, l'oligomère $\alpha,\omega$-bis(azidé) fluoré de formule (IV) présente une masse molaire moyenne en nombre $M_n$ comprise entre 400 et 25000 $g.mol^{-1}$, de manière encore préférée entre 800 et 4000 $g.mol^{-1}$, de manière préférée entre toutes entre 1000 et 2000 $g.mol^{-1}$.

Dans un premier mode de réalisation, q représente 0 et $R_F$ représente une chaîne polyéther fluorée.

En particulier, dans ce mode de réalisation l'oligomère $\alpha,\omega$-bis(azidé) fluoré est de préférence de formule (V) :

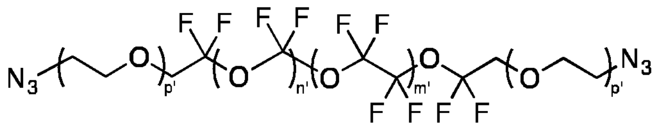

(V),

dans laquelle m', n' et p' sont respectivement tels que m, n et p ci-dessus.

De préférence, l'oligomère $\alpha,\omega$-bis(azidé) fluoré de formule (V) présente une masse molaire moyenne en nombre $M_n$ comprise entre 400 et 25000 $g.mol^{-1}$, de manière encore préférée entre 800 et 4000 $g.mol^{-1}$, de manière préférée entre toutes entre 1000 et 2000 $g.mol^{-1}$. Par exemple, l'oligomère $\alpha,\omega$-bis(azidé) fluoré de formule (V) possède une masse molaire moyenne en nombre $M_n$ d'environ 1250-1900 $g.mol^{-1}$, notamment 1250-1300 $g.mol^{-1}$ ou 1750-1850 $g.mol^{-1}$.

Ainsi, avantageusement, dans ce mode de réalisation, m' est compris entre 1 et 93, de préférence entre 40 et 93. De préférence, n' est compris entre 2 et 128, de préférence entre 50 et 128. De manière particulièrement avantageuse m est compris entre 40 et 93, et n est compris entre 50 et 128. Dans un mode de réalisation, p' est égal à 0. Dans un autre mode de réalisation, p' est égal à 1.75.

L'oligomère $\alpha,\omega$-bis(azidé) fluoré de formule (V) est particulièrement avantageux pour l'invention en particulier parce qu'il est dépourvu de tout groupe amide. Par ailleurs, l'élément fluor lui confère l'inertie chimique nécessaire pour une utilisation prolongée en milieu sévère. Enfin, la présence d'atomes d'oxygène dans la chaîne polymère confère au matériau obtenu après réticulation par chimie click une flexibilité avantageuse pour les applications nécessitant des polymères réticulés particulièrement plastiques, notamment à des températures élevées.

L'oligomère $\alpha,\omega$-bis(azidé) fluoré de formule (V) est par exemple obtenu en deux étapes à partir du diol de formule (II) ci-dessus et est présenté dans le brevet WO 2010/115855. La première étape consiste à transformer les deux groupes hydroxyles primaires en groupes partants, par exemple un groupe sulfonate, notamment choisi parmi le mesylate, le tosylate et le triflate, de préférence un tosylate. Cette première étape est typiquement conduite en présence de chlorure de tosyle et d'une base, telle qu'une trialkylamine, notamment la triéthylamine.

La deuxième étape est alors une substitution nucléophile en présence d'ions azotures, de préférence l'azotures de sodium. Avantageusement, cette deuxième étape est conduite dans un solvant aprotique polaire permettant de solubiliser les réactifs, tel que le DMSO.

Par exemple, lorsque p' est égal à 0, l'oligomère $\alpha,\omega$-bis(azidé) fluoré de formule (V) est obtenu selon les conditions réactionnelles suivantes :

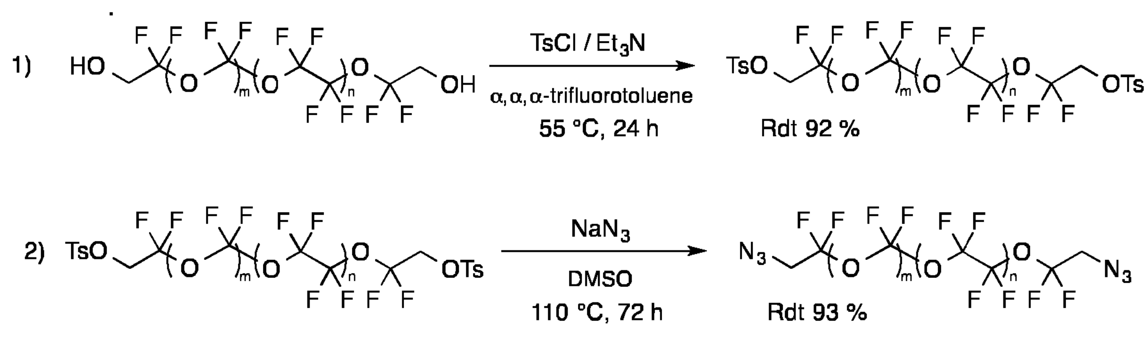


Dans un deuxième mode de réalisation, p vaut 1, et la chaîne $R_F$ est une chaîne perfluorée. Avantageusement, $R_1$ est un atome d'hydrogène et $R_2$ est choisi parmi un atome d'hydrogène, un méthyle ou un vinyle, ou $R_2$ est un atome d'hydrogène et $R_1$ est choisi parmi un atome d'hydrogène, un méthyle ou un vinyle. De préférence, $R_2$ est un atome d'hydrogène et $R_1$ est choisi parmi un atome d'hydrogène, un méthyle ou un vinyle.

Typiquement, la chaîne $R_F$ résulte de la réaction de polymérisation radicalaire par transfert d'iode (ITP) telle que détaillée par exemple dans Chem. Rev. 2006, 106, 3936-3962, et qui met en jeu le composé $I-C_4F_8-I$ et une ou plusieurs oléfines fluorées, de préférence d'une, deux ou trois oléfines fluorées choisies parmi :

Tétrafluoroéthylène (TFE, $F_2C=CF_2$)
Fluorure de vinylidène (VDF, $H_2C=CF_2$)
Hexafluoropropylène (HFP, $F_2C=CF-CF_3$)

Trifluoroéthylène (TrFE, $F_2C=CHF$)
Perfluoro(méthyl vinyl éther) (PMVE, $F_2C=CF-OCF_3$)
3,3,3-trifluoropropène (TFP, $H_2C=CH-CF_3$)
2,3,3,3-tétrafluoropropène (R-1234yf, $H_2C=CF-CF_3$)
1,3,3,3-tétrafluoropropène (R-1234ze, trans $FHC=CH-CF_3$)
Chlorotrifluoroéthylène (CTFE, $F_2C=CFCl$)
Bromotrifluoroéthylène (BTFE, $F_2C=CFBr$)
Iodotrifluoroéthylène (ITFE, $F_2C=CFI$) 2-chloro-3,3,3-trifluoropropène (1233xf, $H_2C=CCl-CF_3$)
Fluorure de vinyle (VF, $H_2C=CHF$)
Perfluoro(éthyl vinyl éther) (PEVE, $F_2C=CF-OCF_2CF_3$)
Perfluoro(propyl vinyléther) (PPVE, $F_2C=CF-OCF_2CF_2CF_3$)
2-bromo-1,1-difluoroéthylène (R-1122B1, $F_2C=CHBr$)
Chlorodifluoroéthylène (CFE)
Dichlorodifluoroéthylène (DCFE, $F_2C=CCl_2$)
1,1,3,3,3-Pentafluoropropène (R-1225zc, $F_2C=CH-CF_3$)
1, 1, 2, 3, 4, 4-Hexafluoro-1,3-butadiène (FC 2316, $F_2C=CF-CF=CF_2$)
1,1,3,3,3-pentafluoro-2-(trifluoromethyl)prop-1-ene (PFIB, $F_2C=C(CF_3)_2$) et leurs dérivés.

Ainsi, avantageusement, dans ce deuxième mode de réalisation, l'oligomère fluoré α,ω-bis-azidé de formule (III) est mieux représenté par la formule (VI) :

$$N_3\text{-}CHR_1CHR_2\text{-}(CR_{a1}R_{b1}CR_{c1}R_{d1})_{n1}\text{-}...\text{-}(CR_{ai}R_{bi}CR_{ci}R_{di})_{ni}\text{-}(CF_2)_r\text{-}(CR_{ci}R_{di}CR_{ai}R_{bi})_{ni}\text{-}...\text{-}(CR_{c1}R_{d1}CR_{a1}R_{b1})_{n1}\text{-}CHR_2CHR_1\text{-}N_3 \quad \text{(VI)},$$

dans laquelle $R_1$ et $R_2$ sont tels que définis ci-dessus,
r est compris entre 2 et 8, et est de préférence égal à 4
i est compris entre 1 et 20, de préférence entre 1 et 5, de manière encore préférée entre 1 et 3 ou entre 1 et 2,
$CR_{a1}R_{b1}CR_{c1}R_{d1}$ à $CR_{ai}R_{bi}CR_{ci}R_{di}$ sont des motifs constitutifs, identiques ou différents, issus de monomères indépendamment choisis parmi les oléfines fluorées listées ci-dessus (i.e. les oléfines $CR_{a1}R_{b1}=CR_{c1}R_{d1}$ à $CR_{ai}R_{bi}=CR_{ci}R_{di}$, identiques ou différentes, sont choisies parmi les oléfines fluorées listées ci-dessus), et
$n_1$ à $n_i$ représentent chacun indépendamment un nombre choisi parmi 1 à 20, de préférence 1 à 5.

Avantageusement, $R_1$ est un atome d'hydrogène et $R_2$ est choisi parmi un atome d'hydrogène, un méthyle ou un vinyle, ou $R_2$ est un atome d'hydrogène et $R_1$ est choisi parmi un atome d'hydrogène, un méthyle ou un vinyle. De préférence, $R_2$ est un atome d'hydrogène et $R_1$ est choisi parmi un atome d'hydrogène, un méthyle ou un vinyle.

Par exemple, dans ce deuxième mode de réalisation, l'oligomère α,ω-bis(azidé) fluoré de formule (VI) est obtenu par polymérisation radicalaire par transfert d'iode (ITP) du composé $I\text{-}C_4F_8\text{-}I$ et des deux oléfines fluorées VDF et PMVE, et peut être représenté par la formule (VII) : $N_3\text{-}CHR_1CHR_2\text{-}(CF_2CH_2)_{n1}\text{-}(CF(OCF_3)CF_2)_{n2}\text{-}(CF_2)_4\text{-}(CF_2CF(OCF_3))_{n2}(CH_2CF_2)_{n1}CHR_2CHR_1N_3$ (VII), dans laquelle $R_1$ et $R_2$ sont tels que définis ci-dessus, et $n_1$ est compris entre 1 et 20, et $n_2$ est compris entre 1 et 20.

Avantageusement, dans la formule (VII), $R_1$ est un atome d'hydrogène et $R_2$ est choisi parmi un atome d'hydrogène, un méthyle ou un vinyle, ou $R_2$ est un atome d'hydrogène et $R_1$ est choisi parmi un atome d'hydrogène, un méthyle ou un vinyle. De préférence, $R_1$ est un atome d'hydrogène et $R_2$ est choisi parmi un atome d'hydrogène, un méthyle ou un vinyle.

De préférence, l'oligomère α,ω-bis(azidé) fluoré de formule (VI) ou (VII) présente une masse molaire moyenne en nombre $M_n$ comprise entre 500 et 8000 $g.mol^{-1}$, de manière encore préférée entre 2000 et 4000 $g.mol^{-1}$, de manière préférée entre toutes entre 1000 et 2000 $g.mol^{-1}$. Par exemple, l'oligomère α,ω-bis(azidé) fluoré de formule (VI) possède une masse molaire moyenne en nombre $M_n$ d'environ 4000 $g.mol^{-1}$.

En particulier, dans ce deuxième mode de réalisation, l'oligomère α,ω-bis(propargylique) fluoré (iii) de formule (VI) est obtenu par :

1) polymérisation par transfert d'iode (ITP) du composé $I\text{-}C_rF_{2r}\text{-}I$ avec r compris entre 2 et 8 (de préférence 4) et d'une ou plusieurs (de préférence 1, 2 ou 3) oléfines fluorées choisies parmi :

Tétrafluoroéthylène (TFE, $F_2C=CF_2$)
Fluorure de vinylidène (VDF, $H_2C=CF_2$)
Hexafluoropropylène (HFP, $F_2C=CF-CF_3$)

Trifluoroéthylène (TrFE, $F_2C{=}CHF$)
Perfluoro(méthyl vinyl éther) (PMVE, $F_2C{=}CF{-}OCF_3$)
3,3,3-trifluoropropène (TFP, $H_2C{=}CH{-}CF_3$)
2,3,3,3-tétrafluoropropène (R-1234yf, $H_2C{=}CF{-}CF_3$)
1,3,3,3-tétrafluoropropène (R-1234ze, trans $FHC{=}CH{-}CF_3$)
Chlorotrifluoroéthylène (CTFE, $F_2C{=}CFCl$)
Bromotrifluoroéthylène (BTFE, $F_2C{=}CFBr$)
Iodotrifluoroéthylène (ITFE, $F_2C{=}CFI$)
2-chloro-3,3,3-trifluoropropène (1233xf, $H_2C{=}CCl{-}CF_3$)
Fluorure de vinyle (VF, $H_2C{=}CHF$)
Perfluoro(éthyl vinyl éther) (PEVE, $F_2C{=}CF{-}OCF_2CF_3$)
Perfluoro(propyl vinyléther) (PPVE, $F_2C{=}CF{-}OCF_2CF_2CF_3$)
2-bromo-1,1-difluoroéthylène (R-1122B1, $F_2C{=}CHBr$)
Chlorodifluoroéthylène (CFE)
Dichlorodifluoroéthylène (DCFE, $F_2C{=}CCl_2$)
1,1,3,3,3-Pentafluoropropène (R-1225zc, $F_2C{=}CH{-}CF_3$)
1, 1, 2, 3, 4, 4-Hexafluoro-1,3-butadiène (FC 2316, $F_2C{=}CF{-}CF{=}CF_2$)
1-Propene, 1, 1, 3, 3, 3-pentafluoro-2-(trifluoromethyl)-1-propène (PFIB, $F_2C{=}C(CF_3)_2$)
et leurs dérivés ;

2) réaction d'homologation à l'aide d'une oléfine non fluorée de formule $CHR_1{=}CHR_2$, dans laquelle $R_1$ et $R_2$ sont tels que définis ci-dessus ; et
3) azidation, de préférence en présence de $NaN_3$.

De préférence, pour l'étape 2), l'oléfine non fluorée de formule $CHR_1{=}CHR_2$ est choisie parmi l'éthylène, le propylène et le butadiène, avantageusement l'éthylène.

Ainsi, dans le cas particulier où l'étape 1) est conduite à l'aide de deux oléfines, le VDF et le PMVE, et l'étape 2) est conduite avec l'éthylène, le schéma de synthèse est présenté ci-après. Les étapes d'ITP et d'éthylénation sont en particulier décrites dans Macromolecules 2010, 43, 3652-3663 et Macromolecules 2012, 45, 7375-7387.

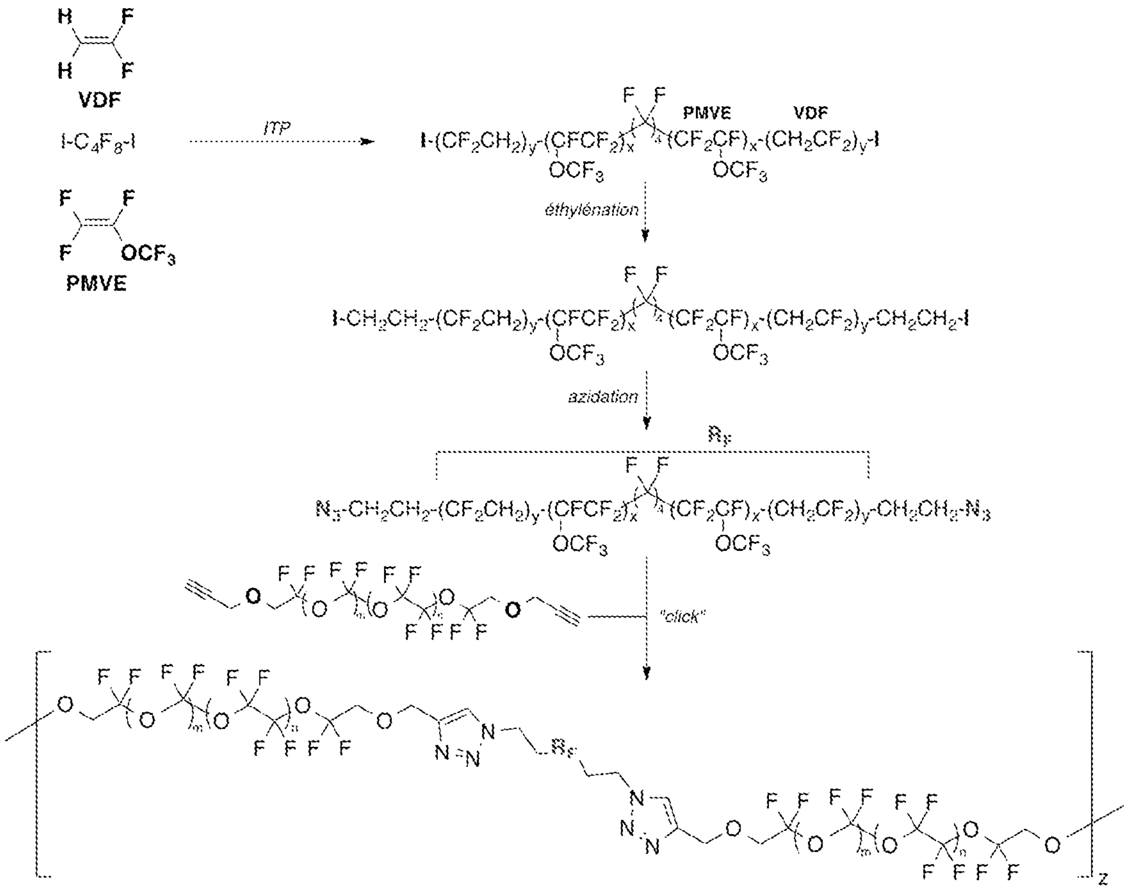


.

Par ailleurs, l'oligomère fluoré $\alpha,\omega$-bis-azidé peut être un mélange d'oligomères fluorés $\alpha,\omega$-bis-azidés issus des deux modes de réalisation ci-dessus. Ainsi, en particulier, l'oligomère fluoré $\alpha,\omega$-bis-azidé (iii) de formule (IV) peut être un mélange d' oligomères de formule (V) et/ou (VI), en toutes proportions.

*Modes de réalisation préférés*

Dans un mode de réalisation particulier, la composition réticulable selon l'invention comprend :

i) un oligomère $\alpha,\omega$-bis(propargylique) fluoré de formule (I) telle que définie ci-dessus, avantageusement avec p = 0 ou p = 1.75; et
ii) un agent de réticulation comprenant trois groupes azotures $-N_3$, notamment de formule (II) telle que définie ci-dessus, de préférence avec $R_3$ représentant un atome d'hydrogène et Y représente OH, $OCH_2CH_2P(O)(OH)_2$ ou $OCH_2CH_2P(O)(OCH_3)_2$, notamment il s'agit du pentaérythritol triazide.

Dans un autre mode de réalisation particulier, la composition réticulable selon l'invention comprend :

i) un oligomère $\alpha,\omega$-bis(propargylique) fluoré de formule (I) telle que définie ci-dessus, avantageusement avec p = 0 ou p = 1.75;
ii) un agent de réticulation comprenant trois groupes azotures $-N_3$, avantageusement de formule (II) telle que définie ci-dessus, de préférence avec $R_3$ représentant un atome d'hydrogène et Y représente OH, $OCH_2CH_2P(O)(OH)_2$ ou $OCH_2CH_2P(O)(OCH_3)_2$, notamment il s'agit du pentaérythritol triazide ; et
iii) un oligomère $\alpha,\omega$-bis(azidé) fluoré choisi parmi les oligomères de formule (V) telle que définie ci-dessus, avec p' typiquement égal à 0 ou 1.75. De préférence, p et p' sont identiques.

Dans un autre mode de réalisation particulier, la composition réticulable selon l'invention comprend :

i) un oligomère $\alpha,\omega$-bis(propargylique) fluoré de formule (I) telle que définie ci-dessus, avantageusement avec p =

0 ou p = 1.75;

ii) un agent de réticulation comprenant trois groupes azotures $-N_3$, avantageusement de formule (III) telle que définie ci-dessus, de préférence, avec $R_3$ représentant un atome d'hydrogène et Y représente OH, $OCH_2CH_2P(O)(OH)_2$ ou $OCH_2CH_2P(O)(OCH_3)_2$, notamment il s'agit du pentaérythritol triazide ; et

iii) un oligomère α,ω-bis(azidé) fluoré choisi parmi les oligomères de formule (VI) telle que définie ci-dessus.

Dans ces modes de réalisation particuliers, les proportions molaires respectives des oligomères (i) et (iii) et de l'agent de réticulation (iii) sont telles que le nombre total de groupes propargyliques ($-CH_2-C\equiv CH$) est égal au nombre total de groupes azotures $-N_3$.

Ainsi, dans le premier mode de réalisation particulier, les proportions molaires respectives oligomère (i) : agent de réticulation (ii) sont égales à 3:2.

Dans les deuxième et troisième modes de réalisation particuliers, les proportions relatives d'agent de réticulation comprenant trois groupes azotures $-N_3$(ii) et d'oligomère α,ω-bis(azidé) fluoré (iii) sont par exemple telles que 20%, 40%, 60% ou 80% (en moles) des groupes azotures $-N_3$ sont apportés par l'agent de réticulation comprenant trois groupes azotures $-N_3$, par rapport au nombre total des groupes azotures.

*Matériaux*

La présente invention vise également un matériau issu de la réaction par chimie « click » entre :

i) un oligomère α,ω-bis(propargylique) fluoré de formule (I) telle que définie ci-dessus, notamment avec p égal à 0 ou 1.75;

ii) un agent de réticulation comprenant au moins trois groupes azotures $-N_3$ notamment de formule (III) telle que définie ci-dessus ; et

iii) optionnellement un oligomère fluoré comprenant deux groupes terminaux azotures $-N_3$, ou oligomère α,ω-bis(azidé) fluoré.

En d'autres termes, le matériau selon l'invention comprend ou est constitué du produit de la réaction par chimie « click » de la composition réticulable selon l'invention.

De préférence, la réaction de chimie « click » est mise en oeuvre en présence d'un catalyseur au cuivre, tel qu'un sel de cuivre (I).

Les modes de réalisation particuliers, avantageux et préférés du matériau selon l'invention sont identiques à ceux de la composition réticulable selon l'invention, notamment en ce qui concerne la définition des oligomères (i) et (iii), de l'agent de réticulation (ii), et de leurs proportions molaires respectives.

Notamment, dans un mode de réalisation préféré, le matériau selon l'invention comprend le produit de la réaction par chimie « click » entre:

i) un oligomère α,ω-bis(propargylique) fluoré de formule (I) telle que définie ci-dessus, notamment avec p égal à 0 ou 1.75; et

ii) un agent de réticulation comprenant trois groupes azotures $-N_3$, avantageusement de formule (III) telle que définie ci-dessus, de préférence, avec $R_3$ représentant un atome d'hydrogène et Y représente OH, $OCH_2CH_2P(O)(OH)_2$ ou $OCH_2CH_2P(O)(OCH_3)_2$, notamment il s'agit du pentaérythritol triazide.

Dans un autre mode de réalisation préféré, le matériau selon l'invention comprend le produit de la réaction par chimie « click » entre :

i) un oligomère α,ω-bis(propargylique) fluoré de formule (I) telle que définie ci-dessus, notamment avec p égal à 0 ou 1.75;

ii) un agent de réticulation comprenant trois groupes azotures $-N_3$, avantageusement de formule (III) telle que définie ci-dessus, de préférence, avec $R_3$ représentant un atome d'hydrogène et Y représente OH, $OCH_2CH_2P(O)(OH)_2$ ou $OCH_2CH_2P(O)(OCH_3)_2$, notamment il s'agit du pentaérythritol triazide ; et

iii) un oligomère α,ω-bis(azidé) fluoré choisi parmi les oligomères de formule (IV) telle que définie ci-dessus, notamment avec p' égal à 0 ou 1.75. De préférence, p et p' sont identiques.

Dans un autre mode de réalisation préféré, le matériau selon l'invention comprend le produit de la réaction par chimie « click » entre :

i) un oligomère α,ω-bis(propargylique) fluoré de formule (I) telle que définie ci-dessus, notamment avec p égal à 0

ou 1.75;

ii) un agent de réticulation comprenant trois groupes azotures $-N_3$, avantageusement de formule (III) telle que définie ci-dessus, de préférence, avec $R_3$ représentant un atome d'hydrogène et Y représente OH, $OCH_2CH_2P(O)(OH)_2$ ou $OCH_2CH_2P(O)(OCH_3)_2$, notamment il s'agit du pentaérythritol triazide ; et

iii) un oligomère $\alpha,\omega$-bis(azidé) fluoré choisi parmi les oligomères de formule (V) et/ou (VI) telle que définie ci-dessus.

Les proportions molaires respectives des composés (i), (ii) et (iii) pour obtenir le matériau selon l'invention sont telles que le nombre total de groupes propargyliques ($-CH_2-C\equiv CH$) est égal au nombre total de groupes azotures $-N_3$.

Ainsi, dans le premier mode de réalisation particulier, les proportions molaires respectives des composés (i) : (ii) sont égales à 3:2.

Dans les deuxième et troisième modes de réalisation particuliers, les proportions relatives d'agent de réticulation comprenant trois groupes azotures $-N_3$ (ii) et d'oligomère $\alpha,\omega$-bis(azidé) fluoré (iii) sont par exemple telles que 20%, 40%, 60% ou 80% (en moles) des groupes azotures $-N_3$ sont apportés par l'agent de réticulation comprenant trois groupes azotures $-N_3$, par rapport au nombre total des groupes azotures.

Comme indiqué plus haut, les propriétés des matériaux selon l'invention peuvent être modulées à façon en fonction de la longueur et de la nature de la chaîne fluorée de l'oligomère $\alpha,\omega$-bis(azidé) et de la proportion molaire d'agent de réticulation dans la composition réticulable de départ. En particulier, les effets des variations de proportion molaire d'agent de réticulation dans la composition réticulable de départ sur la structure du matériau sont expliqués schématiquement sur la figure 16. Rappelons que, avantageusement, dans chaque formulation, le nombre total de groupements azotures apportés indifféremment par la chaîne fluorée de l'oligomère $\alpha,\omega$-bis(azidé) ou par l'agent de réticulation est toujours égal au nombre de groupements propargyliques apportés par l'oligomère $\alpha,\omega$-bis(propargylique) fluoré.

Ainsi, les matériaux selon la présente invention peuvent être assimilés à des matériaux thermodurcissables ou à des élastomères, notamment en fonction de la densité de maillage de leur réseau en 3 dimensions.

Par exemple, une faible quantité d'agent de réticulation triazidé permet la production d'une matrice polymère très flexible mais qui ne coulera plus une fois réticulée. Ce type de matière est notamment recherché pour la réalisation d'encapsulants très flexibles utiles pour la protection d'éléments électroniques dotés de fils de connectiques et de soudures fragiles.

Une proportion croissante d'agent de réticulation triazidé induit un réseau polymère avec une densité plus importante des points de réticulation (voir figure 16), et donc une rigidité mécanique plus élevée qui sera plus adaptée à la production de joints élastomériques.

Ainsi, lorsqu'un matériau thermodurcissable est recherché, on choisira en particulier un matériau selon l'invention dans lequel les proportions relatives d'agent de réticulation comprenant trois groupes azotures $-N_3$ (ii) et d'oligomère $\alpha,\omega$-bis(azidé) fluoré (iii) sont telles que au moins 20%, notamment entre 20% et 80% en moles des groupes azotures $-N_3$ sont apportés par l'agent de réticulation comprenant trois groupes azotures $-N_3$, par rapport au nombre total des groupes azotures. On choisira également de préférence un oligomère $\alpha,\omega$-bis(azidé) fluoré (iii) de formule (VI) telle que définie ci-dessus.

En particulier, les matériaux constitués d'agent de réticulation (ii) et d'oligomère $\alpha,\omega$-bis(propargylique) fluoré (i) sont des matériaux thermodurcissables.

Inversement, lorsqu'un matériau flexible (ou élastomère) est recherché, on choisira en particulier un matériau selon l'invention dans lequel les proportions relatives d'agent de réticulation comprenant trois groupes azotures $-N_3$ (ii) et d'oligomère $\alpha,\omega$-bis(azidé) fluoré (iii) sont telles que au plus 20% (en moles), notamment entre 5% et 20% en moles, des groupes azotures $-N_3$ sont apportés par l'agent de réticulation comprenant trois groupes azotures $-N_3$, par rapport au nombre total des groupes azotures. Dans ce cas, l'oligomère $\alpha,\omega$-bis(azidé) fluoré (iii) n'est pas optionnel. On préférera un oligomère $\alpha,\omega$-bis(azidé) fluoré (iii) de formule (V) telle que définie ci-dessus, ou un oligomère $\alpha,\omega$-bis(azidé) fluoré (iii) de formule (VI) avec une grande longueur de chaîne.

Le matériau selon l'invention, qu'il soit assimilé à un thermodurcissable ou à un élastomère, comprend, outre les motifs fluorés décrits pour les oligomères (i) et (iii) et le motif de l'agent de réticulation (ii), des motifs 1,2,3-triazole disubstitués, et en particulier 1,2,3-triazole 1,4-disubstitués. Les motifs 1,2,3-triazole étant de nature hétéroaromatiques et peu réactifs chimiquement, ceux-ci participent, aux côtés des motifs fluorés, de la stabilité chimique des matériaux selon l'invention, notamment en milieu sévère et/ou à haute température, notamment à une température supérieure à 250 °C, voire 300 °C, par exemple à une température comprise entre 250 °C et 350 °C.

Le matériau selon l'invention possède avantageusement une température de décomposition à une valeur de 10% de perte de poids ($T_d^{10\%}$) sous air supérieure ou égale à 250 °C, de préférence supérieure ou égale à 275 °C, de manière encore préférée supérieure ou égale à 280 °C, de manière préférée entre toutes supérieure ou égale à 300 °C. Par exemple, le matériau selon l'invention possède une $T_d^{10\%}$ comprise entre 250 °C et 400 °C, de préférence entre 250 °C et 350 °C.

Le matériau selon l'invention possède avantageusement au moins une valeur température de transition vitreuse

($T_{g1}$) inférieure ou égale à -70 °C, de préférence inférieure ou égale à -80 °C, de manière préférée entre toutes inférieure ou égale à - 100 °C. Cette valeur de transition vitreuse est choisie en fonction des applications envisagées pour le polymère. Par exemple, le matériau polymère selon l'invention possède une $T_{g1}$ comprise entre -150 °C et -70 °C, de préférence entre -120 °C et -85 °C.

*Procédé de synthèse des polymères selon l'invention*

La présente invention concerne également un procédé de préparation du matériau selon l'invention.

Le procédé selon l'invention repose sur une réaction de chimie « click » qui présente le triple avantage d'avoir un rendement élevé voire quantitatif, d'avoir une cinétique élevée, et de ne produire aucun sous-produit nocif ou toxique. Du fait de ces caractéristiques, le procédé selon l'invention est facilement industrialisable.

Au sens de la présente invention, un rendement élevé est un rendement supérieur ou égal à 90%, avantageusement supérieur ou égal à 95%. Un rendement quantitatif est un rendement de 100%.

En outre, le procédé selon l'invention apporte une grande modularité et une grande facilité de mise en oeuvre. En effet, les oligomères fluorés (i) et éventuellement (iii), ainsi que l'agent de réticulation (ii), sont préparés en amont, de manière totalement indépendante de la réaction de copolymérisation par chimie click, cette dernière étape pouvant être mise en oeuvre à un stade ultérieur. Ceci est particulièrement avantageux dans la mesure où, notamment dans le cas des oligomères α,ω-bis(azidés) fluorés de formule (VI), les produits de départ sont pour la plupart gazeux.

Ainsi, le procédé de préparation du matériau selon l'invention comprend une étape de réticulation par chimie « click » entre :

i) un oligomère α,ω-bis(propargylique) fluoré de formule (I) telle que définie ci-dessus, notamment avec p égal à 0 ou 1.75;

ii) un agent de réticulation comprenant au moins trois groupes azotures $-N_3$; avantageusement de formule (III) telle que définie ci-dessus et

iii) optionnellement un oligomère fluoré comprenant deux groupes terminaux azotures $-N_3$, ou oligomère α,ω-bis(azidé) fluoré.

En d'autres termes, le procédé de préparation du matériau selon l'invention comprend une étape de réticulation par chimie « click » de la composition réticulable de l'invention.

Les modes de réalisation particuliers, avantageux et préférés du procédé selon l'invention sont identiques à ceux de la composition réticulable selon l'invention, notamment en ce qui concerne la définition des oligomères (i) et (iii), de l'agent de réticulation (ii), et de leurs proportions molaires respectives.

Schématiquement, dans un mode de réalisation particulier, le procédé selon l'invention met en oeuvre la réaction suivante :

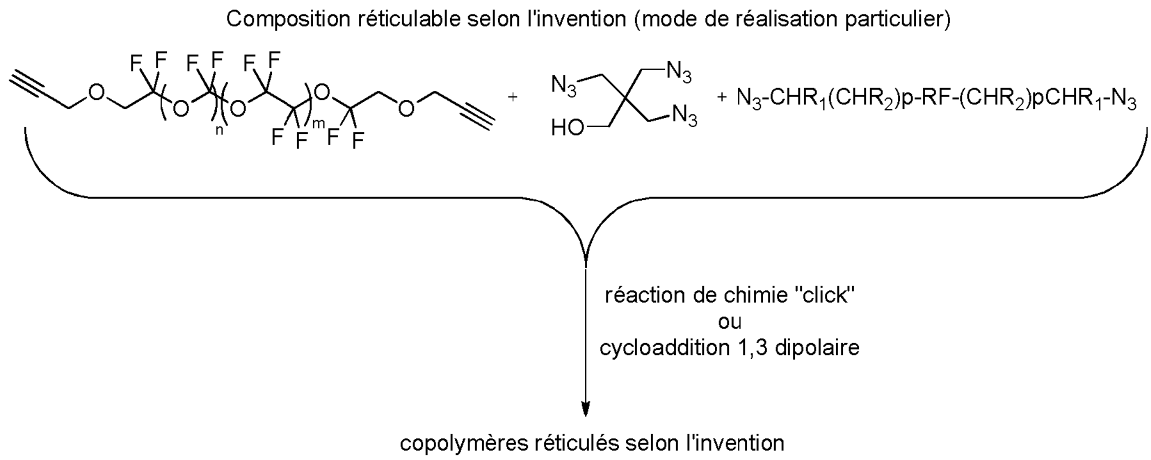


Réticulation par chimie « click » entre un oligomère α,ω-bis(propargylique) fluoré, le pentaérythritol triazide, et un oligomère α,ω-bis(azidé) fluoré.

Avantageusement, les proportions molaires respectives des composés (i), (ii) et (iii) sont telles que le nombre total de groupes propargyliques ($-CH_2-C\equiv CH$) est égal au nombre total de groupes azotures $-N_3$.

La réaction de chimie click possédant un rendement très élevé voire quantitatif, de telles proportions molaires permettent en particulier d'assurer une consommation totale des espèces réactives lors de la mise en oeuvre du procédé selon l'invention.

Par exemple, les proportions relatives d'agent de réticulation comprenant trois groupes azotures $-N_3$ (ii) et d'oligomère $\alpha,\omega$-bis(azidé) fluoré (iii) sont telles que 20% (en moles) des groupes azotures $-N_3$ sont apportés par l'agent de réticulation comprenant trois groupes azotures $-N_3$, par rapport au nombre total des groupes azotures. En d'autres termes, on utilise 1,5 moles de (i) pour 0,2 moles de (ii) et 1,2 moles de (iii).

Dans un deuxième exemple, les proportions relatives d'agent de réticulation comprenant trois groupes azotures $-N_3$ (ii) et d'oligomère $\alpha,\omega$-bis(azidé) fluoré (iii) sont telles que 40% (en moles) des groupes azotures $-N_3$ sont apportés par l'agent de réticulation comprenant trois groupes azotures $-N_3$, par rapport au nombre total des groupes azotures. En d'autres termes, on utilise 1,5 moles de (i) pour 0,4 moles de (ii) et 0,9 moles de (iii).

Dans un troisième exemple, les proportions relatives d'agent de réticulation comprenant trois groupes azotures $-N_3$ (ii) et d'oligomère $\alpha,\omega$-bis(azidé) fluoré (iii) sont telles que 60% (en moles) des groupes azotures $-N_3$ sont apportés par l'agent de réticulation comprenant trois groupes azotures $-N_3$, par rapport au nombre total des groupes azotures. En d'autres termes, on utilise 1,5 moles de (i) pour 0,6 moles de (ii) et 0,6 moles de (iii).

Dans un quatrième exemple, les proportions relatives d'agent de réticulation comprenant trois groupes azotures $-N_3$ (ii) et d'oligomère $\alpha,\omega$-bis(azidé) fluoré (iii) sont telles que 80% (en moles) des groupes azotures $-N_3$ sont apportés par l'agent de réticulation comprenant trois groupes azotures $-N_3$, par rapport au nombre total des groupes azotures. En d'autres termes, on utilise 1,5 moles de (i) pour 0,8 moles de (ii) et 0,3 moles de (iii).

La réticulation par chimie « click » est de préférence mise en oeuvre en présence d'un catalyseur au cuivre, de préférence un sel de cuivre (I) ou (II). De préférence on utilisera un sel de cuivre (I) tel qu'un sel de formule CuX, X représentant un groupe triflate ou atome d'halogène, notamment le chlore, le brome ou l'iode. De manière préférée entre toutes, on utilisera le bromure ou le chlorure de cuivre. Le catalyseur est avantageusement utilisé en une quantité comprise entre 0,001 équivalents molaires et 0,2 équivalents molaires, de préférence entre 0,01 et 0,15 équivalents molaires, par rapport au nombre de moles de l'oligomère $\alpha,\omega$-bis(propargylique) (i). En outre, le sel de cuivre est de préférence utilisé en combinaison avec un ligand pour améliorer sa réactivité et/ou sa stabilité, de préférence une polyamine, telle qu'une triamine ou un tétramine linéaire ou cyclique, ou en présence d'un dérivé de pyridine. On citera à titre d'exemple de ligand du cuivre la 1,1,4,7,7-pentaméthyldiéthylènetriamine (encore appelée *N,N,N',N',N"*-pentamethyldiethylenetriamine ou PMDETA), la 1,4,7,10-tetraazacyclododécane, 1,1,4,7,10,10-Hexamethyltriethylenetetramine (HMTETA), la Tris(2-pyridylmethyl)amine (TPMA), la Tris[2-(dimethylamino)ethyl]amine ($Me_6$TREN), la 2,2'-Bipyridyl (bpy), 4,4'-Dinonyl-2,2'-dipyridyl (dNbpy), la 2,2':6',2"-Terpyridine (tpy), la Tris[(1-benzyl-1*H*-1,2,3-triazol-4-yl)methyl]amine (TBTA), et la Tris(3-hydroxypropyltriazolylmethyl)amine (THPTA).

De préférence, le ligand est la 1,1,4,7,7-pentaméthyldiéthylènetriamine. Le ligand est avantageusement utilisé en une quantité comprise entre 0,001 équivalents molaires et 0,2 équivalents molaires, de préférence entre 0,01 et 0,15 équivalents molaires, par rapport au nombre de moles de l'oligomère $\alpha,\omega$-bis(propargylique) (i). Dans un mode de réalisation particulier, la quantité de ligand et de catalyseur au cuivre en équivalents molaires est identique.

Ainsi, on utilisera de préférence un sel de formule CuX, X représentant un groupe triflate ou un atome d'halogène, notamment le chlore ou le brome, en combinaison avec la 1,1,4,7,7-pentaméthyldiéthylènetriamine.

Dans un mode de réalisation particulier, le catalyseur est supporté sur support solide, et est avantageusement recyclable.

Dans un mode de réalisation particulier, le procédé selon l'invention est mis en oeuvre en l'absence de solvant, notamment pour éviter toute contamination par des résidus de cuivre. Dans ce cas, l'un au moins des oligomères (i) ou (iii) ou l'agent de réticulation (ii) joue le rôle de solvant.

Dans un autre mode de réalisation particulier, le procédé selon l'invention est mis en oeuvre dans un solvant capable de solubiliser les oligomères (i) et (iii) et l'agent de réticulation (ii). De préférence, le solvant est aprotique et polaire. Toutefois, le solvant ne devrait pas interagir de manière délétère avec le cuivre. On évitera donc d'employer des solvants dérivés de la pyridine par exemple. De préférence, le solvant utilisé est choisi parmi le DMF, le DMSO ou l'acétone, avantageusement il s'agit du DMF.

La réticulation par réaction de chimie « click » du procédé selon l'invention est de préférence conduite à pression atmosphérique et à une température comprise entre 15 °C et 200 °C, de manière encore préférée entre 18 °C et 180 °C. Dans un mode de réalisation particulier, la température de la réaction est comprise entre 18 °C et 25 °C. Dans un autre mode de réalisation, la température de la réaction est comprise entre 100 °C et 200 °C, de préférence entre 140 °C et 180 °C.

*Modes de réalisation particuliers*

Dans un mode de réalisation particulier, le procédé de préparation du matériau selon l'invention comprend une étape de réticulation par chimie « click » entre :

i) un oligomère α,ω-bis(propargylique) fluoré de formule (I) telle que définie ci-dessus, notamment avec p égal à 0 ou 1.75; et

ii) un agent de réticulation comprenant trois groupes azotures $-N_3$ Avantageusement de formule (III) telle que définie ci-dessus.

De préférence, dans ce mode de réalisation particulier l'agent de réticulation est de formule (III) telle que définie ci-dessus avec $R_3$ représentant un atome d'hydrogène et Y représente OH, $OCH_2CH_2P(O)(OH)_2$ ou $OCH_2CH_2P(O)(OCH_3)_2$, notamment il s'agit du pentaérythritol triazide. Lorsque p est égal à 0, le procédé selon l'invention peut alors être représenté schématiquement comme suit :

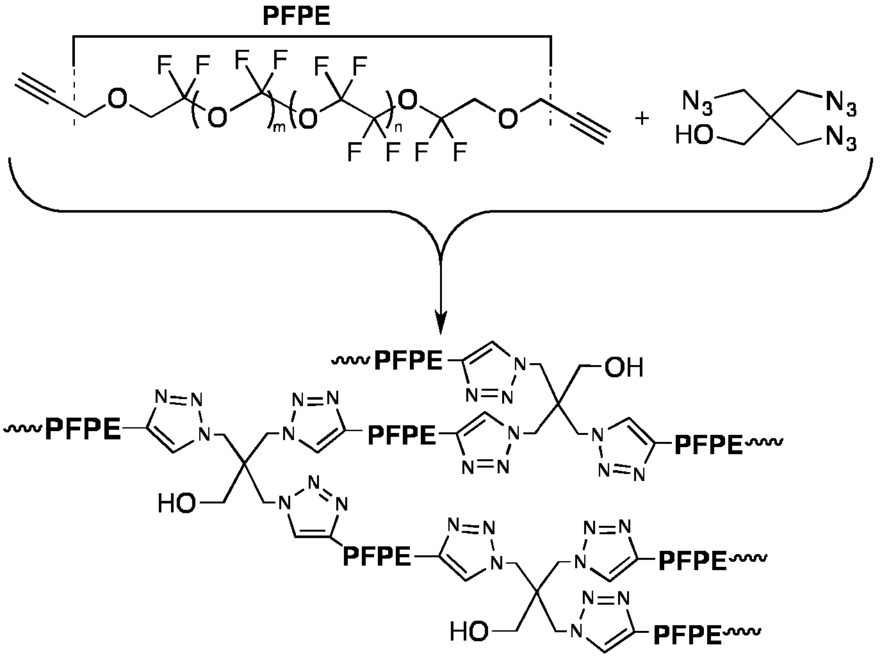

Réticulation par chimie « click » entre un oligomère α,ω-bis(propargylique) fluoré de formule (I) et le pentaérythritol triazide.

Dans ce mode de réalisation particulier, le procédé selon l'invention est de préférence mis en œuvre sans solvant.

De préférence, dans ce mode de réalisation, la réaction de chimie « click » est conduite à pression atmosphérique et à une température comprise entre 80 °C et 200 °C, de préférence entre 100°C et 200°C, notamment entre 140 °C et 180 °C. En outre, dans ce mode de réalisation, la réaction est de préférence conduite sans solvant. Les composants de la composition réticulable sont mélangés puis chauffés à une température permettant la mise en oeuvre de la réaction click, par exemple environ 160 °C.

Dans un autre mode de réalisation particulier, le procédé de préparation du matériau selon l'invention comprend une étape de réticulation par chimie « click » entre :

i) un oligomère α,ω-bis(propargylique) fluoré de formule (I) telle que définie ci-dessus, notamment avec p égal à 0 ou 1.75; et

ii) un composé de formule (III) telle que définie ci-dessus avec de préférence $R_3$ représentant un atome d'hydrogène et Y représente OH, $OCH_2CH_2P(O)(OH)_2$ ou $OCH_2CH_2P(O)(OCH_3)_2$, notamment le pentaérythritol triazide : qu'agent de réticulation ; et

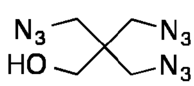
,

en tant

iii) un oligomère α,ω-bis(azidé) fluoré de formule (V) telle que définie ci-dessus, notamment avec p' égal à 0 ou

1.75. De préférence, p' est égal à p.

De préférence, dans ce mode de réalisation, la réaction de chimie « click » est conduite à pression atmosphérique et à une température comprise entre 100 °C et 200 °C, de préférence entre 140 °C et 180 °C. En outre, dans ce mode de réalisation, la réaction est de préférence conduite sans solvant. Les deux ou trois composants du mélange sont mélangés puis chauffés à une température permettant la mise en oeuvre de la réaction click, par exemple environ 160 °C.

Dans un troisième mode de réalisation particulier, le procédé de préparation du matériau selon l'invention comprend une étape de réticulation par chimie « click » entre :

i) un oligomère $\alpha,\omega$-bis(propargylique) fluoré de formule (I) telle que définie ci-dessus, notamment avec p égal à 0 ou 1.75; et

ii) un composé de formule (III) telle que définie ci-dessus avec de préférence $R_3$ représentant un atome d'hydrogène et Y représente OH, $OCH_2CH_2P(O)(OH)_2$ ou $OCH_2CH_2P(O)(OCH_3)_2$, notamment le pentaérythritol triazide : qu'agent de réticulation; et

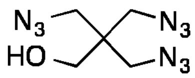

en tant

iii) un oligomère $\alpha,\omega$-bis(azidé) fluoré de formule (VI) telle que définie ci-dessus.

De préférence, dans ce mode de réalisation, la réaction de chimie « click » est conduite à pression atmosphérique et à une température comprise entre 100 °C et 200 °C, de préférence entre 140 °C et 180 °C. En outre, dans ce mode de réalisation, la réaction est de préférence conduite sans solvant. Les deux ou trois composants du mélange sont mélangés puis chauffés à une température permettant la mise en oeuvre de la réaction click, par exemple environ 160 °C.

Dans ces trois modes de réalisation particuliers, les proportions molaires respectives des composés (i), (ii) et (iii) sont telles que le nombre total de groupes propargyliques ($-CH_2-C\equiv CH$) est égal au nombre total de groupes azotures $-N_3$.

Ainsi, dans le premier mode de réalisation particulier, les proportions molaires respectives des composés (i) : (ii) sont égales à 3:2.

Dans les deuxième et troisième modes de réalisation particuliers, les proportions relatives d'agent de réticulation comprenant trois groupes azotures $-N_3$ (ii) et d'oligomère $\alpha,\omega$-bis(azidé) fluoré (iii) sont par exemple telles que 20%, 40%, 60% ou 80% (en moles) des groupes azotures $-N_3$ sont apportés par l'agent de réticulation comprenant trois groupes azotures $-N_3$, par rapport au nombre total des groupes azotures.

*Utilisations*

La présente invention a également trait à l'utilisation du matériau selon l'invention en tant qu'isolant électrique, notamment en milieu sévère, et/ou à haute température, en particulier à une température supérieure à 250 °C, voire supérieure à 300 °C. La possibilité de synthétiser puis produire des matrices dotées potentiellement d'une faible valeur de Tg permet également leur utilisation en tant que matrices souples à basse température jusqu'à -100 °C.

Les matériaux selon l'invention sont en particulier utiles dans le domaine des systèmes embarqués, car ils sont non contraignants sur le plan mécanique (du fait de leur souplesse) et chimique (grande stabilité notamment en milieu sévère et/ou à haute température et/ou en milieu humide) pour les éléments électriques ou électroniques qui doivent être recouverts par lesdits matériaux, qui agissent à la fois en tant qu'agents protecteurs et isolants électriques.

Ainsi, les matériaux selon l'invention sont notamment utiles en tant qu'encapsulants de cartes électroniques dans les modules de puissance ou systèmes embarqués, revêtements de machines tournantes ou moteurs électriques, éléments d'emballage semi-rigides, connectiques.

Les matériaux selon l'invention trouvent typiquement des applications en tant qu'éléments d'étanchéité se trouvant sur des aéronefs, notamment sur divers organes de vols et qui peuvent se décliner sous la forme de cordons d'étanchéité, de joints toriques, d'éléments de corps ou encore de connectiques de pompe.

On peut également mentionner des applications dans le secteur du génie chimique, en particulier du transport, du stockage et de la manipulation de produits corrosifs tels que les hydrocarbures, notamment en tant que joints d'étanchéité, de revêtement de réacteur ou de conteneur de stockage.

En résumé, les matériaux selon l'invention présentent les avantages suivants :

- leur synthèse est aisée et facile à mettre en oeuvre, notamment à l'échelle industrielle, à la fois en termes de sécurité (absence de sous-produits toxiques lors de la réaction de réticulation), de cinétiques de réaction, de conditions réactionnelles, de rendements (le rendement de la réaction de réticulation est très élevé voire quantitatif), ce qui rend leur coût de fabrication acceptable pour des polymères fluorés,
- une grande modularité des propriétés des matériaux, apportée notamment par la quantité d'agent réticulant (ii), et la séquence $\mathbf{R_F}$ de l'oligomère fluoré $\alpha,\omega$-bis(azidé) (iii), permettant de produire à façon des matériaux thermodurcissables ou élastomériques pour des applications variées en tant qu'isolants électriques notamment.

**PRESENTATION DES FIGURES**

La figure 1 représente le spectre RMN $^{19}F$ dans le méthanol deutéré (MeOH-$d_4$) du poly(oxyde de tétrafluoroéthylène-*co*-oxyde de difluorométhylène)-$\alpha,\omega$-diol (Fomblin® Z-DOL), ainsi que le calcul de la masse molaire moyenne en nombre $M_n$ (1200 g.mol$^{-1}$). L'axe des abscisses représente les déplacements chimiques en ppm.

La figure 2 représente une superposition des spectres RMN $^{19}F$ dans le méthanol deutéré (MeOH-$d_4$) du poly(oxyde de tétrafluoroéthylène-*co*-oxyde de difluorométhylène)-$\alpha,\omega$-diol (Fomblin® Z-DOL) et du PFPE-dialcyne éther de l'exemple 1. L'axe des abscisses représente les déplacements chimiques en ppm.

La figure 3 représente le spectre RMN $^{1}H$ dans le méthanol deutéré (MeOH-$d_4$) du PFPE-dialcyne éther de l'exemple 1. L'axe des abscisses représente les déplacements chimiques en ppm.

La figure 4 représente une superposition des spectres infrarouge (FTIR) du poly(oxyde de tétrafluoroéthylène-*co*-oxyde de difluorométhylène)-$\alpha,\omega$-diol (Fomblin® Z-DOL) et du PFPE-dialcyne éther de l'exemple 1. L'axe des abscisses représente le nombre d'ondes (en cm$^{-1}$), et l'axe des ordonnées représente la transmittance (en %).

La figure 5 représente une superposition des spectres infrarouge à transformée de Fourier (FTIR) du pentaérythritol triazide de l'exemple 5, ainsi que du PFPE-dialcyne éther de l'exemple 1 et du matériau « binaire » de l'exemple 7.1, ainsi que le spectre IR du composé tribromé précurseur du pentaérythritol triazide. L'axe des abscisses représente le nombre d'ondes (en cm$^{-1}$), et l'axe des ordonnées représente la transmittance (en %).

La figure 6 représente une superposition des thermogrammes de thermogravimétrie (TGA) à 10 °C par minute sous air, du matériau « binaire » de l'exemple 7.1 et de ses précurseurs, le pentaérythritol triazide de l'exemple 5, et le PFPE-dialcyne éther de l'exemple 1. L'axe des abscisses représente la température (en °C), et l'axe des ordonnées représente le poids (en %).

La figure 7 représente le thermogramme d'analyses de calorimétrie différentielle à balayage (DSC) du matériau « binaire » de l'exemple 7.1. L'axe des abscisses représente la température (en °C), et l'axe des ordonnées représente le flux de chaleur par unité de masse (en mW/mg).

La figure 8 représente une superposition des spectres RMN $^{19}F$ dans le méthanol deutéré (MeOH-$d_4$) du poly(oxyde de tétrafluoroéthylène-co-oxyde de difluorométhylène)-$\alpha,\omega$-diol (Fomblin® Z-DOL) et du $\alpha,\omega$-bis(tosylate) PFPE de l'étape 1 de l'exemple 2. L'axe des abscisses représente les déplacements chimiques en ppm.

La figure 9 représente une superposition des spectres RMN $^{1}H$ dans le méthanol deutéré (MeOH-$d_4$) du poly(oxyde de tétrafluoroéthylène-*co*-oxyde de difluorométhylène)-$\alpha,\omega$-diol (Fomblin® Z-DOL) et du $\alpha,\omega$-bis(tosylate) PFPE de l'étape 1 de l'exemple 2. L'axe des abscisses représente les déplacements chimiques en ppm.

La figure 10 représente une superposition des spectres RMN $^{19}F$ dans le méthanol deutéré (MeOH-$d_4$) du $\alpha,\omega$-bis(tosylate) PFPE de l'étape 1 de l'exemple 2 et du $\alpha,\omega$-bis(azido) PFPE de l'étape 2 de l'exemple 2. L'axe des abscisses représente les déplacements chimiques en ppm.

La figure 11 représente une superposition des spectres RMN $^{1}H$ dans le méthanol deutéré (MeOH-$d_4$) du $\alpha,\omega$-bis(tosylate) PFPE de l'étape 1 de l'exemple 2 et du $\alpha,\omega$-bis(azido) PFPE de l'étape 2 de l'exemple 2. L'axe des abscisses représente les déplacements chimiques en ppm.

La figure 12 représente une superposition des spectres IR (FTIR) du $\alpha,\omega$-bis(tosylate) PFPE de l'étape 1 de l'exemple 2 et du $\alpha,\omega$-bis(azido) PFPE de l'étape 2 de l'exemple 2. L'axe des abscisses représente le nombre d'ondes (en cm$^{-1}$), et l'axe des ordonnées représente la transmittance (en %).

La figure 13 représente une superposition des thermogrammes de thermogravimétrie (TGA) à 10 °C par minute sous air, du matériau « ternaire » de l'exemple 7.2 et de ses précurseurs, le pentaérythritol triazide de l'exemple 5, et le PFPE-dialcyne éther de l'exemple 1. L'axe des abscisses représente la température (en °C), et l'axe des ordonnées représente le poids (en %).

La figure 14 représente le thermogramme d'analyse calorimétrique différentielle à balayage (DSC) du matériau « ternaire » de l'exemple 7.2. L'axe des abscisses représente la température (en °C), et l'axe des ordonnées représente le flux de chaleur (en mW/mg).

La figure 15 représente le thermogramme d'analyse calorimétrique différentielle à balayage (DSC) des matériaux « ternaires » LG-75, LG-76, LG-77 et LG-78 de l'exemple 7.3. L'enthalpie de réticulation est donnée en J/g. L'axe

des abscisses représente la température (en °C), et l'axe des ordonnées représente le flux de chaleur (en mW/mg).

La figure 16 est une représentation schématique des différences de structure des matériaux réticulés selon l'invention en fonction de la proportion d'agent de réticulation (ii) et d'oligomère $\alpha,\omega$-bis(azidé) (iii) dans la composition réticulable de départ. La flèche indique le sens croissant de la proportion en agent de réticulation (ii). La structure d'un point de réticulation est indiquée sous la flèche.

La figure 17 décrit l'évolution cinétique (160 °C) des modules viscoélastiques de la formulation réactive binaire (i)+(ii) dans un ratio molaire 3:2. L'axe des abscisses représente le temps (en min), et l'axe des ordonnées représente les modules G' et G" (en Pa).

La figure 18 représente le spectre RMN $^{19}F$ dans l'acétone deutéré ($(CH_3)_2CO$-d6) du Fluorolink® E10H. L'axe des abscisses représente les déplacements chimiques en ppm.

La figure 19 représente une superposition des spectres RMN $^{1}H$ dans l'acétone deutéré ($(CH_3)_2CO$-d6) du Fluorolink® E10H et du PFPE-dialcyne éther A' de l'exemple 3. L'axe des abscisses représente les déplacements chimiques en ppm.

La figure 20 représente une superposition des spectres IR (FTIR) du Fluorolink® E10H et du PFPE-dialcyne éther A' de l'exemple 3. L'axe des abscisses représente le nombre d'ondes (en $cm^{-1}$), et l'axe des ordonnées représente la transmittance (en %).

La figure 21 représente une superposition des spectres RMN $^{1}H$ dans l'acétone deutéré ($(CH_3)_2CO$-d6) du Fluorolink® E10H et du $\alpha,\omega$-bis(tosylate) PFPE de l'étape 1 de l'exemple 4. L'axe des abscisses représente les déplacements chimiques en ppm.

La figure 22 représente une superposition des spectres RMN $^{1}H$ dans l'acétone deutéré ($(CH_3)_2CO$-d6) du $\alpha,\omega$-bis(tosylate) PFPE de l'étape 1 de l'exemple 4 et du $\alpha,\omega$-bis(azido) PFPE B' de l'étape 2 de l'exemple 4 L'axe des abscisses représente les déplacements chimiques en ppm.

La figure 23 représente une superposition des spectres IR (FTIR) du Fluorolink® E10H, du $\alpha,\omega$-bis(tosylate) PFPE de l'étape 1 de l'exemple 4, et du $\alpha,\omega$-bis(azido) PFPE B' de l'étape 1 de l'exemple 4. L'axe des abscisses représente le nombre d'ondes (en $cm^{-1}$), et l'axe des ordonnées représente la transmittance (en %).

La figure 24 représente les thermogrammes d'analyses calorimétriques différentielles à balayage (DSC) des mélanges A+C (1/0.67, mol/mol) et A+C+C' (1/0.603/0.067). L'enthalpie de réticulation est donnée en J/g. L'axe des abscisses représente la température (en °C), et l'axe des ordonnées représente le flux de chaleur (en mW/mg). trait plein : mélange A+C+C' ; pointillés : mélange A+C.

## EXEMPLES

La présente invention est illustrée par les exemples ci-dessous, qui ne sauraient toutefois être considérés comme limitatifs.

Abréviations :

| | |
|---|---|
| Eq. | Equivalent molaire |
| RMN | Résonance Magnétique Nucléaire |
| IR | Infrarouge |
| FTIR | Infrarouge à Transformée de Fourrier |
| DMSO | Diméthylsulfoxyde |
| DMF | Diméthylformamide |
| DSC | Calorimétrie différentielle à balayage |
| TGA | AnalyseThermoGravimétrique |

**Exemple 1: Synthèse du PFPE-dialcyne éther (Oligomère A)**

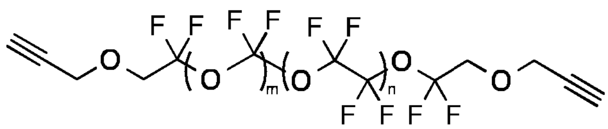

Le poly(oxyde de tétrafluoroéthylène-co-oxyde de difluorométhylène)-$\alpha,\omega$-diol (Fomblin® Z-DOL, $M_n$ 1200 $g.mol^{-1}$ (FIG.1), 20 g, 16,7 mmol, 1 eq.) est ajouté à un mélange de $CH_3CN$ (80 mL) et de THF (80 mL), contenant également de d'hydroxyde de sodium (3,2 g, 83,5 mmol, 5 eq.). Cette suspension est chauffée à 55 °C sous atmosphère d'azote. On ajoute du bromure de propargyle (solution à 80 % en poids dans le toluène, 10 mL, 83,5 mmol, 5 eq.) au mélange réactionnel. Celui-ci est chauffé à 55 °C sous agitation forte pendant 3 jours (> 250 tours/min). Le mélange réactionnel est ensuite refroidi, filtré sur un fritté moyen et le solvant est évaporé. Le produit brut est séché sous vide ($90.10^{-3}$ mbar) puis purifié par filtration à travers un filtre en polyéthersulfone 0,22 mm. On obtient 17,5 g (82 %) d'une huile visqueuse marron clair.

Les spectres RMN $^{19}F$ et $^{1}H$ sont présentés sur les FIG.2 et FIG.3, respectivement, et le spectre IR (FTIR) sur la FIG.4.

**Exemple 2: Synthèse du a,w-bis(azido) PFPE (Oligomère B)**

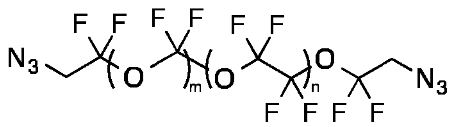

*Etape 1. Synthèse du $\alpha,\omega$-bis(tosylate) PFPE.*

Le poly(oxyde de tétrafluoroéthylène-co-oxyde de difluorométhylène)-$\alpha,\omega$-diol (Fomblin® Z-DOL, $M_n$ 1200 $g.mol^{-1}$, 1 g, 0,83 mmol, 1 eq.) est dissous dans un mélange de $\alpha,\alpha,\alpha$-trifluorotoluène (10 mL) et de triéthylamine (210 mg, 2 mmol, 2,5 eq.). Du chlorure de tosyle (400 mg, 2 mmol, 2,5 eq.) est ajouté au mélange réactionnel, qui est chauffé à 55 °C sous agitation forte pendant 24h. Ensuite, 10mL d'eau et 1 mL de MeOH sont ajoutés au mélange réactionnel. La phase hydroalcoolique est éliminée, et le produit brut est séché sous pression réduite ($90.10^{-3}$ mbar) à 100 °C. On obtient 1,15 g (92%) d'une huile brun clair. Les spectres RMN $^{19}F$ et $^{1}H$ sont représentés sur les FIG.8 et FIG.9 respectivement, et le spectre infrarouge (FTIR) sur la FIG.12.

*Etape 2. Synthèse du $\alpha,\omega$-bis(azido) PFPE*

Un mélange d' $\alpha,\omega$-bis(tosylate) PFPE (1 g, 0,66 mmol, 1 eq.), de $NaN_3$ (260 mg, 3,98 mmol, 6 eq.), et de DMSO (20 mL) est agité à 110 °C pendant 3 jours. Le mélange réactionnel est ensuite versé dans l'eau (100 mL), puis extrait au 1,1,1,3,3-pentafluorobutane (3x50 mL). Les phases organiques sont combinées, lavées avec de l'eau (3x50 mL), séchées ($MgSO_4$), filtrées et concentrées sous vide. On obtient 770 mg (93 %) d'une huile marron clair.

Les spectres RMN $^{19}F$ et $^{1}H$ sont représentés sur les FIG.10 et FIG.11 respectivement, et le spectre infrarouge (FTIR) sur la FIG.12.

**Exemple 3: Synthèse du PFPE-dialcyne éther (Oligomère A')**

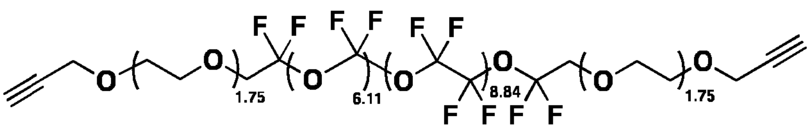

Le Fluorolink® E10H (FIG.19, 1800 $g.mol^{-1}$, 100 g, 55,6 mmol, 1 eq.) est ajouté à un mélange de $CH_3CN$ (150 mL) et de THF (150 mL), contenant également de l'hydroxyde de sodium (16 g, 400 mmol, 7,2 eq.). Cette suspension est chauffée à 55 °C sous atmosphère d'azote. On ajoute du bromure de propargyle (solution à 80 % en poids dans le toluène, 50 mL, 449 mmol, 8,1 eq.) au mélange réactionnel. Celui-ci est chauffé à 55 °C sous agitation forte (> 250 tours/min) pendant 7 jours. Le mélange réactionnel est ensuite refroidi, filtré sous vide et le solvant est évaporé. Le produit brut est séché sous vide ($20.10^{-3}$ mbar) à 100 °C puis purifié par filtration à travers un filtre en PTFE 0,45 $\mu$m. On obtient 86 g (environ 85 %) d'une huile marron clair. Le spectre RMN $^{1}H$ est présenté sur la FIG.19, et le spectre IR (FTIR) sur la FIG.20. L'analyse par calorimétrie différentielle à balayage a révélé une température de transition vitreuse de -100 °C.

**Exemple 4: Synthèse du a,w-bis(azido) PFPE (Oligomère B')**

*Etape 1. Synthèse du $\alpha,\omega$-bis(tosylate) PFPE*

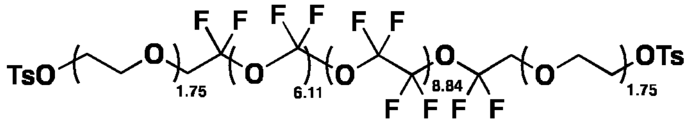

Le Fluorolink® E10H (1800 g.mol$^{-1}$, 100 g, 55,6 mmol, 1 eq.) est dissous dans un mélange de 1,1,1,3,3-pentafluorobutane (300 mL) et de triéthylamine (28 g, 277 mmol, 5 eq.). Du chlorure de tosyle (53 g, 278 mmol, 5 eq.) est ajouté au mélange réactionnel, qui est chauffé à 30 °C sous agitation forte pendant 7 jours. La phase fluorée est ensuite lavée à l'eau (3x300 mL), séchée sur $MgSO_4$, filtrée, puis évaporée sous pression réduite. Le produit brut est ensuite séché sous pression réduite (20.10$^{-3}$ mbar) à 100 °C. On obtient 70 g (environ 70 %) d'une huile marron clair. Le spectre RMN $^1H$ est présenté sur la FIG.21, et le spectre IR (FTIR) sur la FIG.23.

*Etape 2. Synthèse du $\alpha,\omega$-bis(azido) PFPE B'*

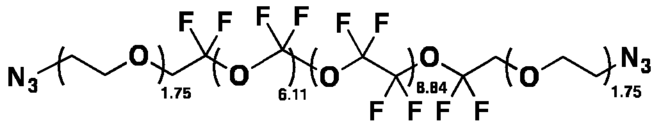

Un mélange d'$\alpha,\omega$-bis(tosylate) PFPE (70 g, 38,9 mmol, 1 eq.) synthétisé lors de la première étape, de $NaN_3$ (27 g, 415 mmol, 10,7 eq.), et de DMSO (300 mL) est agité à 100 °C pendant 7 jours. Le mélange réactionnel est ensuite versé dans l'eau (300 mL), puis extrait au 1,1,1,3,3-pentafluorobutane (3x150 mL). Les phases organiques sont combinées, lavées avec de l'eau (3x150 mL), séchées ($MgSO_4$), filtrées et concentrées sous vide. Le produit brut est ensuite séché sous pression réduite (20.10$^{-3}$ mbar) à 100 °C. On obtient 42 g (environ 60 %) d'une huile marron clair. Le spectre RMN $^1H$ est présenté sur la FIG.22, et le spectre IR (FTIR) sur la FIG.23. L'analyse par calorimétrie différentielle à balayage a révélé une température de transition vitreuse de -105 °C et une température de dégradation d'environ 180 °C.

**Exemple 5: Synthèse du Pentaérythritol triazide (agent de réticulation C)**

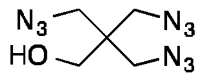

Cette synthèse emploie comme composé de départ le 3-Bromo-2,2-bis(bromométhyl) propanol (produit commercial disponible par exemple chez ABCR), selon un protocole décrit notamment dans Dalton Trans. 2012, 41, 4335.; Biomaterials 2014, 35, 2322.; Chem. Commun. 2007, 380.; WO2012131278.

Un mélange de 3-Bromo-2,2-bis(bromométhyl) propanol (10 g, 31 mmol, 1 eq.), de $NaN_3$ (12 g, 186 mmol, 6 eq.), et de DMSO (30 mL) est mélangé à 100 °C pendant 2 jours. Le mélange réactionnel est ensuite versé dans de l'eau (200 mL), puis extrait au chloroforme $CHCl_3$ (3x100 mL). Les phases organiques sont combinées puis lavées à l'eau (3x100 mL), séchées ($MgSO_4$), filtrées et concentrée sous pression réduite pour donner 5,70 g (87 %) d'une huile jaune pâle.

RMN $^1H$ ($CDCl_3$), $\delta$ (ppm) : 2.20 (br. s, 1H, -OH); 3.36 (s, 6H, -$CH_2$-$N_3$); 3.52 (s, 2H, - $CH_2$-OH).

FTIR-ATR : 2100 cm$^{-1}$ ($\nu_{N3}$); 3400 cm$^{-1}$ ($\nu_{OH}$).

**Exemple 6: agent réticulant phosphoré (agent de réticulation C')**

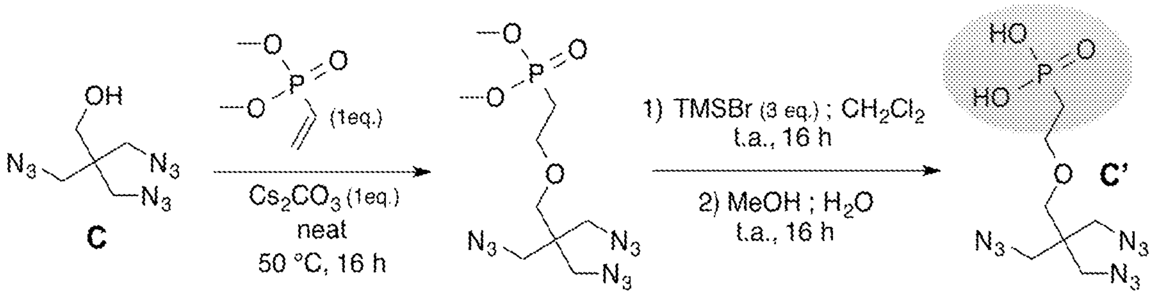


C

LG-124
Rdt 23 %

C'

LG-125
Rdt 100 %

Le pentaérythritol triazide (2 g, 9,47 mmol, 1 eq.) est chauffé 16 h à 50 °C en présence de diméthyl vinylphosphonate (1,29 g, 9,47 mmol, 1 eq.) et de carbonate de césium (3,08 g, 9,47 mmol, 1 eq.). Le milieu réactionnel est ensuite dilué avec de l'eau (100 mL) puis extrait par de l'acétate d'éthyle (3x100 mL). Les phases organiques sont rassemblées, séchées ($MgSO_4$), puis évaporées sous pression réduite. Le résidu est purifié par chromatogrpahie sur colonne de gel de silice (éluant: dichlorométhane/acétate d'étyle, 90/10) pour conduire à 760 mg (23 %) d'une huile incolore.

RMN $^1$H ($CDCl_3$), δ (ppm) : 2.09 (dt, 2H, P-$CH_2$, $^2J_{HP}$ = 18.6 Hz, $^3J_{HH}$ = 7.3 Hz); 3.28 (s, 2H, O-$CH_2$-C($CH_2N_3$)$_3$; 3.32 (s, 6H, O-$CH_2$-C($CH_2N_3$)$_3$; 3.68 (dt, 2H, P-$CH_2$-$CH_2$, $^3J_{HP}$ = 13.2 Hz, $^3J_{HH}$ = 7.3 Hz); 3.73 (d, 6H, $CH_3O$-, $^3J_{HP}$ = 10.9 Hz).

RMN $^{13}$C ($CDCl_3$), δ (ppm) : 25.9 (d, P-$CH_2$, $^1J_{CP}$ = 140.4 Hz); 45.0 (C($CH_2N_3$)$_3$); 51.2 (C($CH_2N_3$)$_3$); 52.5 (d, $CH_3O$, $^2J_{CP}$ = 6.5 Hz); 65.3 (d, P-$CH_2$-$CH_2$, $^2J_{CP}$ = 1.8 Hz); 69.3 (O-$CH_2$-C($CH_2N_3$)$_3$).

RMN $^{31}$P (CDCl3), δ (ppm) : 30.7

Cette dernière est diluée dans du dichlorométhane (10 mL) puis du bromure de triméthylsilyle (environ 0,5 mL) est ajouté goutte à goutte. Le milieu réactionnel est abandonné 16 h à température ambiante avant d'être concentré sous pression réduite. Le résidu est ensuite dilué avec un mélange méthanol/eau (10 mL/10 mL) pendant 16 h à température ambiante. Après évaporation, une huile légèrement jaune est obtenue (420 mg, 100%).

RMN $^1$H ($CDCl_3$), δ (ppm) : 2.15 (dt, 2H, P-$CH_2$, $^2J_{HP}$ = 16.5 Hz, $^3J_{HH}$ = 7.1 Hz); 3.27 (s, 2H, O-$CH_2$-C($CH_2N_3$)$_3$; 3.29 (s, 6H, O-$CH_2$-C($CH_2N_3$)$_3$; 3.72 (dt, 2H, P-$CH_2$-$CH_2$, $^3J_{HP}$ = 12.8 Hz, $^3J_{HH}$ = 7.1 Hz); 9.49 (br. s, 1H, HO-).

RMN $^{13}$C ($CDCl_3$), δ (ppm) : 26.5 (d, P-$CH_2$, $^1J_{CP}$ = 145.6 Hz); 44.7 (C($CH_2N_3$)$_3$); 51.8 (C($CH_2N_3$)$_3$); 65.0 (P-$CH_2$-$CH_2$); 69.6 (O-$CH_2$-C($CH_2N_3$)$_3$).

RMN $^{31}$P ($CDCl_3$), δ (ppm) : 32.5

**<u>Exemple 7.</u> Matériaux réticulés.**

**7.1. Réticulation par chimie "click" avec le PFPE-dialcyne éther et le Pentaérythritol triazide (matériau « binaire »)**

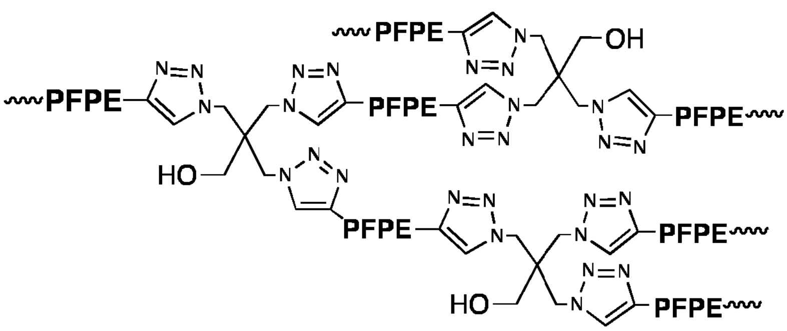

Le PFPE-dialcyne éther (4,55 g, 3,57 mmol, 1 eq.) et le pentaérythritol triazide (810 mg, 3,83 mmol, 1,07 eq.) sont suspendus dans le DMF (20 mL). Le mélange est dégazé par bullage d'azote pendant 30 min. Ensuite, du CuBr (52 mg, 0,036 mmol, 0,1 eq.) et de la *N,N,N',N",N"*-Pentamethyldiéthylènetriamine (PMDETA, 62 mg, 0,036 mmol, 0,1 eq.) sont ajoutés dans le milieu réactionnel qui prend instantanément une couleur verte. Après agitation pendant 1h, le polymère insoluble est lavé à plusieurs reprises avec un mélange DMF:PMDETA (20 mL:1 mL) jusqu'à ce que la solution

de rinçage reste incolore. Le polymère est ensuite séché sous vide à 100 °C jusqu'à l'obtention d'un poids constant (4,40 g, 82 % en poids).

Le polymère est ensuite analysé par spectroscopie infrarouge (FTIR, FIG.5).

L'analyse thermogravimétrique (TGA) révèle une température de décomposition à une valeur de 10% de perte de poids $(T_d^{10\%})$ de 280 °C, sous air. (FIG.6). Une analyse par calorimétrie différentielle à balayage révèle une valeur de température de transition vitreuse ($T_g$) à -87 °C (FIG.7).

**7.2. Réticulation "click" avec le PFPE-dialcyne éther, le a,w-bis(azido) PFPE et le Pentaérythritol triazide (matériau « ternaire »)**

Le PFPE-dialkyne éther (472 mg, 0,37 mmol, 0,525 eq.), le α,ω-bis(azido) PFPE (400 mg, 0,32 mmol, 0,450 eq.) et le pentaérythritol triazide (10 mg, 0,04 mmol, 0,05 eq.) sont suspendus dans le DMF (20 mL). Le mélange est dégazé par bullage d'azote pendant 30 min. Ensuite, du CuBr (10 mg, 0,07 mmol, 0,1 eq.) et de la *N,N,N',N",N"*-Pentamethyldiethylenetriamine (PMDETA, 10 mg, 0,07 mmol, 0,1 eq.) sont ajoutés dans le milieu réactionnel qui prend instantanément un couleur verte. Après agitation pendant 1h, le polymère insoluble est lavé à plusieurs reprises avec un mélange DMF:PMDETA (20 mL:1 mL) jusqu'à ce la solution de rinçage reste incolore. Le polymère est ensuite séché sous vide à 100 °C jusqu'à l'obtention d'un poids constant (800 mg, 91 % en poids).

L'analyse rhéologique cinétique (FIG.17) révèle que le point de gel associé à la production critique du réseau polymère est détecté par la divergence des modules viscoélastiques. Le temps de gel associé est de l'ordre de 6 minutes à la température de 160 °C alors que la réaction semble avoir atteint un degré d'avancement limite au bout de 30 minutes. Ces valeurs soulignent que le mélange présente une réactivité qui est parfaitement compatible avec les attentes de productivité d'une transformation industrielle.

L'analyse thermogravimétrique (TGA) révèle une température de décomposition à une valeur de 10% de perte de poids $(T_d^{10\%})$ de 291 °C, sous air (FIG.13). Une analyse par calorimétrie différentielle à balayage révèle une valeur de température de transition vitreuse ($T_g$) à -100 °C (FIG.14).

**7.3. Formulations ternaires avec le pentaérythritol triazide el les des exemples 1 et 2: variation de la proportion d'agent réticulant**

Quatre matériaux ont été préparés avec des rapports d'oligomères α,ω-bis(azidé)/agent de réticulation (ou agent réticulant, ici le pentaérythritol triazide) variables dans la composition réticulable de départ. Ils sont désignés de la sorte :

<u>LG-75:</u> matériau obtenu par réticulation d'une composition réticulable au sein de laquelle 20% des fonctions azotures sont apportées par l'agent réticulant (i.e. les 80% restants sont apportés par l'oligomère α,ω-bis(azidé), le α,ω-bis(azido) PFPE) ;
<u>LG-76:</u> matériau obtenu par réticulation d'une composition réticulable au sein de laquelle 40% des fonctions azidé sont apportées par l'agent réticulant (i.e. les 60% restants sont apportés par l'oligomère α,ω-bis(azidé), le α,ω-bis(azido) PFPE) ;
<u>LG-77:</u> matériau obtenu par réticulation d'une composition réticulable au sein de laquelle 60% des fonctions azidé sont apportées par l'agent réticulant (i.e. les 40% restants sont apportés par l'oligomère α,ω-bis(azidé), le α,ω-bis(azido) PFPE).
<u>LG-78:</u> matériau obtenu par réticulation d'une composition réticulable au sein de laquelle 80% des fonctions azidé sont apportées par l'agent réticulant (i.e. les 20% restants sont apportés par l'oligomère α,ω-bis(azidé), le α,ω-bis(azido) PFPE).

L'étude comparative du comportement calorimétrique de ces quatre matériaux en fonction de la température est proposée sur la figure 15.

Le matériau LG-75 est celui qui contient le moins d'agent de réticulation. Son domaine réactionnel semble être constitué par deux exothermes.

L'augmentation de la teneur en agent réticulant avec la formulation LG-76 entraine une augmentation de l'enthalpie totale de réticulation. En d'autres termes, la substitution des oligomères α,ω-bis(azidés) par l'agent de réticulation induit un dégagement de chaleur plus important. L'épaulement à haute température se décale vers les températures plus élevées révélant une différence de réactivité entre les deux molécules azidées (agent de réticulation et oligomère α,ω-bis(azidé)) vis-à-vis de l'oligomère α,ω-bis(propargylique).

Les mêmes tendances sont exacerbées avec le mélange le plus riche en agent de réticulation (i.e. formulation LG-78).

Il est important de noter que, en dépit de la différence de réactivité des molécules azidées (agent de réticulation et oligomère α,ω-bis(azidé)), la réaction de réticulation est quantitative. Ainsi, la préparation du mélange en quantités stœchiométriques (i.e. dans les compositions réticulables menant aux matériaux LG-75, LG-76, LG-77 et LG-78, les proportions molaires respectives des oligomères (i), (ii) et (iii) sont telles que le nombre total de groupes propargyliques ($-CH_2-C{\equiv}CH$) égal au nombre total de groupes azotures $-N_3$) assure une consommation totale des espèces réactives.

La $T_g$ la plus faible de l'ordre de -100 °C est quant à elle observée avec la formulation la plus pauvre en agent de réticulation, ce qui est cohérent avec le fait que le réseau est produit par des chaînons perfluorés très souples.

**7.4. Formulations ternaires avec le pentaérythritol triazide et les oligomères A' et B' (exemples 3 et 4): variation de la proportion d'agent réticulant C**

Six matériaux ont été préparés avec des rapports d'oligomères α,ω-bis(azidé)/agent de réticulation (ici le pentaérythritol triazide C) variables dans la composition réticulable de départ (formulations F1 à F6 ci-dessous) :

| nom | Pourcentage de fonctions azide apportées par C |
|---|---|
| F1 | 100% |
| F2 | 80% |
| F3 | 60% |
| F4 | 40% |
| F5 | 20% |
| F6 | 7% |

Les analyses par spectroscopie infrarouge ont confirmé la disparition des bandes caractéristiques des groupements de type alcyne et azide. La polymérisation est donc quantitative. L'augmentation de la teneur en agent réticulant entraine une augmentation de l'enthalpie totale de réticulation (mesurée par analyse calorimétrique différentielle) : H(F1) = 188 J/g, H(F2) = 185 J/g, H(F3) = 172 J/g, H(F4) = 148 J/g, H(F5) = 141 J/g, H(F6) = 137 J/g.

Les analyses rhéologiques cinétiques à 120 °C permettent le suivi de la réaction des trois espèces réactives. Dans tous les cas, les formulations conduisent à des matrices thermodurcissables. Leur temps de gel (associé à durée minimale nécessaire pour la formation critique d'un réseau percolant) est évalué par le temps correspondant à la divergence des modules viscoélastiques. Ils sont respectivement de 0.8, 1.2, 1.8, 3.5, 8.25 et 16 heures, pour les formulations F1, F2, F3, F4, F5, et F6. Autrement dit, le temps de gel augmente lorsque la proportion d'agent réticulant diminue dans la formulation réactionnelle. Dans le même temps, la rigidité mécanique diminue elle aussi avec la teneur décroissante en agent réticulant.

Des analyses calorimétriques menées sur les formulations après consommation totale des espèces réactives démontrent la présence de deux températures de transition vitreuse. Celle enregistrée à basse température est inhérente aux segments (macro)moléculaires PFPE donnés par A ' et/ou B'. Elle est qualifiée de Tg secondaire et reste constante (indépendante de la teneur en agent réticulant) à environ -103 °C. Celle observée à plus haute température, qualifiée de Tg principale du réseau polymère, diminue avec une proportion décroissante d'agent de réticulation du fait d'une maille polymère plus grande et plus flexible. Elle va d'environ -25 °C pour la formulation F1, à environ - 85 °C pour la formulation F6.

Enfin, les analyses thermogravimétriques réalisées sous atmosphère oxydante ont confirmé la formation de matériaux présentant une haute stabilité thermique, avec une valeur de température de décomposition à une valeur de 10% de perte de poids $T_d^{10\%}$ supérieure à 300 °C pour les six compositions F1 à F6.

**Exemple 8 : Polymérisation avec un agent réticulant phosphoré**

L'enthalpie de réticulation d'un mélange binaire A+C est déterminée par DSC, à l'aide du logiciel « Proteus Analysis ». Elle est du même ordre de grandeur que celle d'un mélange A+C contenant 10 % molaire de C' (voir Fig. 24). La présence du groupement phosphoré n'est donc pas en soi un élément perturbateur de la réactivité globale du mélange.

## Revendications

1. Composition réticulable comprenant :

i) un oligomère $\alpha,\omega$-bis(propargylique) fluoré de formule (I) :

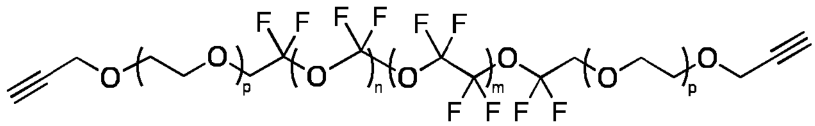

(I)

dans laquelle m est compris entre 1 et 100, par exemple entre 1 et 93,
n est compris entre 2 et 150, par exemple entre 1 et 128, et
p est compris entre 0 et 2, de préférence entre 0 ou 1.75,
n, m et p étant choisis de manière que l'oligomère $\alpha,\omega$-bis(propargylique) fluoré de formule (I) soit de masse molaire moyenne en nombre $M_n$ mesurée par spectroscopie RMN $^{19}F$ comprise entre 400 et 25000;

ii) un agent de réticulation de formule (III) :

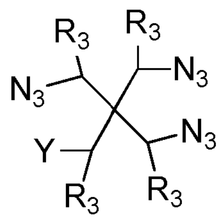

(III)

dans laquelle $R_3$ représente un atome d'hydrogène, un groupe aliphatique en $C_1$-C6, ou un groupe aromatique, et
Y représente un groupe choisi parmi H, OH, un groupe aromatique, un groupe aliphatique en $C_1$-$C_6$, ou un groupe $O(CH_2)SP(O)(OR_4)_2$ avec
s un entier compris entre 2 et 20, de préférence compris entre 2 et 6, de manière encore préférée égal à 2,
$R_4$ représentant H ou un groupe aliphatique en $C_1$-$C_6$,; et

iii) optionnellement un oligomère fluoré comprenant deux groupes terminaux azotures $N_3$, ou oligomère $\alpha,\omega$-bis(azidé) fluoré.

2. Composition réticulable selon la revendication 1, **caractérisée en ce que** les proportions molaires respectives des oligomères (i) et (iii) et de l'agent de réticulation (ii) sont telles que le nombre total de groupes propargyliques ($-CH_2-C\equiv CH$) est égal au nombre total de groupes azotures $-N_3$.

3. Composition réticulable selon la revendication 1 ou 2, **caractérisée en ce que**, dans la formule (III), Y représente OH, $OCH_2CH_2P(O)(OH)_2$ ou $OCH_2CH_2P(O)(OCH_3)_2$.

4. Composition réticulable selon la revendication 3, **caractérisée en ce que** l'agent de réticulation est le pentaérythritol triazide :

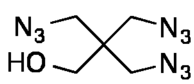

.

5. Composition réticulable selon l'une quelconque des revendications 1 à 4, **caractérisée en ce que** l'oligomère $\alpha,\omega$-bis(azidé) fluoré est représenté par la formule (IV) suivante :

$$N_3-CHR_1(CHR_2)_q-R_F-(CHR_2)_qCHR_1-N_3 \qquad (IV)$$

dans laquelle le radical $R_F$ représente une chaîne fluorée, q est 0 ou 1, et $R_1$ et $R_2$ sont indépendamment choisis parmi un atome d'hydrogène, un alkyle en $C_1$-$C_6$ et un alcényle en $C_2$-$C_6$.

**6.** Composition réticulable selon la revendication 5, **caractérisée en ce que** l'oligomère α,ω-bis(azidé) fluoré est représenté par la formule (V) :

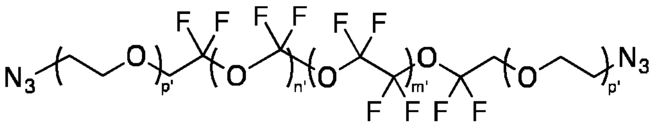
(V),

dans laquelle m' est compris entre 1 et 100, par exemple entre 1 et 93,

n' est compris entre 2 et 150, par exemple entre 1 et 128, et
p' est compris entre 0 et 2, par exemple 0 ou 1.75,
n', et m' et p' étant choisis de manière que l'oligomère α,ω-bis(propargylique) fluoré de formule (I) soit de masse molaire moyenne en nombre $M_n$ mesurée par spectroscopie RMN $^{19}F$ comprise entre 400 et 25000, ou l'oligomère α,ω-bis(azidé) fluoré est représenté par la formule (VI) :

$N_3$-$CHR_1CHR_2$-$(CR_{a1}R_{b1}CR_{c1}R_{d1})_{n1}$-...-$(CR_{ai}R_{bi}CR_{ci}R_{di})_{ni}$-$(CF_2)_4$-$(CR_{ci}R_{di}CR_{ai}R_{bi})_{ni}$-...-$(CR_{c1}R_{d1}CR_{a1}R_{b1})_{n1}$-$CHR_2CHR_1$-$N_3$ (VI),

dans laquelle $R_1$ et $R_2$ sont tels que définis à la revendication 5,
i est compris entre 1 et 20, de préférence entre 1 et 5, de manière encore préférée entre 1 et 3 ou entre 1 et 2,
$CR_{a1}R_{b1}CR_{c1}R_{d1}$ à $CR_{ai}R_{bi}CR_{ci}R_{di}$ sont des motifs constitutionnels, identiques ou différents, issus de monomères indépendamment choisis parmi les oléfines fluorées suivantes : Tétrafluoroéthylène, Fluorure de vinylidène, Hexafluoropropylène, Trifluoroéthylène, Perfluoro(méthyl vinyl éther), 3,3,3-trifluoropropène, 2,3,3,3-tétrafluoropropène, 1,3,3,3-tétrafluoropropène, Chlorotrifluoroéthylène, Bromotrifluoroéthylène, Iodotrifluoroéthylène, 2-chloro-3,3,3-trifluoropropène, Fluorure de vinyle, Perfluoro(éthyl vinyl éther), Perfluoro(propyl vinyléther), 2-bromo-1,1-difluoroéthylène, Chlorodifluoroéthylène, Dichlorodifluoroéthylène, 1,1,3,3,3-Pentafluoropropène, 1,1,2,3,4,4-Hexafluoro-1,3-butadiène, 1-Propene, 1,1,3,3,3-pentafluoro-2-(trifluoromethyl)-1-propène, et leurs dérivés, et
$n_1$ à $n_i$ représentent chacun indépendamment un nombre choisi parmi 1 à 20, de préférence 1 à 5.

**7.** Composition selon l'une quelconque des revendications 1 à 6, **caractérisée en ce que**, dans l'oligomère α,ω-bis(propargylique) fluoré de formule (I), p est égal à 1.75.

**8.** Matériau comprenant le produit de la réaction par chimie « click » de la composition réticulable selon l'une quelconque des revendications 1 à 7 et possédant au moins une valeur température de transition vitreuse inférieure ou égale à -70 °C, la réaction de chimie « click » étant de préférence mise en oeuvre en présence d'un catalyseur au cuivre.

**9.** Matériau selon la revendication 8, **caractérisé en ce qu'**il possède une température de décomposition à une valeur de 10% de perte de poids $(T_d^{10\%})$ supérieure ou égale à 250 °C, mesurée par analyse thermogravimétrique, sous air.

**10.** Matériau selon l'une quelconque des revendications 8 ou 9, **caractérisé en ce qu'**il possède au moins une valeur température de transition vitreuse inférieure ou égale à - 80 °C.

**11.** Procédé de préparation du matériau selon l'une quelconque des revendications 8 à 10, comprenant une étape de réticulation par chimie « click » entre :

i) un oligomère α,ω-bis(propargylique) fluoré de formule (I) :

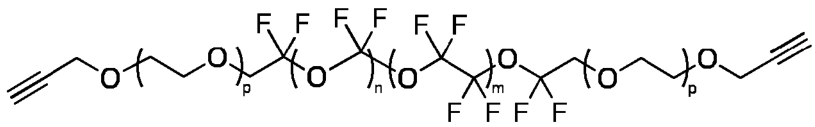

(I)

dans laquelle m est compris entre 1 et 100, par exemple entre 1 et 93,

n est compris entre 2 et 150, par exemple entre 1 et 128, et
p est compris entre 0 et 2, par exemple 0 ou 1.75,
n, m et p étant choisis de manière que l'oligomère α,ω-bis(propargylique) fluoré de formule (I) soit de masse molaire moyenne en nombre $M_n$ mesurée par spectroscopie RMN $^{19}F$ comprise entre 400 et 25000;

ii) un agent de réticulation de formule (III) :

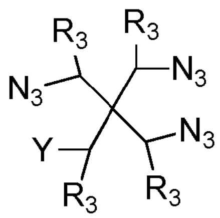

(III)

dans laquelle $R_3$ représente un atome d'hydrogène, un groupe aliphatique en $C_1$-C6, ou un groupe aromatique, et

Y représente un groupe choisi parmi H, OH, un groupe aromatique, un groupe aliphatique en $C_1$-$C_6$, un groupe $O(CH_2)_sP(O)(OR_4)_2$ avec
s un entier compris entre 2 et 20, de préférence compris entre 2 et 6, de manière encore préférée égal à 2,
$R_4$ représentant H ou un groupe aliphatique en $C_1$-$C_6$, notamment un groupe méthyle, éthyle, isopropyle, de préférence méthyle; et

iii) optionnellement un oligomère fluoré comprenant deux groupes terminaux azotures $-N_3$, ou oligomère α,ω-bis(azidé) fluoré.

12. Procédé selon la revendication 11, **caractérisé en ce que** l'étape de réticulation est mise en oeuvre en présence d'un catalyseur au cuivre, avantageusement en une quantité comprise entre 0,001 équivalents molaires et 0,2 équivalents molaires, et de préférence utilisé en combinaison avec un ligand pour améliorer sa réactivité et/ou sa stabilité, notamment la 1,1,4,7,7-pentaméthyldiéthylènetriamine.

13. Utilisation d'un matériau comprenant le produit de la réaction par chimie « click » de la composition réticulable, ladite composition réticulable comprenant :

i) un oligomère α,ω-bis(propargylique) fluoré de formule (I) :

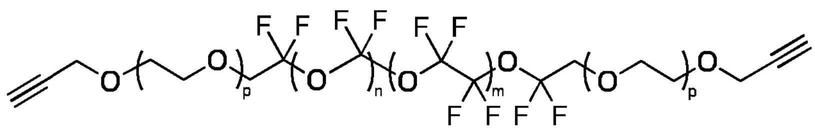

(I)

dans laquelle m est compris entre 1 et 100, par exemple entre 1 et 93,

n est compris entre 2 et 150, par exemple entre 1 et 128, et
p est compris entre 0 et 2, de préférence entre 0 ou 1.75,
n, m et p étant choisis de manière que l'oligomère α,ω-bis(propargylique) fluoré de formule (I) soit de masse molaire moyenne en nombre $M_n$ mesurée par spectroscopie RMN $^{19}F$ comprise entre 400 et 25000;

ii) un agent de réticulation comprenant au moins trois groupes azotures $N_3$; et
iii) optionnellement un oligomère fluoré comprenant deux groupes terminaux azotures $N_3$, ou oligomère α,ω-bis(azidé) fluoré ;
en tant qu'isolant électrique, typiquement d'éléments se trouvant sur des aéronefs, notamment en milieu sévère, et/ou à une température supérieure à 250 °C, voire supérieure à 300 °C.

**14.** Utilisation selon la revendication 13 en tant qu'encapsulant de cartes électroniques dans les modules de puissance ou systèmes embarqués, revêtements de machines tournantes ou moteurs électriques, éléments d'emballage semi-rigides, connectiques.

**15.** Utilisation selon la revendication 13 ou 14, **caractérisée en ce que** le matériau est tel que défini dans l'une quelconque des revendications 8 à 10.

## Patentansprüche

**1.** Vernetzbare Zusammensetzung, umfassend:

i) ein fluoriertes α,ω-Bis(propargyl)-Oligomer der Formel (I):

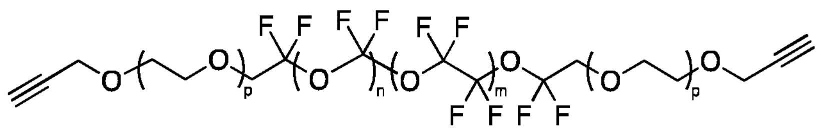
(I)

wobei m zwischen 1 und 100, beispielsweise zwischen 1 und 93 liegt,

n zwischen 2 und 150, beispielsweise zwischen 1 und 128 liegt, und
p zwischen 0 und 2, vorzugsweise zwischen 0 und 1,75 liegt,
n, m und p derart gewählt sind, dass das fluorierte α,ω-Bis(propargyl)-Oligomer der Formel (I) eine zahlenmittlere Molmasse $M_n$, mittels $^{19}$F-NMR-Spektroskopie gemessen, aufweist, die zwischen 400 und 25.000 liegt;

ii) ein Vernetzungsmittel der Formel (III):

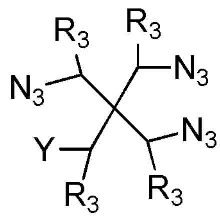
(III)

wobei $R_3$ für ein Wasserstoffatom, eine aliphatische $C_1$-$C_6$-Gruppe oder eine aromatische Gruppe steht, und

Y für eine Gruppe steht, die aus H, OH, einer aromatischen Gruppe, einer aliphatischen $C_1$-$C_6$-Gruppe oder einer $O(CH_2)_sP(O)(OR_4)_2$-Gruppe ausgewählt ist, mit
s eine ganze Zahl ist, die zwischen 2 und 20, vorzugsweise zwischen 2 und 6 liegt, noch mehr bevorzugt gleich 2 ist,
$R_4$ für H oder eine aliphatische $C_1$-$C_6$-Gruppe steht; und

iii) optional ein fluoriertes Oligomer, umfassend zwei $N_3$-Azid-Endgruppen, oder ein fluoriertes α,ω-Bis(azid)-Oligomer.

**2.** Vernetzbare Zusammensetzung nach Anspruch 1, **dadurch gekennzeichnet, dass** die jeweiligen molaren Anteile der Oligomere (i) und (iii) und des Vernetzungsmittels (ii) derart sind, dass die Gesamtanzahl an Propargylgruppen

($-CH_2-C{\equiv}CH$) gleich der Gesamtanzahl von $-N_3$-Azidgruppen ist.

3. Vernetzbare Zusammensetzung nach Anspruch 1 oder 2, **dadurch gekennzeichnet, dass** in der Formel (III) Y für OH, $OCH_2CH_2P(O)(OH)_2$ oder $OCH_2CH_2P(O)(OCH_3)_2$ steht.

4. Vernetzbare Zusammensetzung nach Anspruch 3, **dadurch gekennzeichnet, dass** das Vernetzungsmittel Pentaerythrittriazid ist:

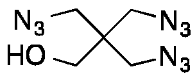
.

5. Vernetzbare Zusammensetzung nach einem der Ansprüche 1 bis 4, **dadurch gekennzeichnet, dass** das fluorierte $\alpha,\omega$-Bis(azid)-Oligomer durch die folgende Formel (IV) dargestellt wird:

$$N_3\text{-}CHR_1(CHR_2)_q\text{-}R_F\text{-}(CHR_2)_qCHR_1\text{-}N_3 \qquad \text{(IV)},$$

wobei das Radikal $R_F$ für eine fluorierte Kette steht, q 0 oder 1 ist, und $R_1$ und $R_2$ unabhängig aus einem Wasserstoffatom, einem $C_1$-$C_6$-Alkyl und einem $C_2$-$C_6$-Alkenyl ausgewählt sind.

6. Vernetzbare Zusammensetzung nach Anspruch 5, **dadurch gekennzeichnet, dass** das fluorierte $\alpha,\omega$-Bis(azid)-Oligomer durch die Formel (V) dargestellt wird:

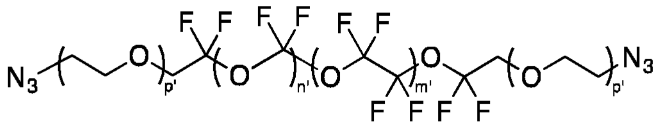
(V),

wobei m' zwischen 1 und 100, beispielsweise zwischen 1 und 93 liegt,

n' zwischen 2 und 150, beispielsweise zwischen 1 und 128 liegt, und
p' zwischen 0 und 2, beispielsweise zwischen 0 und 1,75 liegt,
n', m' und p' derart gewählt sind, dass das fluorierte $\alpha,\omega$-Bis(propargyl)-Oligomer der Formel (I) eine zahlenmittlere Molmasse $M_n$, mittels $^{19}F$-NMR-Spektroskopie gemessen, aufweist, die zwischen 400 und 25.000 liegt, oder
das fluorierte $\alpha,\omega$-Bis(azid)-Oligomer durch die Formel (VI) dargestellt wird:

$$N_3\text{-}CHR_1CHR_2\text{-}(CR_{a1}R_{b1}CR_{c1}R_{d1})_{n1}\text{-}...\text{-}(CR_{ai}R_{bi}CR_{ci}R_{di})_{ni}\text{-}(CF_2)_4\text{-}(CR_{ci}R_{di}CR_{ai}R_{bi})_{ni}\text{-}...\text{-}(CR_{c1}R_{d1}CR_{a1}R_{b1})_{n1}\text{-}CHR_2CHR_1\text{-}N_3 \qquad \text{(VI)},$$

wobei $R_1$ und $R_2$ wie in Anspruch 5 definiert sind,

i zwischen 1 und 20, vorzugsweise zwischen 1 und 5, noch mehr bevorzugt zwischen 1 und 3 oder zwischen 1 und 2 liegt,
$CR_{a1}R_{b1}CR_{c1}R_{d1}$ bis $CR_{ai}R_{bi}CR_{ci}R_{di}$ Bestandsmotive, gleich oder verschieden, sind, die von Monomeren stammen, die unabhängig aus den folgenden fluorierten Olefinen ausgewählt sind: Tetrafluorethylen, Vinylidenfluorid, Hexafluorpropylen, Trifluorethylen, Perfluor(methylvinylether), 3,3,3-Trifluorpropen, 2,3,3,3-Tetrafluorpropen, 1,3,3,3-Tetrafluorpropen, Chlortrifluorethylen, Bromtrifluorethylen, Iodtrifluorethylen, 2-Chlor-3,3,3-trifluorpropen, Vinylfluorid, Perfluor(ethylvinylether), Perfluor(propylvinylether), 2-Brom-1,1-difluorethylen, Chlordifluorethylen, Dichlordifluorethylen, 1,1,3,3,3-Pentafluorpropen, 1,1,2,3,4,4-Hexafluor-1,3-butadien, 1-Propen, 1,1,3,3,3-Pentafluor-2-(trifluormethyl)-1-propen und deren Derivaten, und
$n_1$ bis $n_i$ jeweils unabhängig für eine Zahl stehen, die aus 1 bis 20, vorzugsweise 1 bis 5 ausgewählt ist.

7. Zusammensetzung nach einem der Ansprüche 1 bis 6, **dadurch gekennzeichnet, dass** in dem fluorierten $\alpha,\omega$-Bis(propargyl)-Oligomer der Formel (I) p gleich 1,75 ist.

8. Material, umfassend das "Click"-Chemiereaktionsprodukt der vernetzbaren Zusammensetzung nach einem der

Ansprüche 1 bis 7 und verfügend über mindestens einen Glasübergangstemperaturwert kleiner als oder gleich -70°C, wobei die "Click"-Chemiereaktion vorzugsweise in Gegenwart eines Kupferkatalysators durchgeführt wird.

**9.** Material nach Anspruch 8, **dadurch gekennzeichnet, dass** es über eine Zersetzungstemperatur höher als oder gleich 250 °C mit einem Gewichtsverlustwert $(T_d^{10\%})$ von 10% verfügt, gemessen mittels thermogravimetrischer Analyse an der Luft.

**10.** Material nach einem der Ansprüche 8 oder 9, **dadurch gekennzeichnet, dass** es über mindestens einen Glasübergangstemperaturwert kleiner als oder gleich -80°C verfügt.

**11.** Verfahren zur Herstellung des Materials nach einem der Ansprüche 8 bis 10, umfassend einen Schritt eines Vernetzens durch "Click"-Chemie zwischen:

i) einem fluorierten α,ω-Bis(propargyl)-Oligomer der Formel (I):

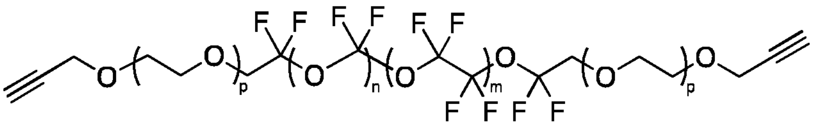

(I)

wobei m zwischen 1 und 100, beispielsweise zwischen 1 und 93 liegt,

n zwischen 2 und 150, beispielsweise zwischen 1 und 128 liegt, und
p zwischen 0 und 2, beispielsweise zwischen 0 und 1,75 liegt,
n, m und p derart gewählt sind, dass das fluorierte α,ω-Bis(propargyl)-Oligomer der Formel (I) eine zahlenmittlere Molmasse $M_n$, mittels $^{19}F$-NMR-Spektroskopie gemessen, aufweist, die zwischen 400 und 25.000 liegt;

ii) einem Vernetzungsmittel der Formel (III):

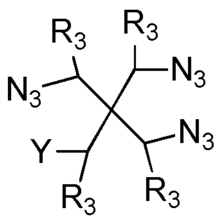

(III)

wobei $R_3$ für ein Wasserstoffatom, eine aliphatische $C_1$-$C_6$-Gruppe oder eine aromatische Gruppe steht, und

Y für eine Gruppe steht, die aus H, OH, einer aromatischen Gruppe, einer aliphatischen $C_1$-$C_6$-Gruppe, einer $O(CH_2)_sP(O)(OR_4)_2$-Gruppe ausgewählt ist, mit
s eine ganze Zahl ist, die zwischen 2 und 20, vorzugsweise zwischen 2 und 6 liegt, noch mehr bevorzugt gleich 2 ist,
$R_4$ für H oder eine aliphatische $C_1$-$C_6$-Gruppe, insbesondere eine Methyl-, Ethyl-, Isopropyl-, vorzugsweise Methylgruppe steht; und

iii) optional einem fluorierten Oligomer, umfassend zwei $N_3$-Azid-Endgruppen, oder einem fluorierten α,ω-Bis(azid)-Oligomer.

**12.** Verfahren nach Anspruch 11, **dadurch gekennzeichnet, dass** der Schritt des Vernetzens in Gegenwart eines Kupferkatalysators durchgeführt wird, vorzugsweise in einer Menge, die zwischen 0,001 molaren Äquivalenten und 0,2 molaren Äquivalenten liegt, und der vorzugsweise in Kombination mit einem Liganden verwendet wird, um seine Reaktivität und/oder seine Stabilität zu verbessern, insbesondere 1,1,4,7,7-Pentamethyldiethylentriamin.

**13.** Verwendung eines Materials, umfassend das "Click"-Chemiereaktionsprodukt der vernetzbaren Zusammensetzung, wobei die vernetzbare Zusammensetzung umfasst:

i) ein fluoriertes α,ω-Bis(propargyl)-Oligomer der Formel (I):

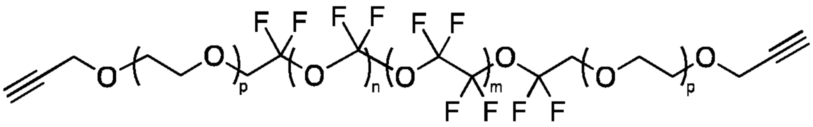

(I)

wobei m zwischen 1 und 100, beispielsweise zwischen 1 und 93 liegt,

n zwischen 2 und 150, beispielsweise zwischen 1 und 128 liegt, und
p zwischen 0 und 2, vorzugsweise zwischen 0 und 1,75 liegt,
n, m und p derart gewählt sind, dass das fluorierte α,ω-Bis(propargyl)-Oligomer der Formel (I) eine zahlenmittlere Molmasse $M_n$, mittels $^{19}F$-NMR-Spektroskopie gemessen, aufweist, die zwischen 400 und 25.000 liegt;

ii) ein Vernetzungsmittel, umfassend mindestens drei $N_3$-Azidgruppen; und
iii) optional ein fluoriertes Oligomer, umfassend zwei $N_3$-Azid-Endgruppen, oder ein fluoriertes α,ω-Bis(azid)-Oligomer;
als Isolator, in der Regel von Elementen, die sich auf Luftfahrzeugen, insbesondere in rauer Umgebung und/oder bei eine Temperatur höher als 250°C, sogar höher als 300°C befinden.

**14.** Verwendung nach Anspruch 13 als Einkapselungsmittel für Elektronikkarten in Leistungsmodulen oder eingebetteten Systemen, Beschichtungen von drehenden Maschinen oder Elektromotoren, halbsteifen Verpackungselementen, Steckverbindern.

**15.** Verwendung nach Anspruch 13 oder 14, **dadurch gekennzeichnet, dass** das Material wie in einem der Ansprüche 8 bis 10 definiert ist.

## Claims

**1.** Crosslinkable composition comprising:

i) a fluorinated α,ω-bis(propargyl) oligomer of formula (I):

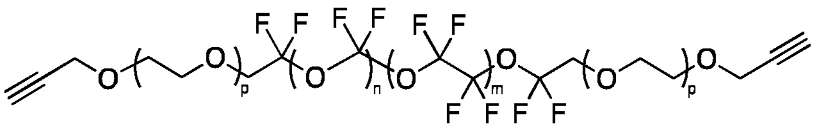

(I)

wherein m is comprised between 1 and 100, for example between 1 and 93,

n is comprised between 2 and 150, for example between 1 and 128, and
p is comprised between 0 and 2, preferably between 0 and 1.75,
n, m and p being chosen so that the fluorinated α,ω-bis(propargyl) oligomer of formula (I) is of number-average molar mass $M_n$ measured by $^{19}F$ NMR spectroscopy comprised between 400 and 25,000;

ii) a crosslinking agent of formula (III):

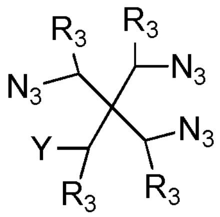

(III)

wherein $R_3$ represents a hydrogen atom, a $C_1$-$C_6$ aliphatic group or an aromatic group, and

Y represents a group chosen from H, OH, an aromatic group, a $C_1$-$C_6$ aliphatic group or an $O(CH_2)_sP(O)(OR_4)_2$ group with
s an integer comprised between 2 and 20, preferably comprised between 2 and 6, still more preferably equal to 2,
$R_4$ representing H or a $C_1$-$C_6$ aliphatic group; and

iii) optionally a fluorinated oligomer comprising two $N_3$ azide terminal groups, or fluorinated α,ω-bis(azide) oligomer.

2. Crosslinkable composition according to claim 1, **characterized in that** the respective molar proportions of the oligomers (i) and (iii) and the crosslinking agent (ii) are such that the total number of propargyl groups ($-CH_2-C\equiv CH$) is equal to the total number of $-N_3$ azide groups.

3. Crosslinkable composition according to claim 1 or 2, **characterized in that**, in formula (III), Y represents OH, $OCH_2CH_2P(O)(OH)_2$ or $OCH_2CH_2P(O)(OCH_3)_2$.

4. Crosslinkable composition according to claim 3, **characterized in that** the crosslinking agent is pentaerythritol triazide:

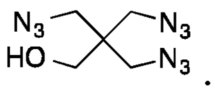

5. Crosslinkable composition according to any one of claims 1 to 4, **characterized in that** the fluorinated α,ω-bis(azide) oligomer is represented by the following formula (IV):

$$N_3\text{-}CHR_1(CHR_2)_q\text{-}R_F\text{-}(CHR_2)_qCHR_1\text{-}N_3 \qquad (IV)$$

wherein the radical $R_F$ represents a fluorinated chain, q is 0 or 1, and $R_1$ and $R_2$ are independently chosen from a hydrogen atom, a $C_1$-$C_6$ alkyl and a $C_2$-$C_6$ alkenyl.

6. Crosslinkable composition according to claim 5, **characterized in that** the fluorinated α,ω-bis(azide) oligomer is represented by formula (V):

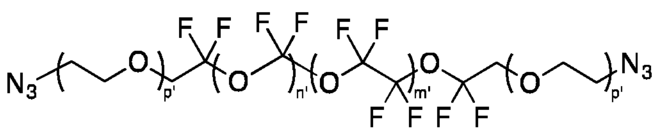

(V),

wherein m' is comprised between 1 and 100, for example between 1 and 93,

n' is comprised between 2 and 150, for example between 1 and 128, and
p' is comprised between 0 and 2, for example 0 and 1.75,
n', m' and p' being chosen so that the fluorinated α,ω-bis(propargyl) oligomer of formula (I) is of number-average molar mass $M_n$ measured by $^{19}F$ NMR spectroscopy comprised between 400 and 25,000, or
the fluorinated α,ω-bis(azide) oligomer is represented by formula (VI):

$N_3\text{-}CHR_1CHR_2\text{-}(CRa_1Rb_1CR_{c1}R_{d1})_{n1}\text{-}...\text{-}(CR_{ai}R_{bi}CR_{ci}R_{di})_{ni}\text{-}(CF_2)4\text{-}(CR_{ci}R_{di}CR_{ai}R_{bi})_{ni}\text{-}...\text{-}(CR_{c1}R_{d1}CR_{a1}R_{b1})_{n1}\text{-}CHR_2CHR_1\text{-}N_3$ (VI),

wherein $R_1$ and $R_2$ are as defined in claim 5,

i is comprised between 1 and 20, preferably between 1 and 5, more preferably between 1 and 3 or between 1 and 2,
$CR_{a1}R_{b1}CR_{c1}R_{d1}$ to $CR_{ai}R_{bi}CR_{ci}R_{di}$ are identical or different constitutional units derived from monomers independently chosen from the following fluorinated olefins: Tetrafluoroethylene, Vinylidene fluoride, Hexafluoropropylene, Trifluoroethylene, Perfluoro(methyl vinyl ether), 3,3,3-trifluoropropene, 2,3,3,3-tetrafluoropropene, 1,3,3,3-tetrafluoropropene, Chlorotrifluoroethylene, Bromotrifluoroethylene, Iodotrifluoroethylene, 2-chloro-3,3,3-trifluoropropene, Vinyl fluoride, Perfluoro(ethyl vinyl ether), Perfluoro(propyl vinyl ether), 2-bromo-1,1-difluoroethylene, Chlorodifluoroethylene, Dichlorodifluoroethylene, 1,1,3, 3,3-Pentafluoropropene, 1,1,2,3,4,4-Hexafluoro-1,3-butadiene, 1-Propene, 1,1,3,3,3-pentafluoro-2-(trifluoromethyl)-1- propene, and their derivatives, and
$n_1$ to $n_i$ independently represent a number chosen from 1 to 20, preferably 1 to 5.

**7.** Composition according to any one of claims 1 to 6, **characterized in that** the fluorinated α,ω-bis(propargyl) oligomer of formula p is equal to 1.75.

**8.** Material comprising the product of the click chemistry reaction of the crosslinkable composition according to any one of claims 1 to 7 and having at least one glass transition temperature value less than or equal to -70°C, the click chemistry reaction being preferably implemented in the presence of a copper catalyst.

**9.** Material according to claim 8, **characterized in that** it has a decomposition temperature at a value of 10% weight loss $(T_d^{10\%})$ greater than or equal to 250°C, measured by thermogravimetric analysis, under air.

**10.** Material according to any one of claims 8 or 9, further **characterized in that** it has at least one glass transition temperature value less than or equal to -80°C.

**11.** Method for preparing a material according to any one of claims 8 to 10, comprising a step of crosslinking by click chemistry between:

i) a fluorinated α,ω-bis(propargyl) oligomer of formula (I):

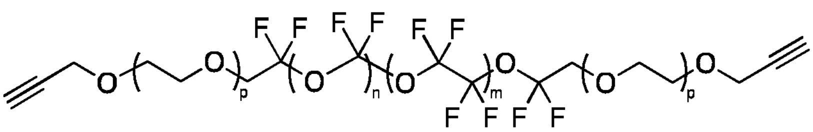

(I)

wherein m is comprised between 1 and 100, for example between 1 and 93,

n is comprised between 2 and 150, for example between 1 and 128, and
p is comprised between 0 and 2, for example 0 or 1.75,
n, m and p being chosen so that the fluorinated α,ω-bis(propargyl) oligomer of formula (I) is of number-average molar mass $M_n$ measured by $^{19}F$ NMR spectroscopy comprised between 400 and 25,000;

ii) a crosslinking agent of formula (III):

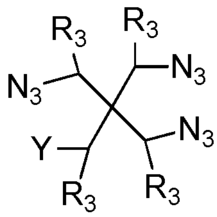

(III)

wherein $R_3$ represents a hydrogen atom, a $C_1$-$C_6$ aliphatic group or an aromatic group, and

Y represents a group chosen from H, OH, an aromatic group, a $C_1$-$C_6$ aliphatic group or an $O(CH_2)_sP(O)(OR_4)_2$ group with
s a whole number comprised between 2 and 20, preferably comprised between 2 and 6, still more preferably equal to 2,
$R_4$ representing H or a $C_1$-$C_6$ aliphatic group, especially a methyl, ethyl, isopropyl group, preferably methyl; and

iii) optionally, a fluorinated oligomer comprising two -$N_3$ azide terminal groups, or fluorinated α,ω-bis(azide) oligomer.

**12.** Method according to claim 11, **characterized in that** the crosslinking step is implemented in the presence of a copper catalyst, advantageously in a quantity comprised between 0.001 molar equivalents and 0.2 molar equivalents, and preferably used in combination with a ligand to improve its reactivity and/or its stability, especially 1,1,4,7,7-pentamethyldiethylenetriamine.

**13.** Use of a material comprising the product of the click chemistry reaction of the crosslinkable composition, said crosslinkable composition comprising:

i) a fluorinated α,ω-bis(propargyl) oligomer of formula (I):

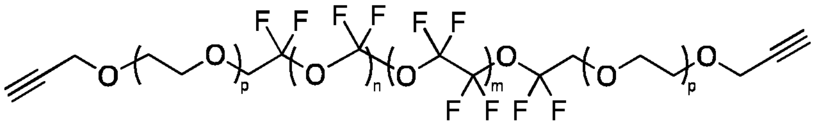

(I)

wherein m is comprised between 1 and 100, for example between 1 and 93,

n is comprised between 2 and 150, for example between 1 and 128, and
p is comprised between 0 and 2, preferably between 0 and 1.75,
n, m and p being chosen so that the fluorinated α,ω-bis(propargyl) oligomer of formula (I) is of number-average molar mass $M_n$ measured by $^{19}F$ NMR spectroscopy comprised between 400 and 25,000;

ii) a crosslinking agent comprising at least three $N_3$ azide groups; and
iii) optionally, a fluorinated oligomer comprising two $N_3$ azide terminal groups, or fluorinated α,ω-bis(azide) oligomer;
as electrical insulator, typically for aircraft components, especially in harsh environments, and/or at a temperature above 250°C, or even above 300°C.

**14.** Use according to claim 13 as encapsulant for electronic cards in power modules or on-board systems, coatings for rotating machines or electric motors, semirigid packaging elements or connectors.

**15.** Use according to claim 13 or 14, **characterized in that** the material is such as defined in any one of claims 8 to 10.

FIG. 1

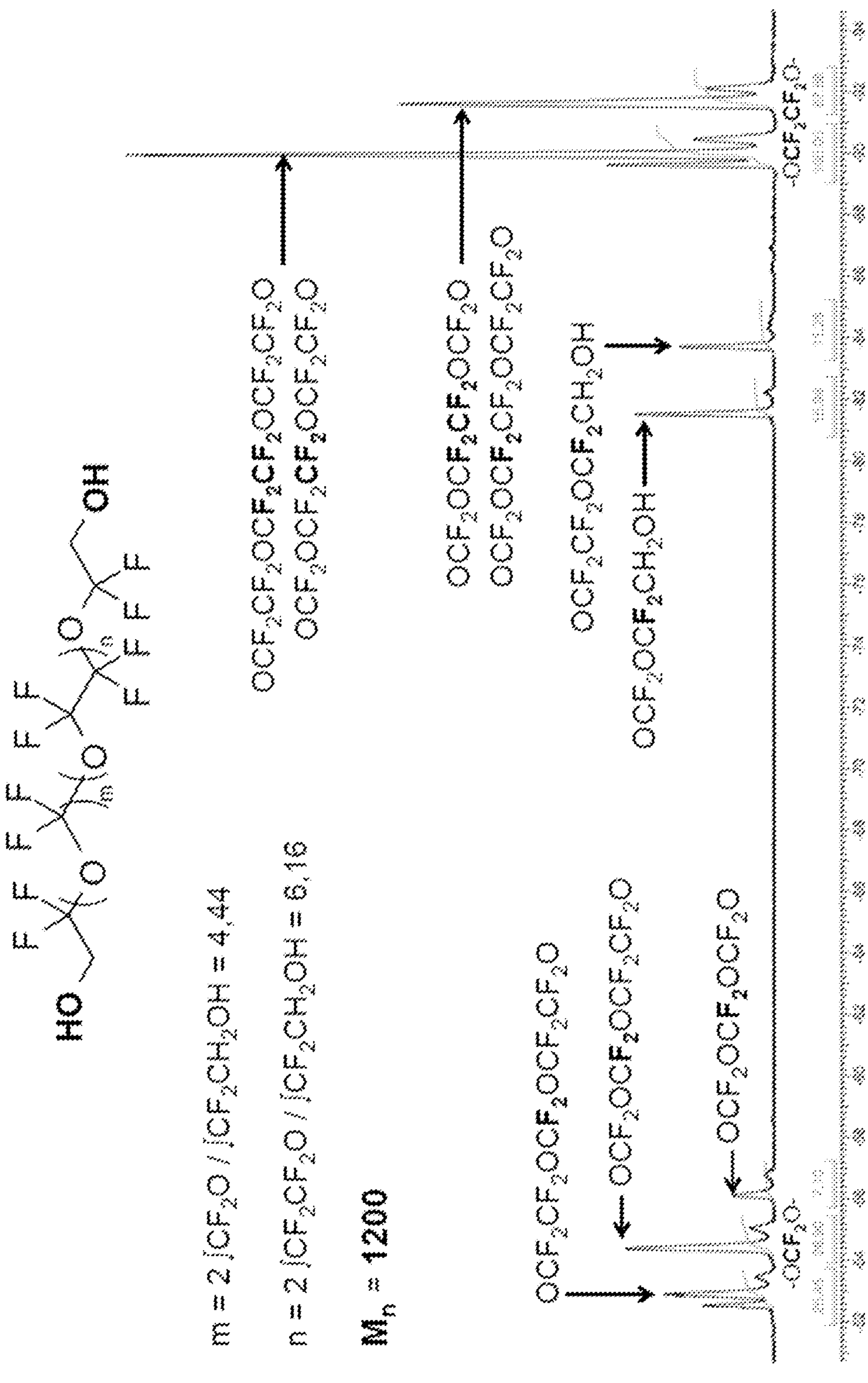
m = 2 ∫CF2O / ∫CF2CH2OH = 4.44
n = 2 ∫CF2CF2O / ∫CF2CH2OH = 6.16
Mn = 1200
OCF2CF2OCF2CF2OCF2CF2O
OCF2OCF2CF2OCF2CF2O
OCF2OCF2CF2OCF2O
OCF2OCF2CF2OCF2CF2O
OCF2CF2OCF2CH2OH
OCF2OCF2CH2OH
OCF2CF2OCF2OCF2CF2O
OCF2OCF2OCF2CF2O
OCF2OCF2OCF2O
-OCF2-
-OCF2CF2O-

FIG. 2

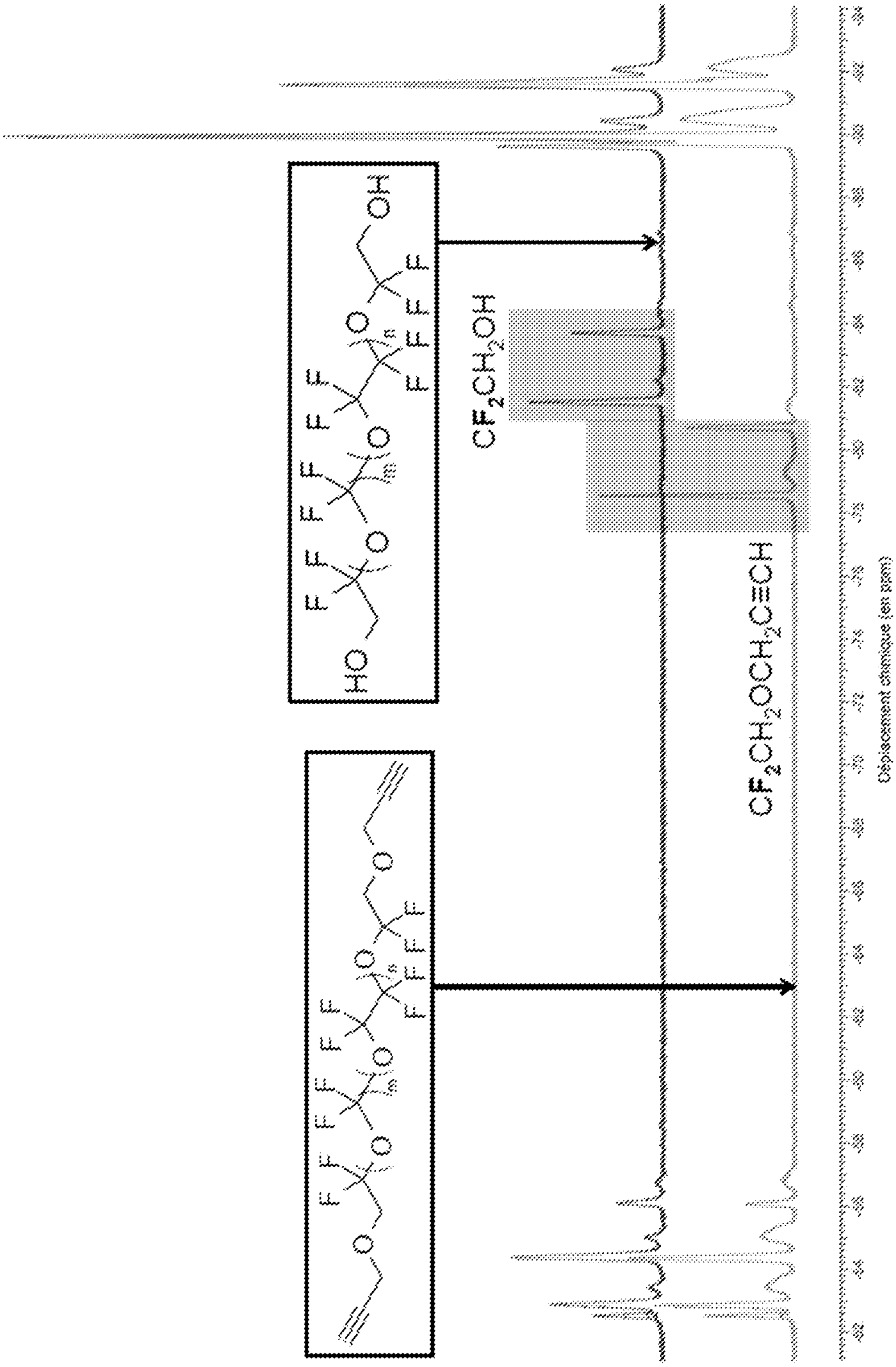
$CF_2CH_2OH$
$CF_2CH_2OCH_2C{\equiv}CH$
Déplacement chimique (en ppm)

FIG. 3

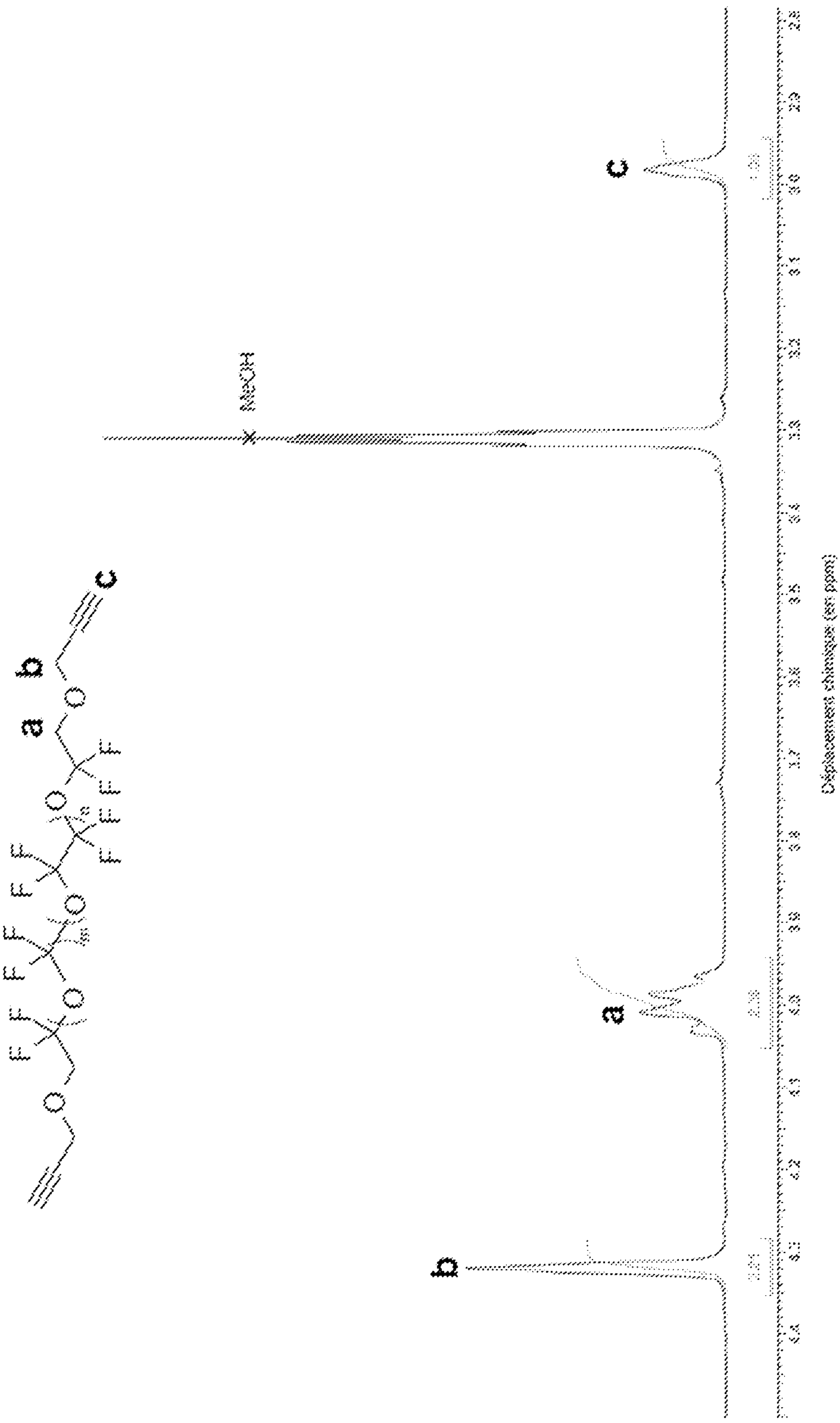

FIG. 4

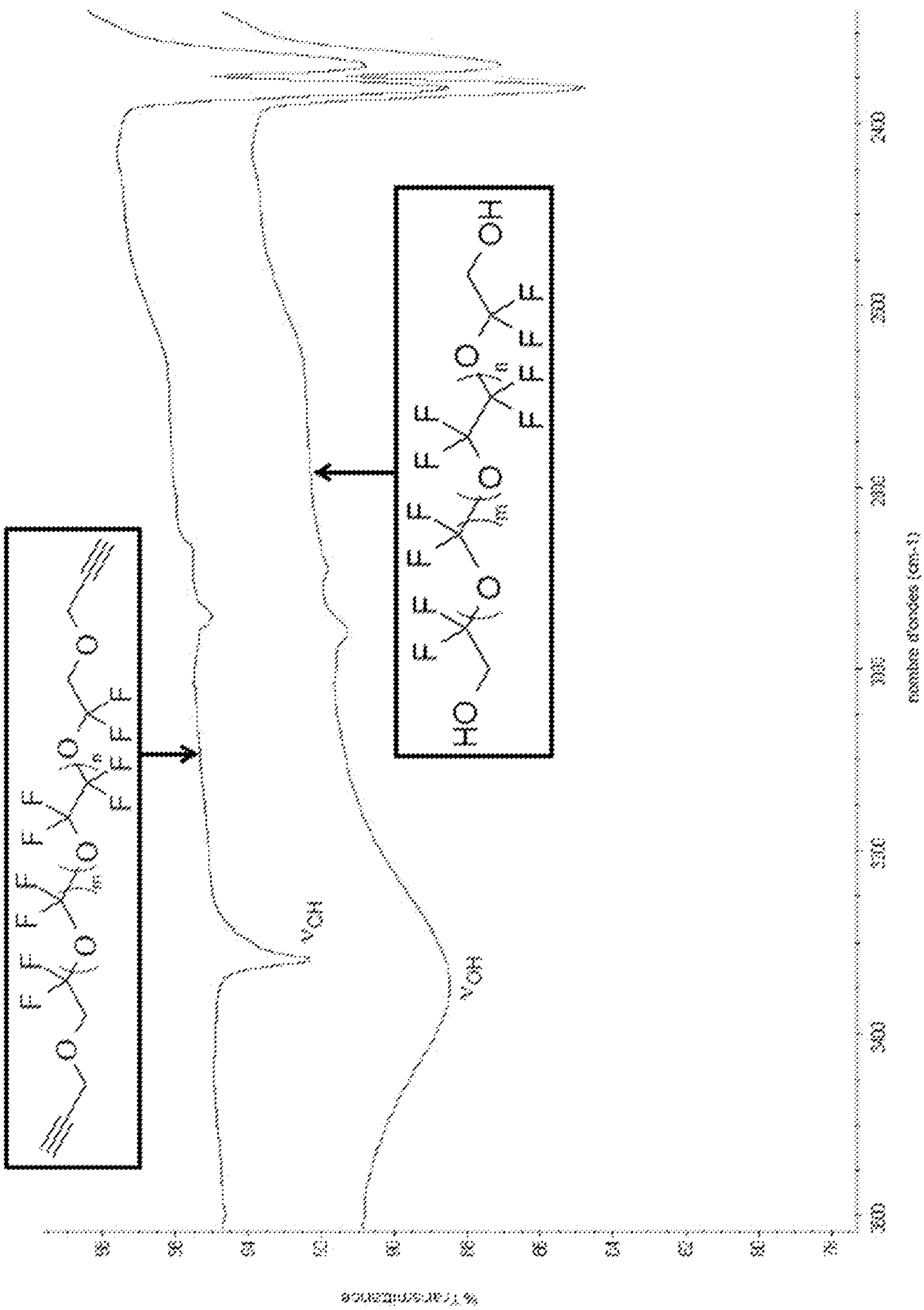

FIG. 5

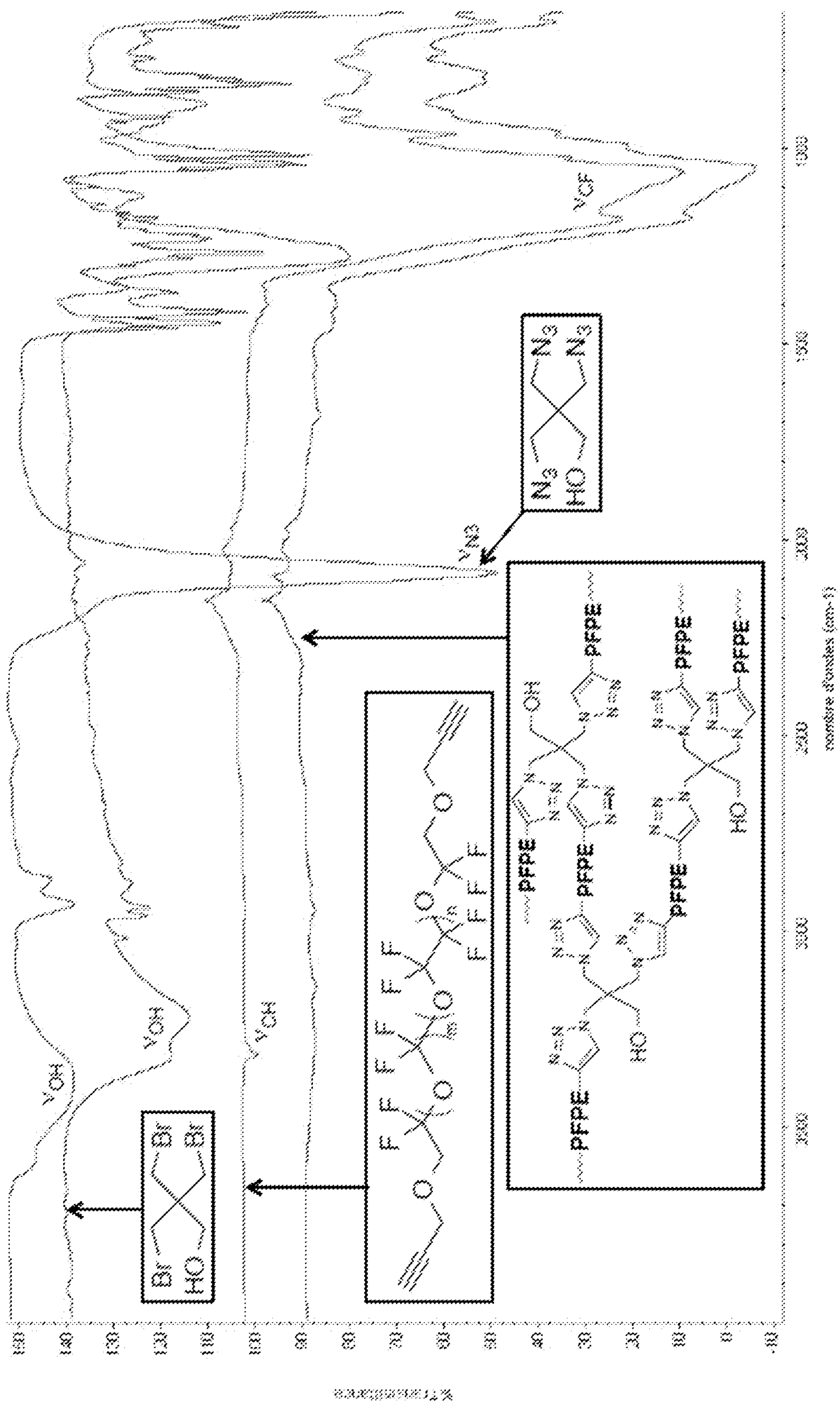

FIG. 6

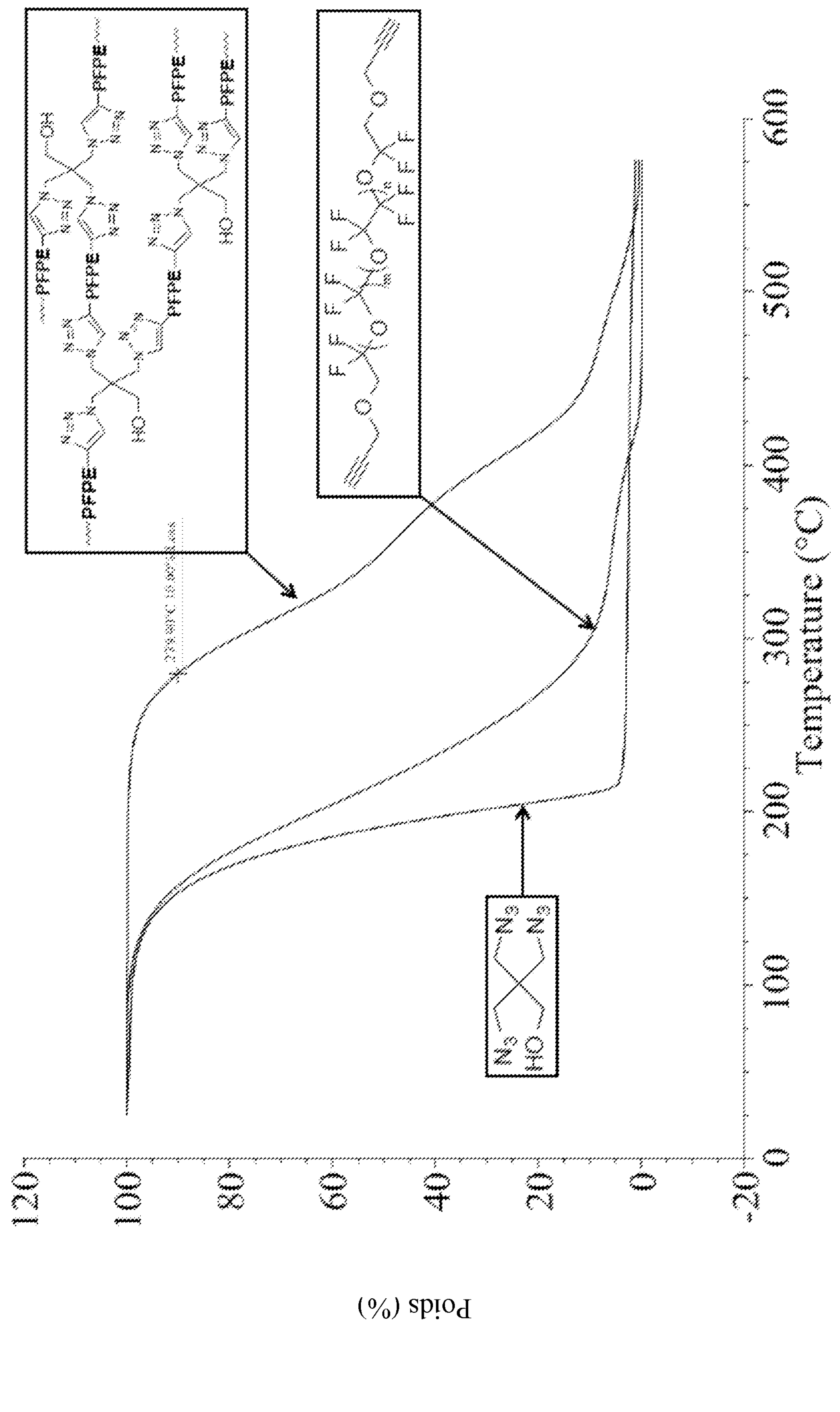
Temperature (°C)
Poids (%)
0
100
200
300
400
500
600
120
100
80
60
40
20
0
-20

FIG. 7

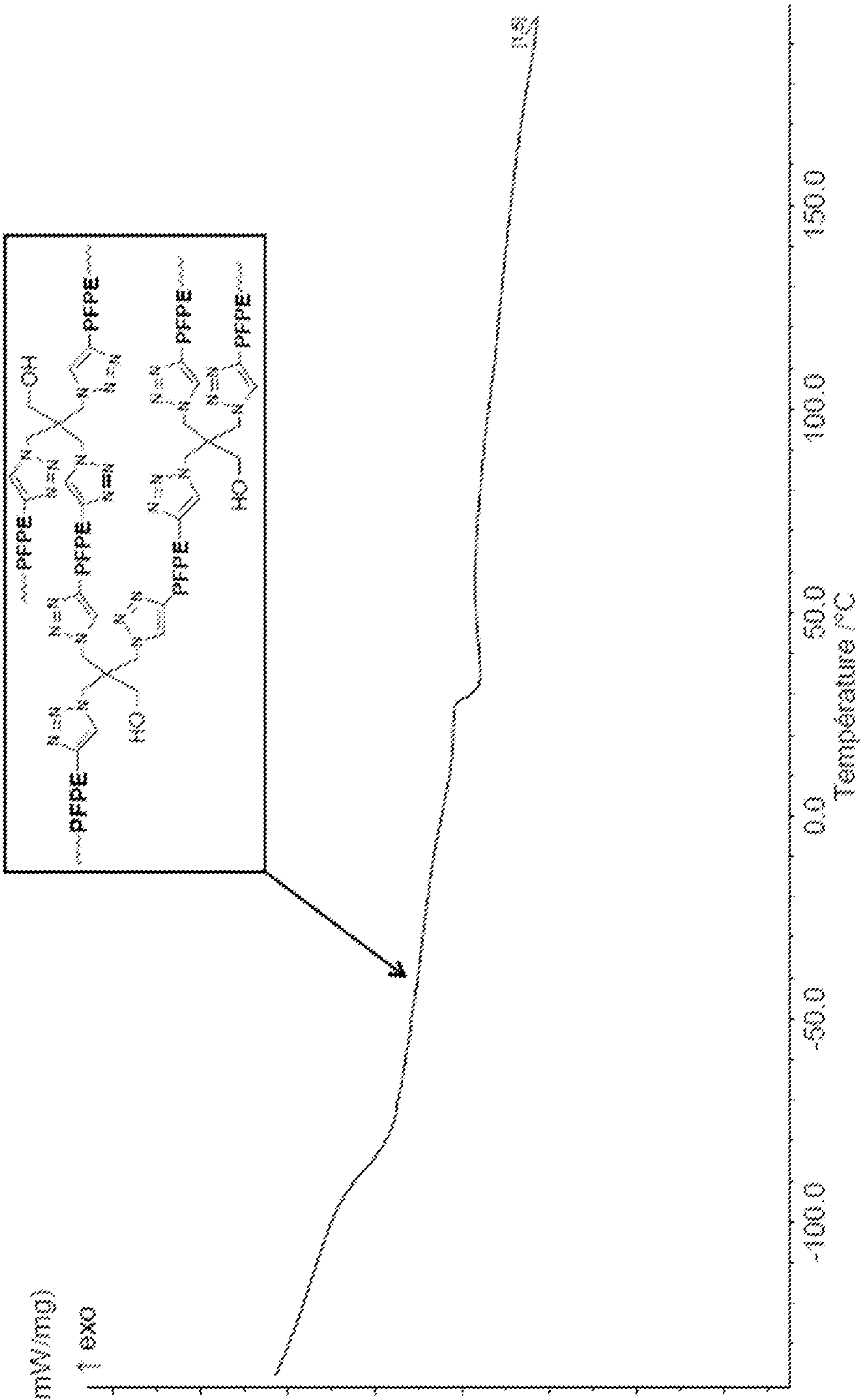
DSC /(mW/mg)
↑ exo
-0.05
-0.1
-0.15
-0.2
-0.25
-0.3
-0.35
-0.4
-100.0
-50.0
0.0
50.0
100.0
150.0
Température /°C

FIG. 8

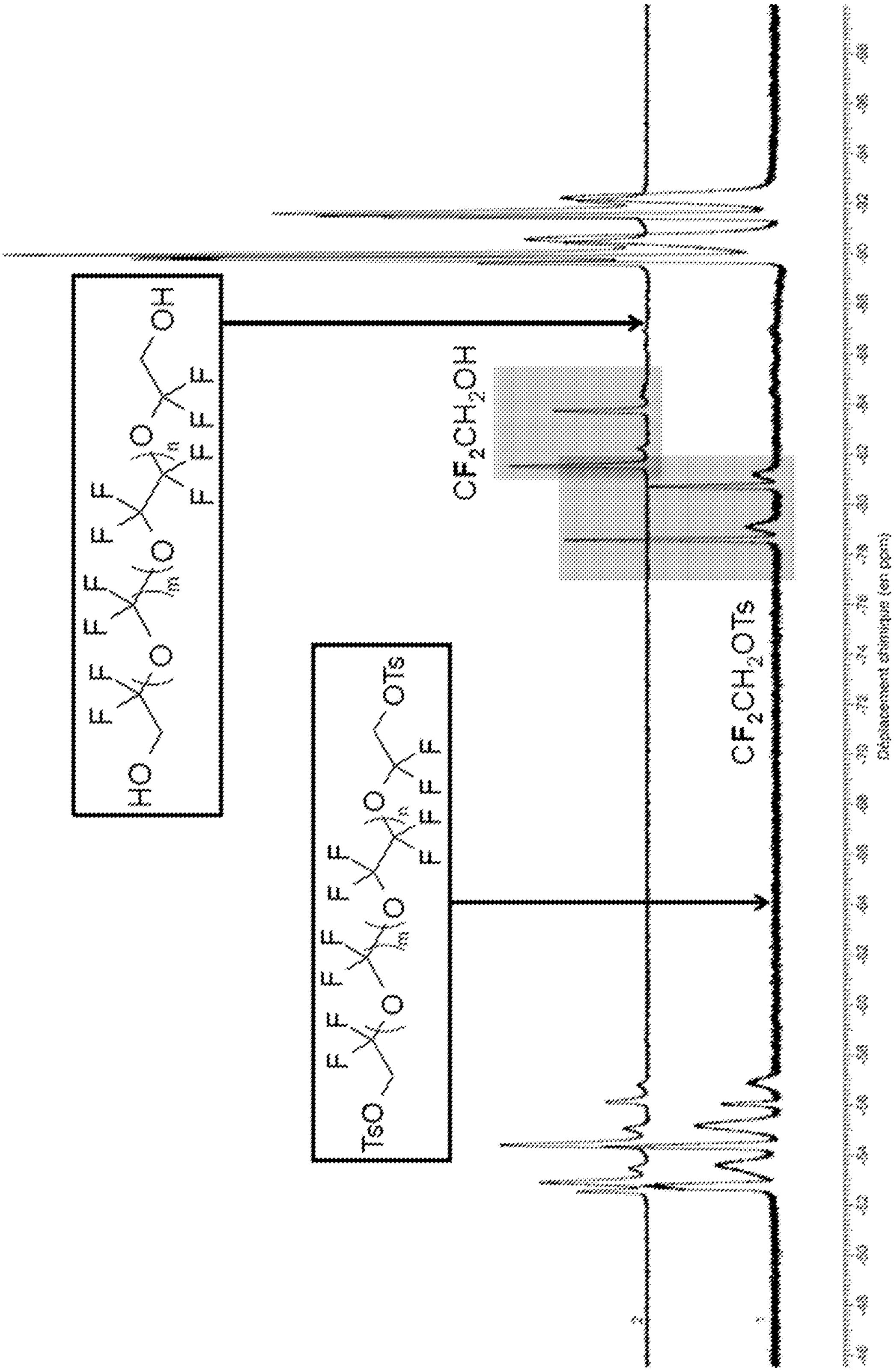

**FIG. 9**

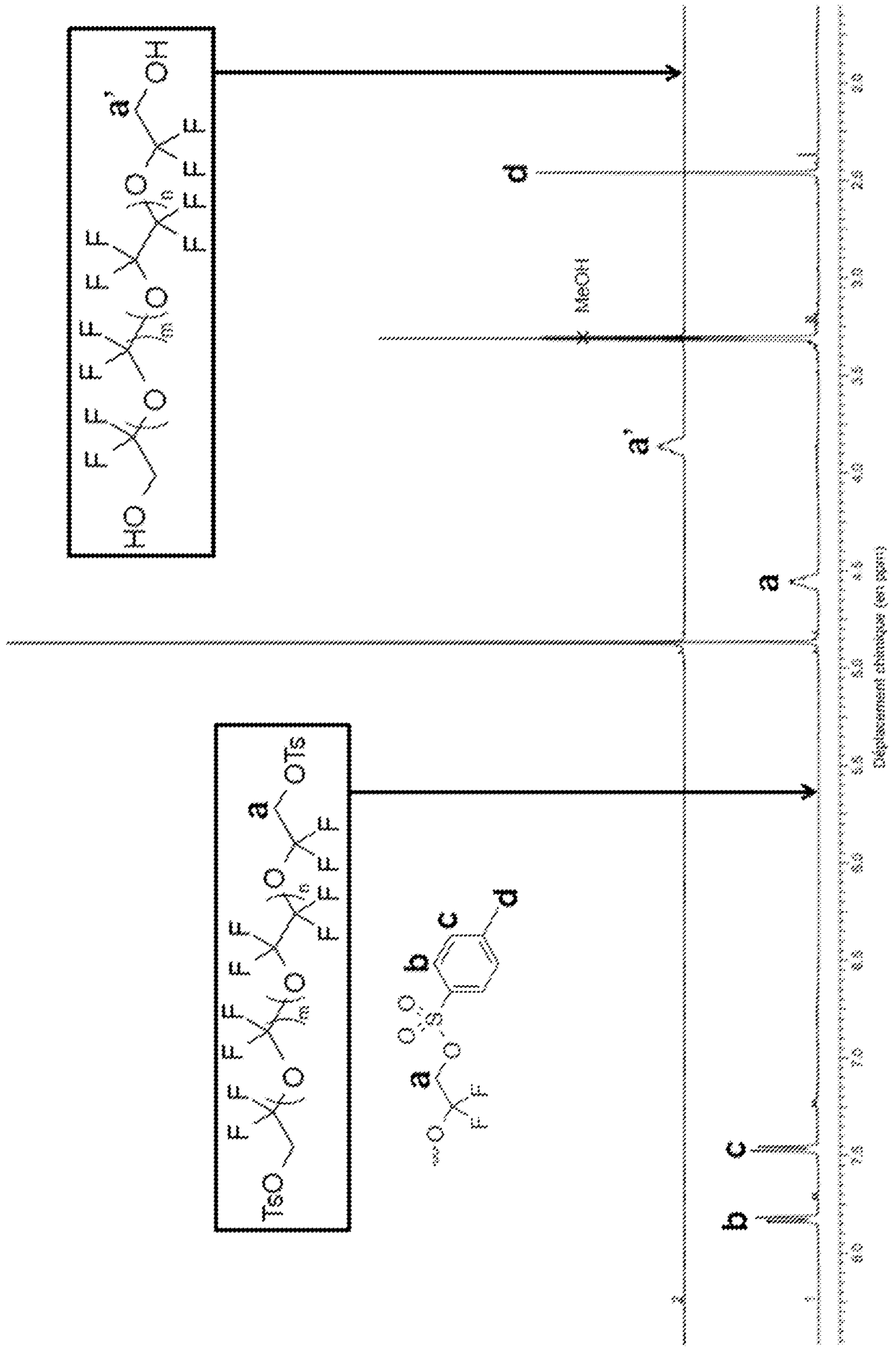

FIG. 10

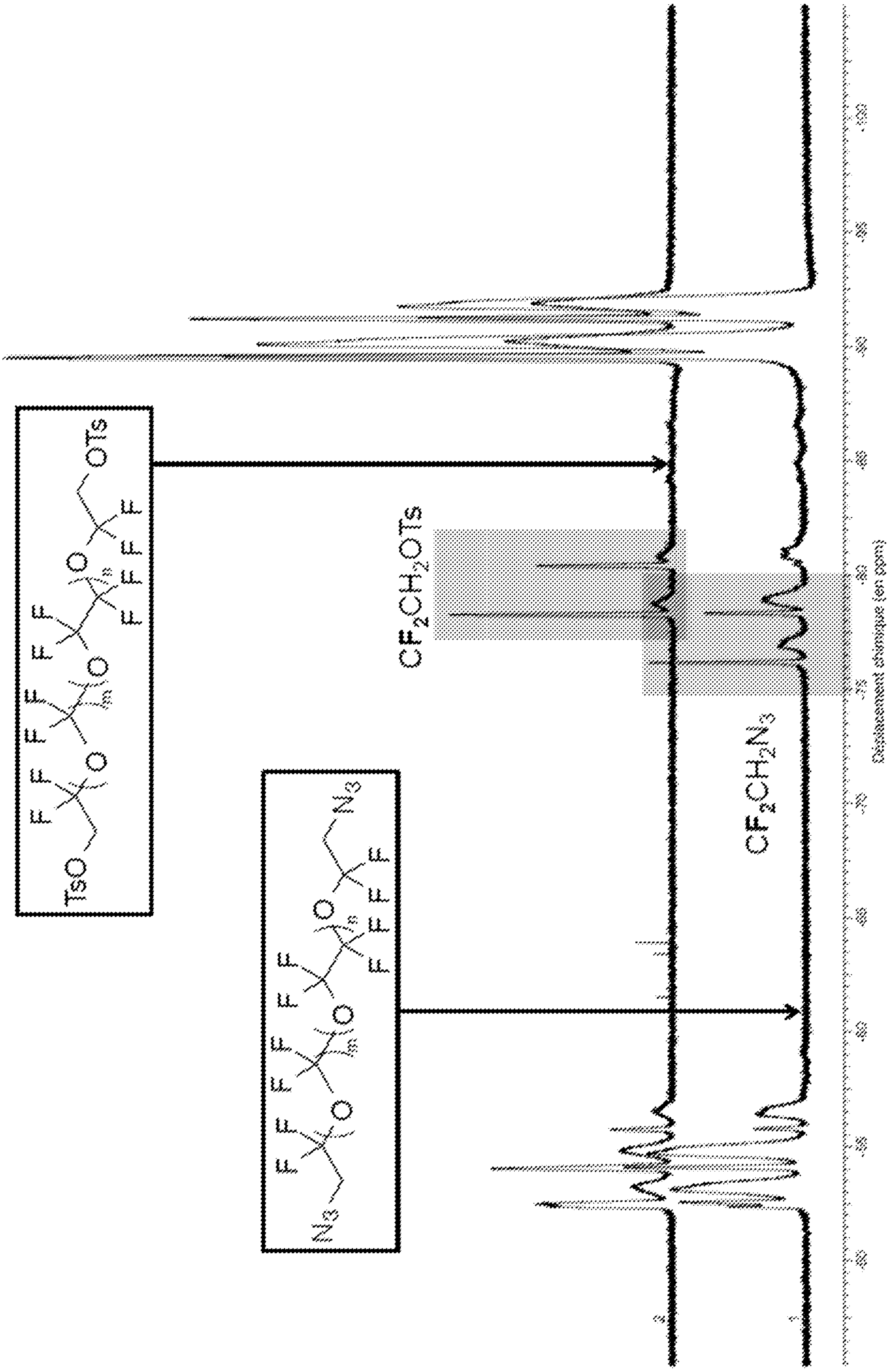

FIG. 11

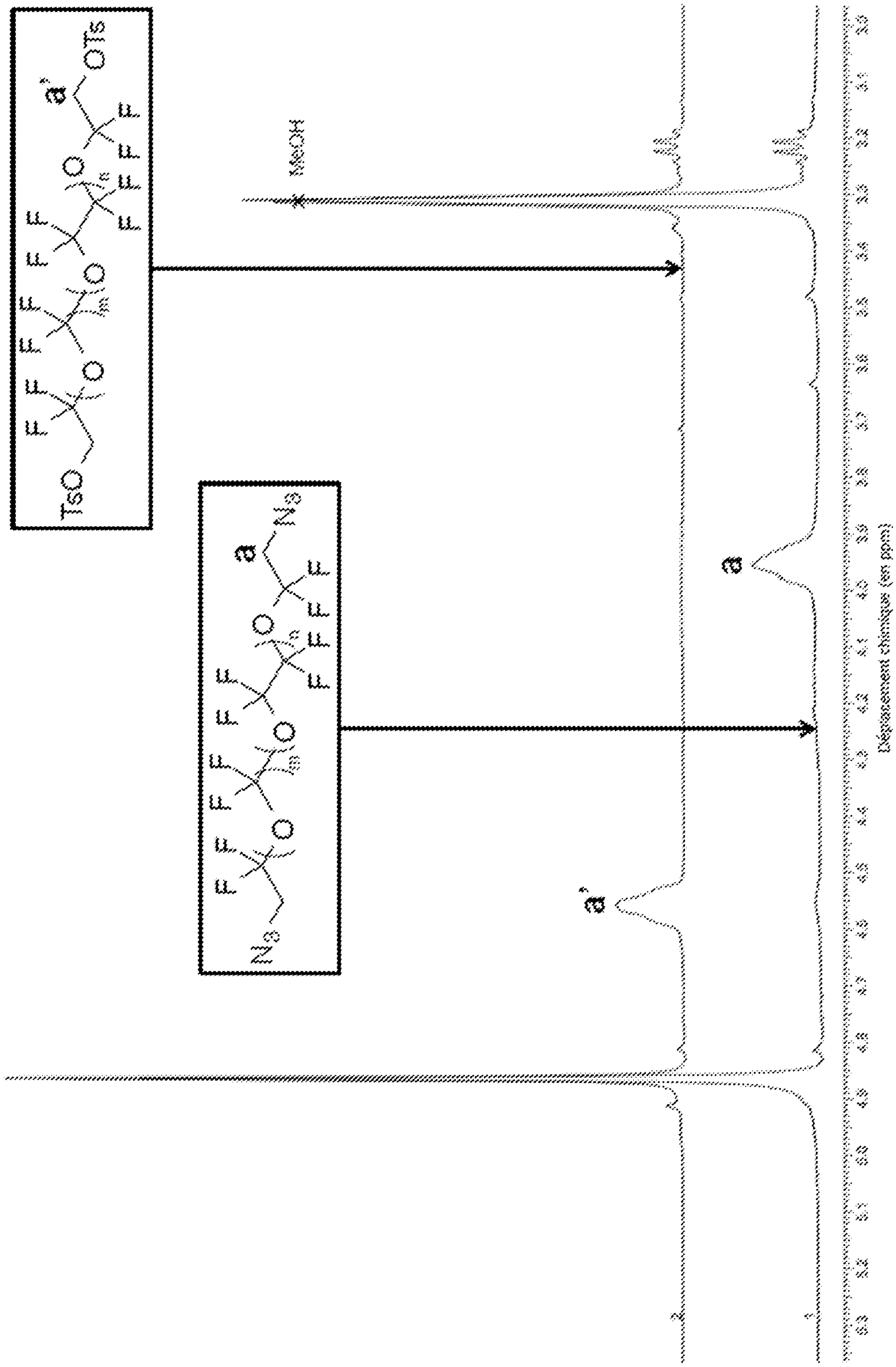

FIG. 12

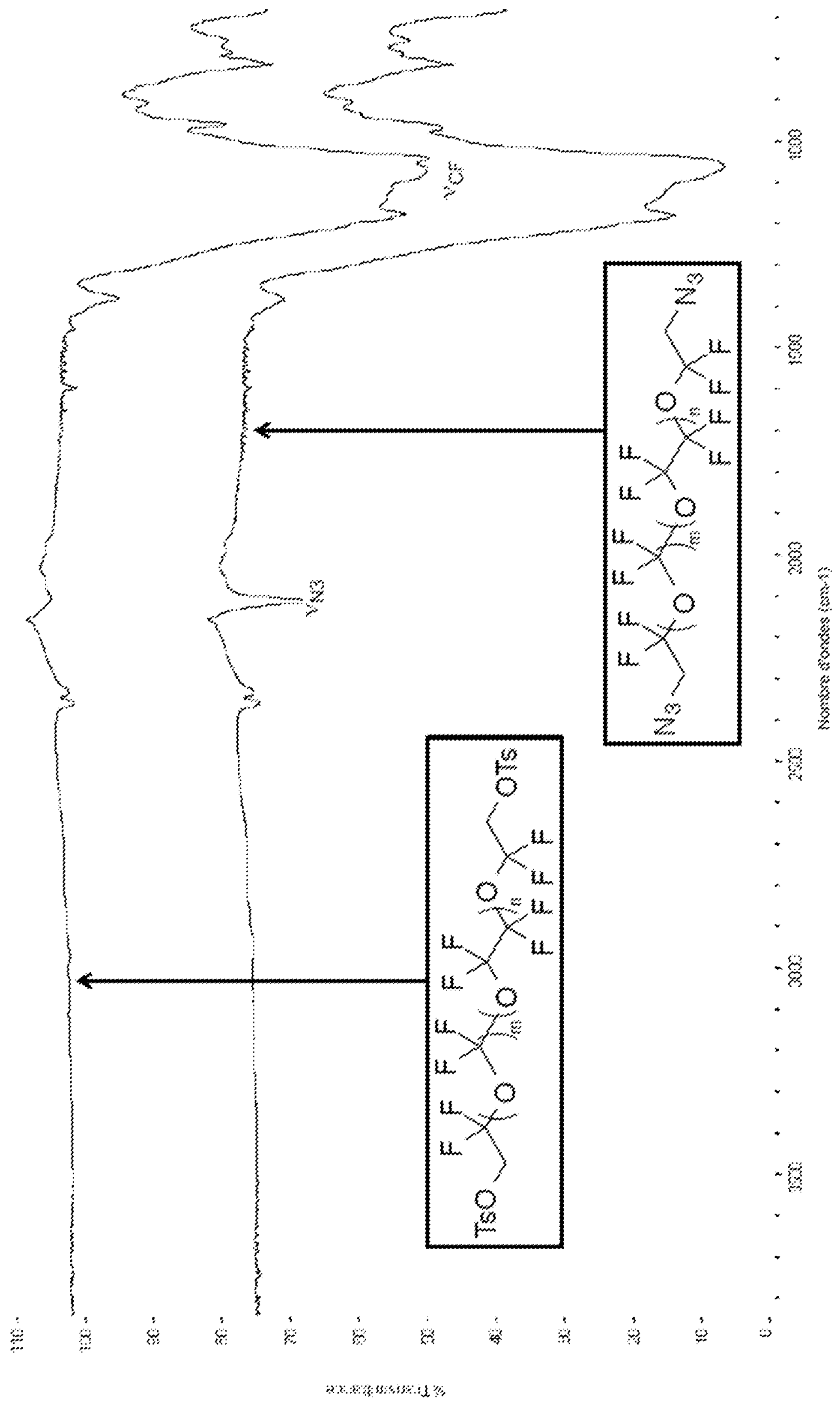

FIG. 13

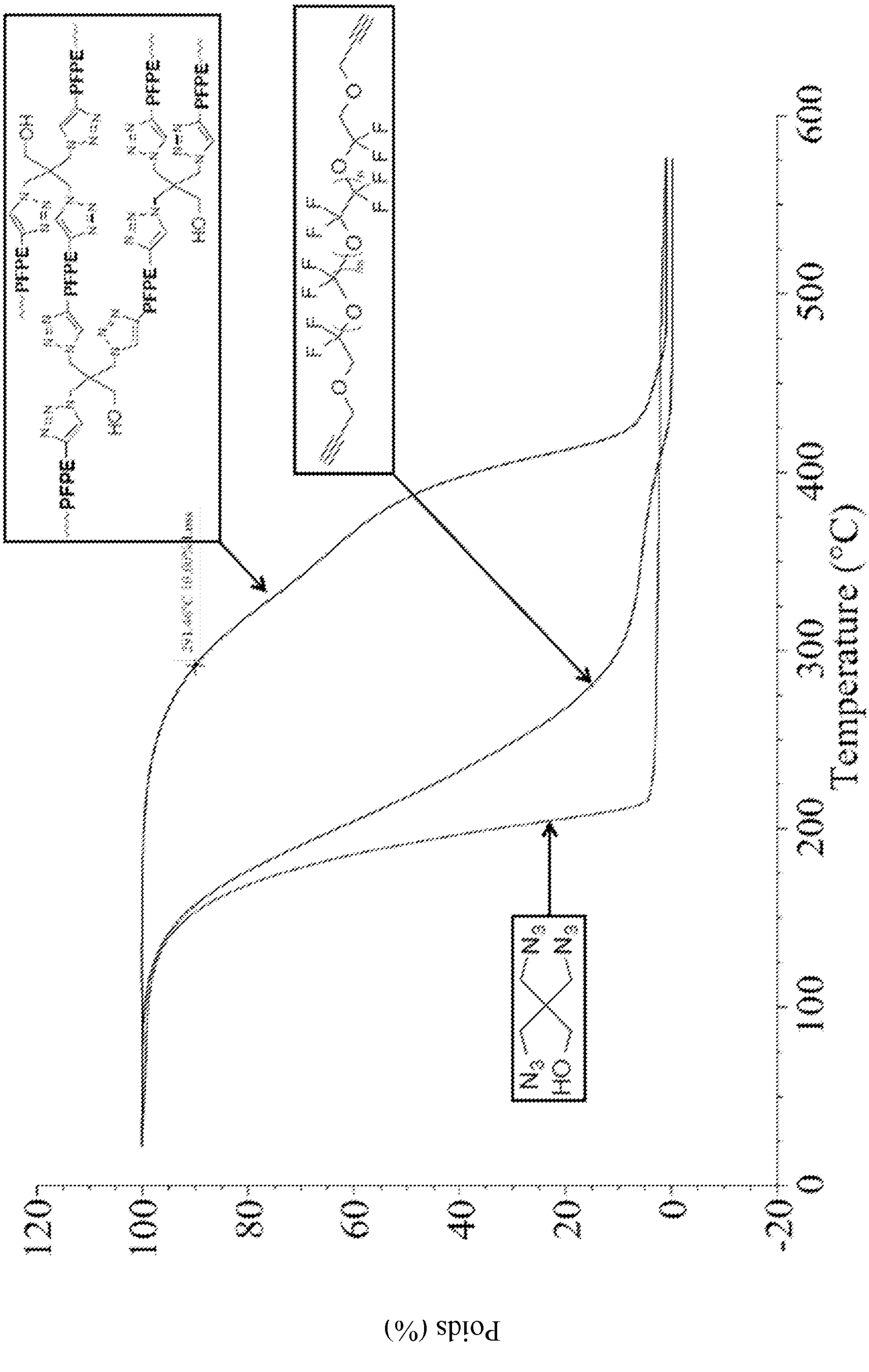
Temperature (°C)
Poids (%)
0
100
200
300
400
500
600
120
100
80
60
40
20
0
-20

FIG. 14

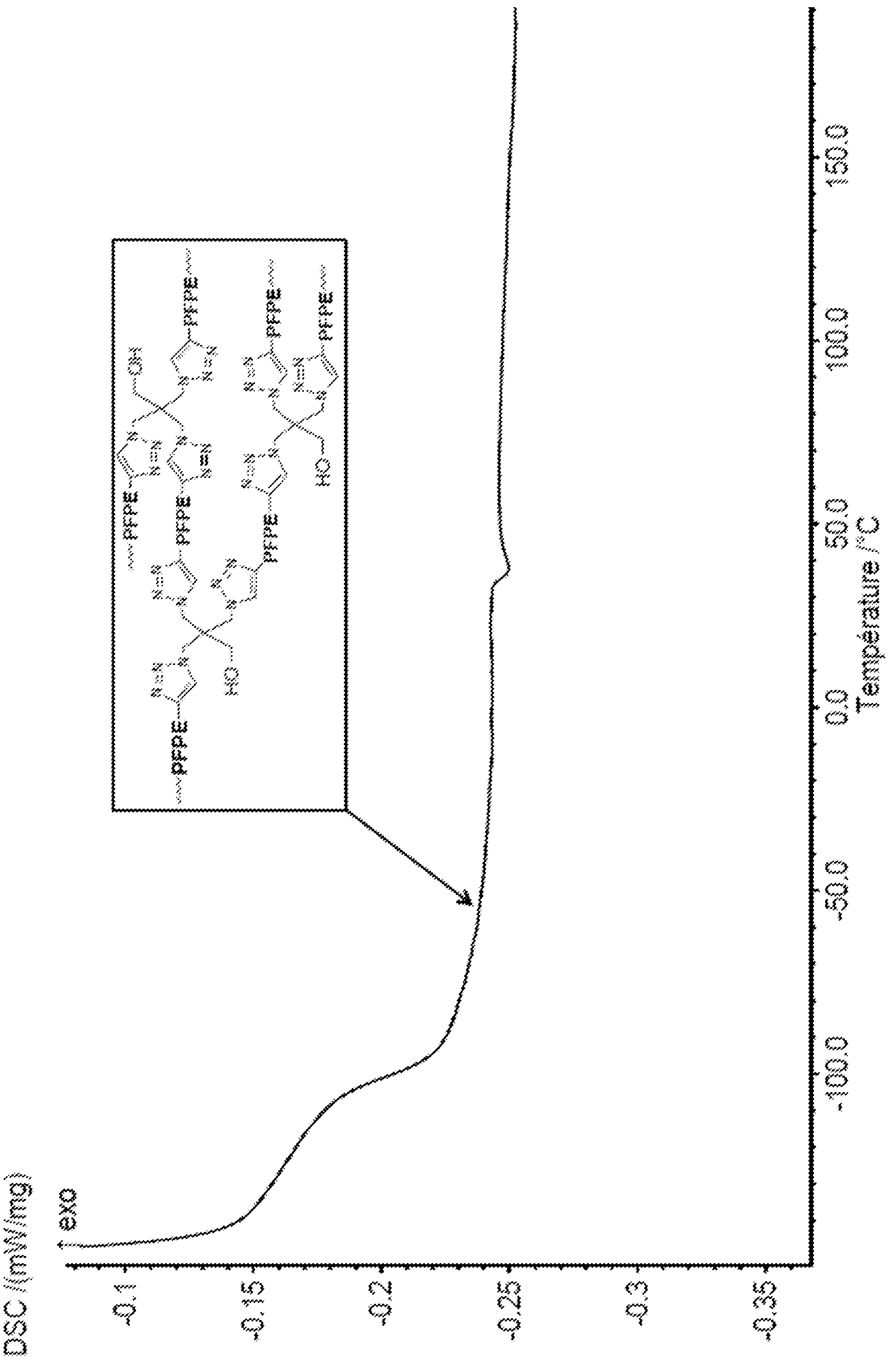
DSC /(mW/mg)
exo
-0,1
-0,15
-0,2
-0,25
-0,3
-0,35
-100,0
-50,0
0,0
50,0
100,0
150,0
Température /°C

FIG. 15

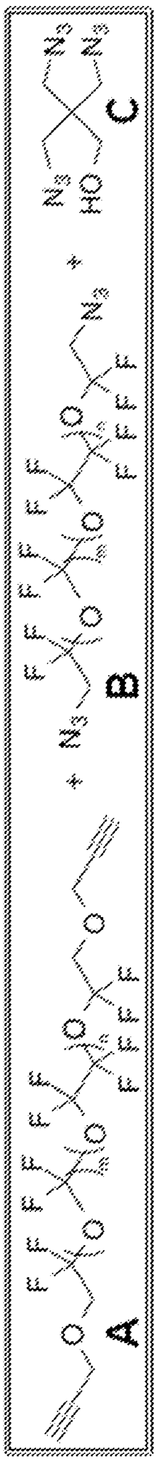

A

B

C

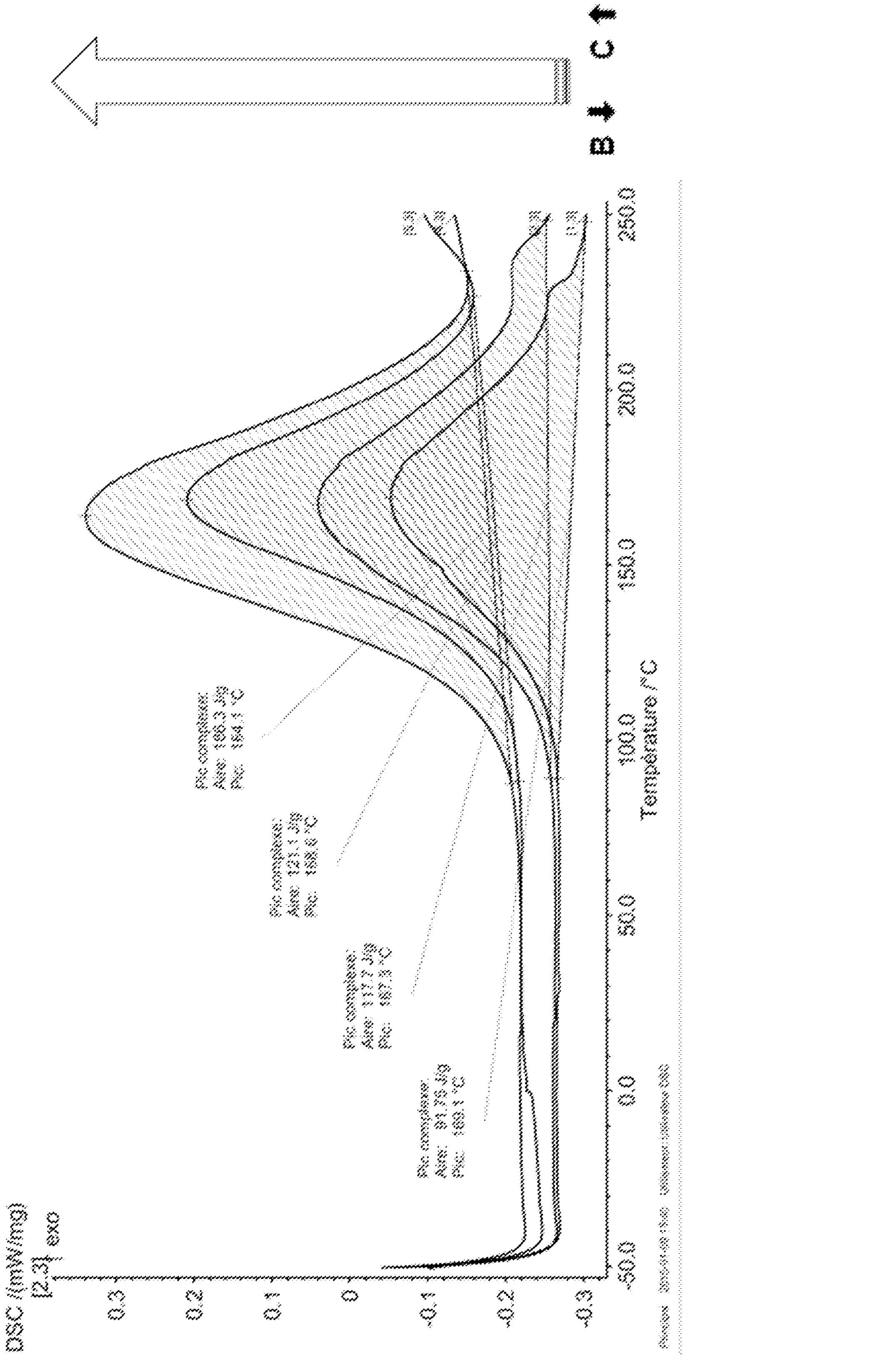
DSC /(mW/mg)
[2.3] exo
0.3
0.2
0.1
0
-0.1
-0.2
-0.3
-50.0
0.0
50.0
100.0
150.0
200.0
250.0
Température /°C
B
C

FIG. 16

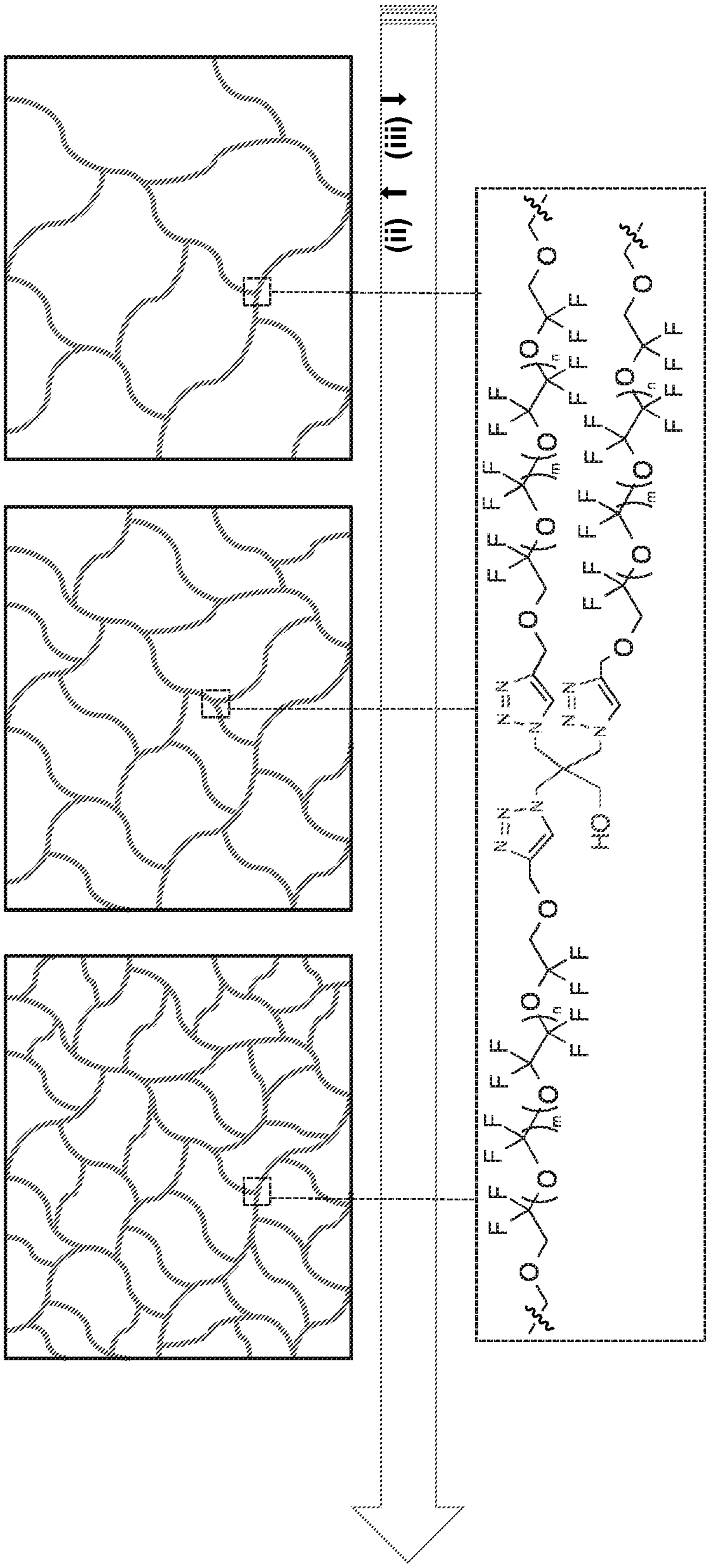

FIG. 17

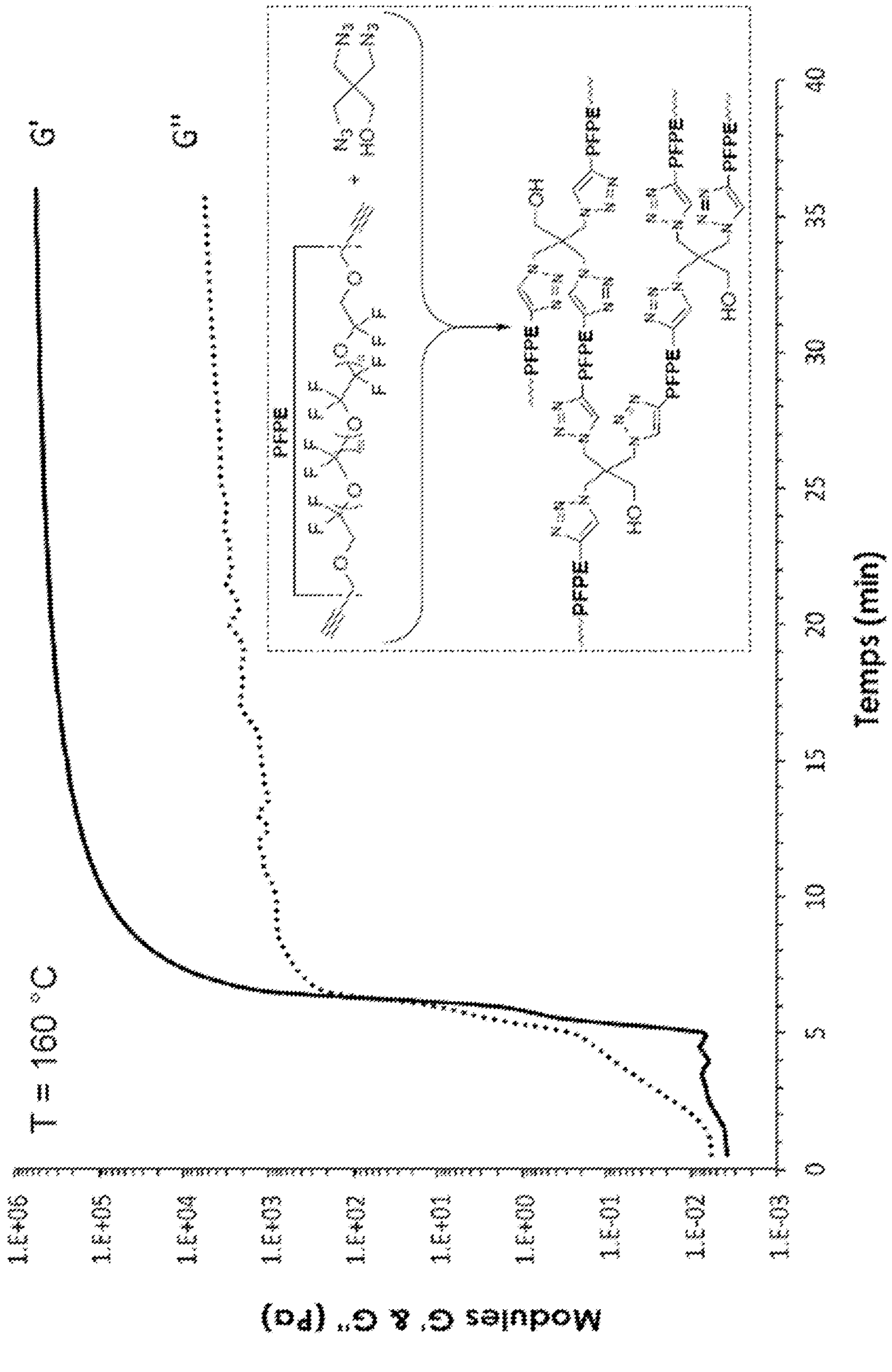
T = 160 °C
G'
G''
PFPE
Temps (min)
Modules G' & G'' (Pa)
1.E+06
1.E+05
1.E+04
1.E+03
1.E+02
1.E+01
1.E+00
1.E-01
1.E-02
1.E-03
0
5
10
15
20
25
30
35
40

FIG. 18

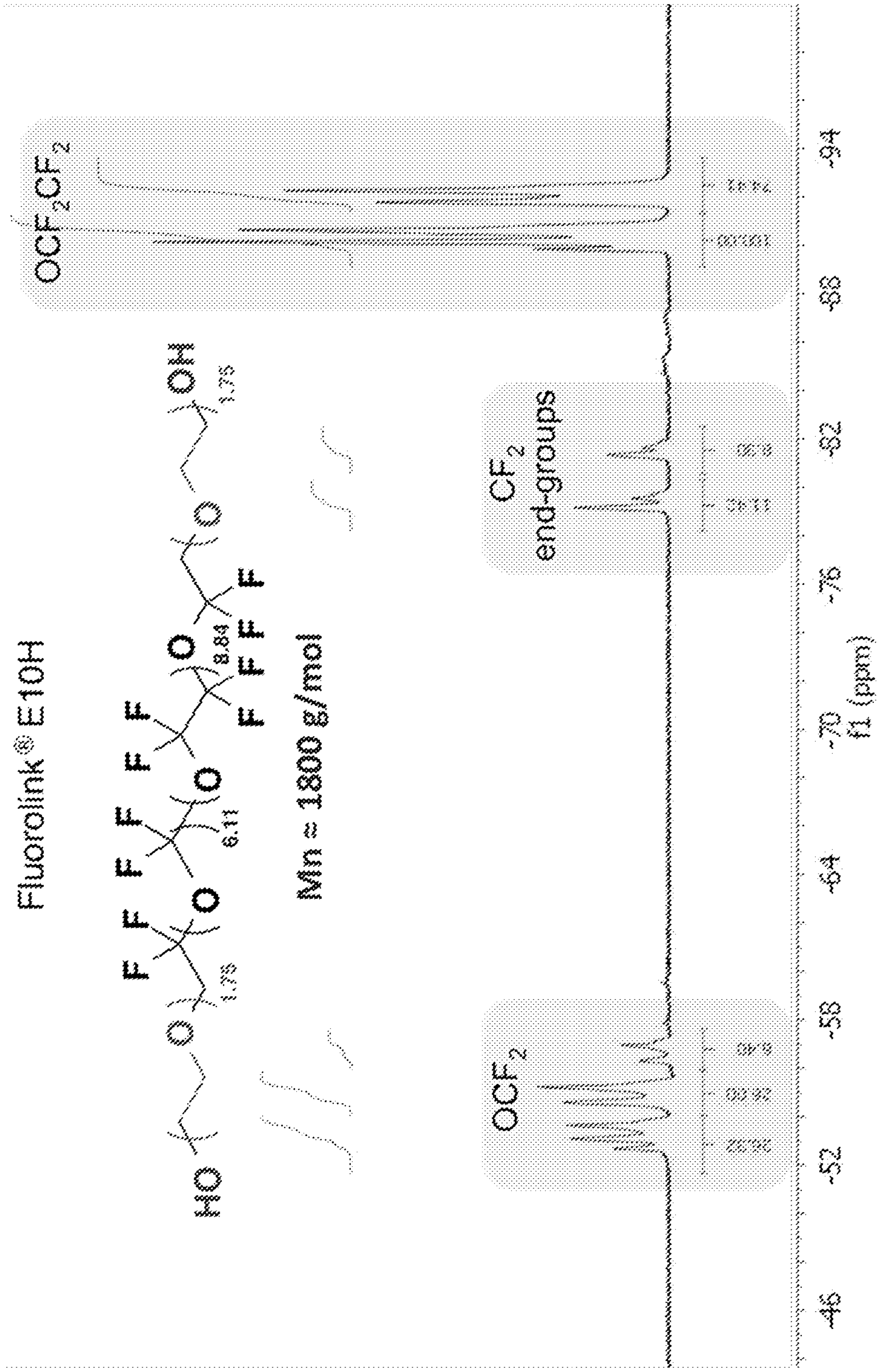
Fluorolink® E10H
Mn ≈ 1800 g/mol
OCF2CF2
CF2
end-groups
OCF2
-46
-52
-58
-64
-70
-76
-82
-88
-94
f1 (ppm)

FIG. 19

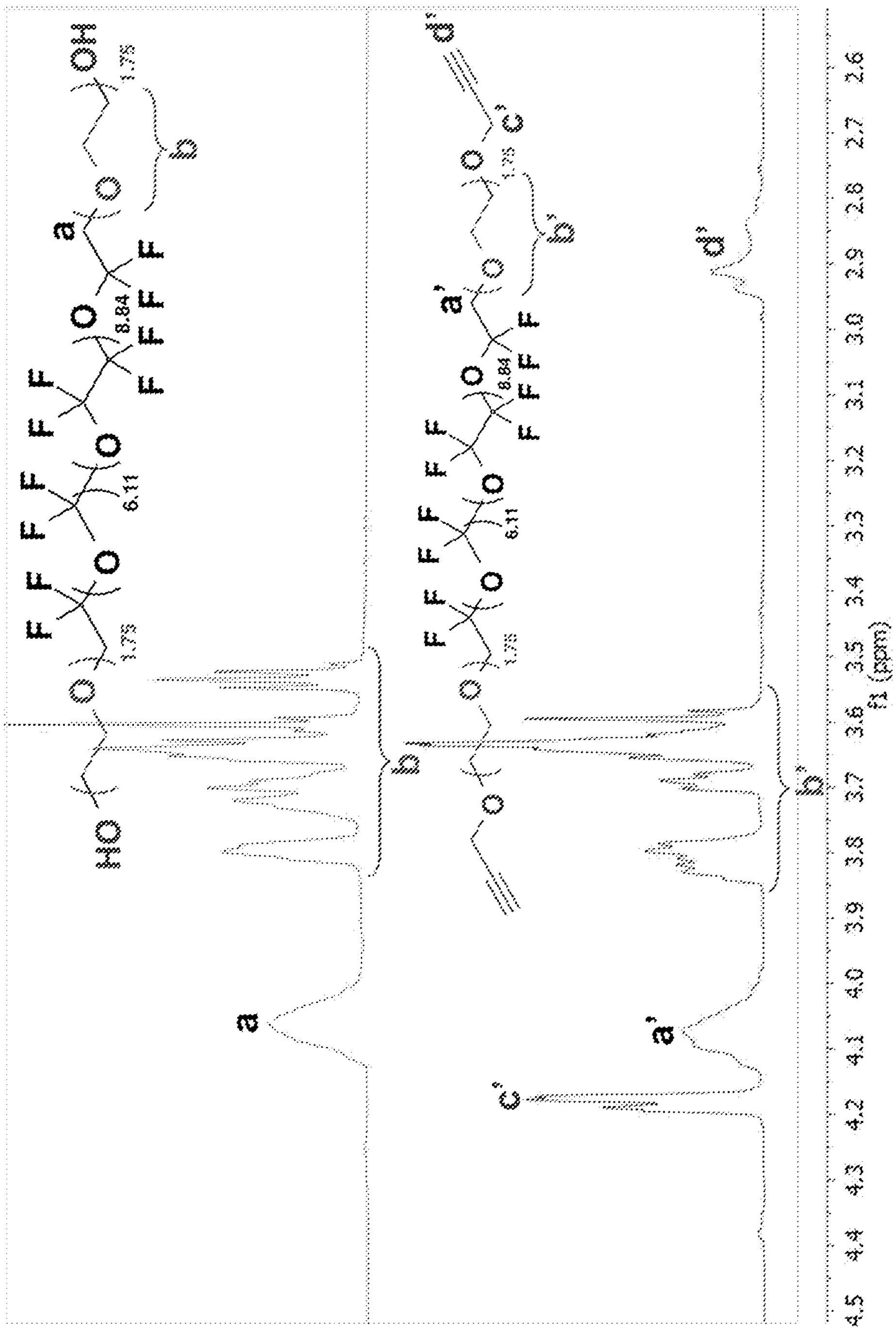

# FIG. 20

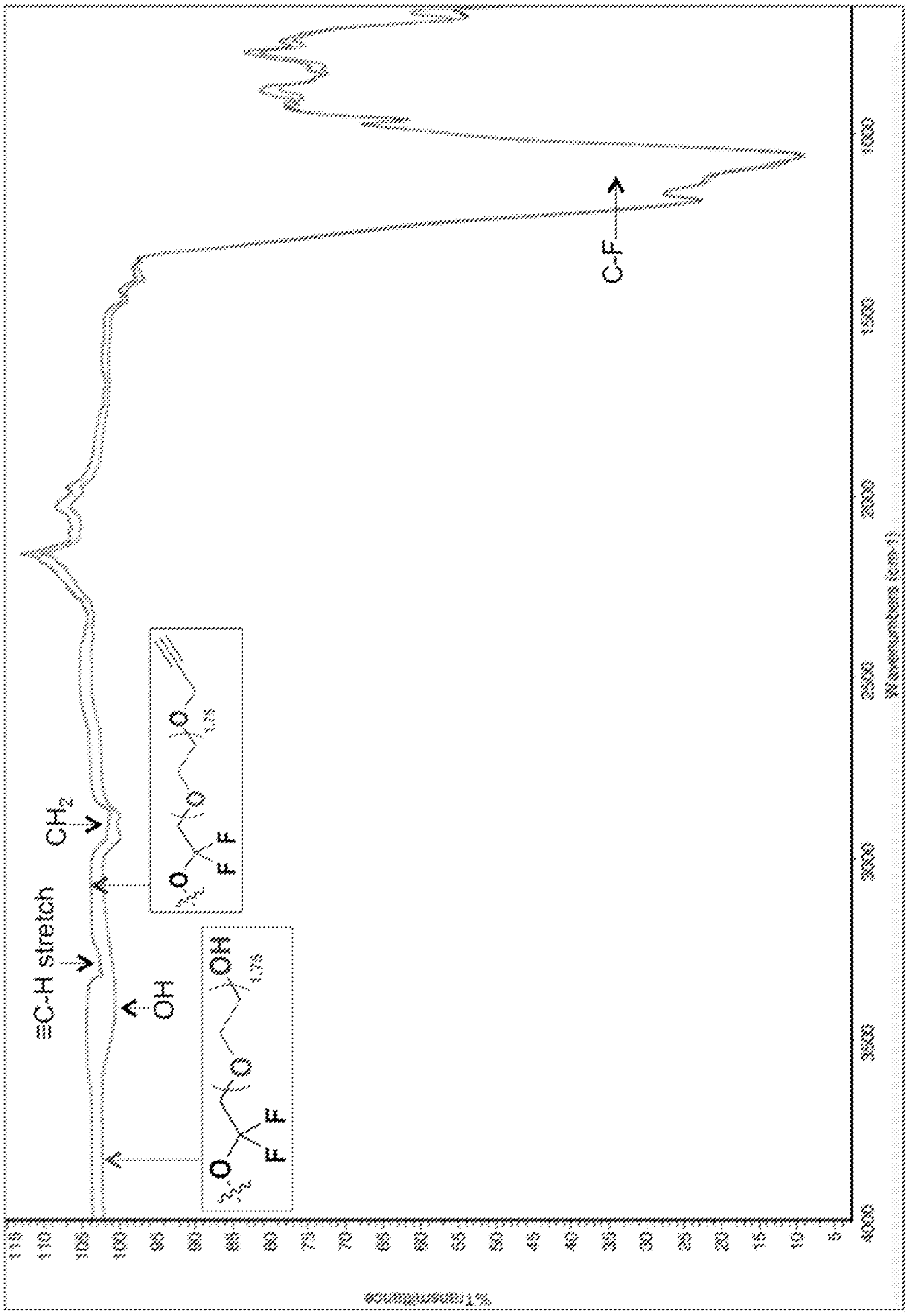

**FIG. 21**

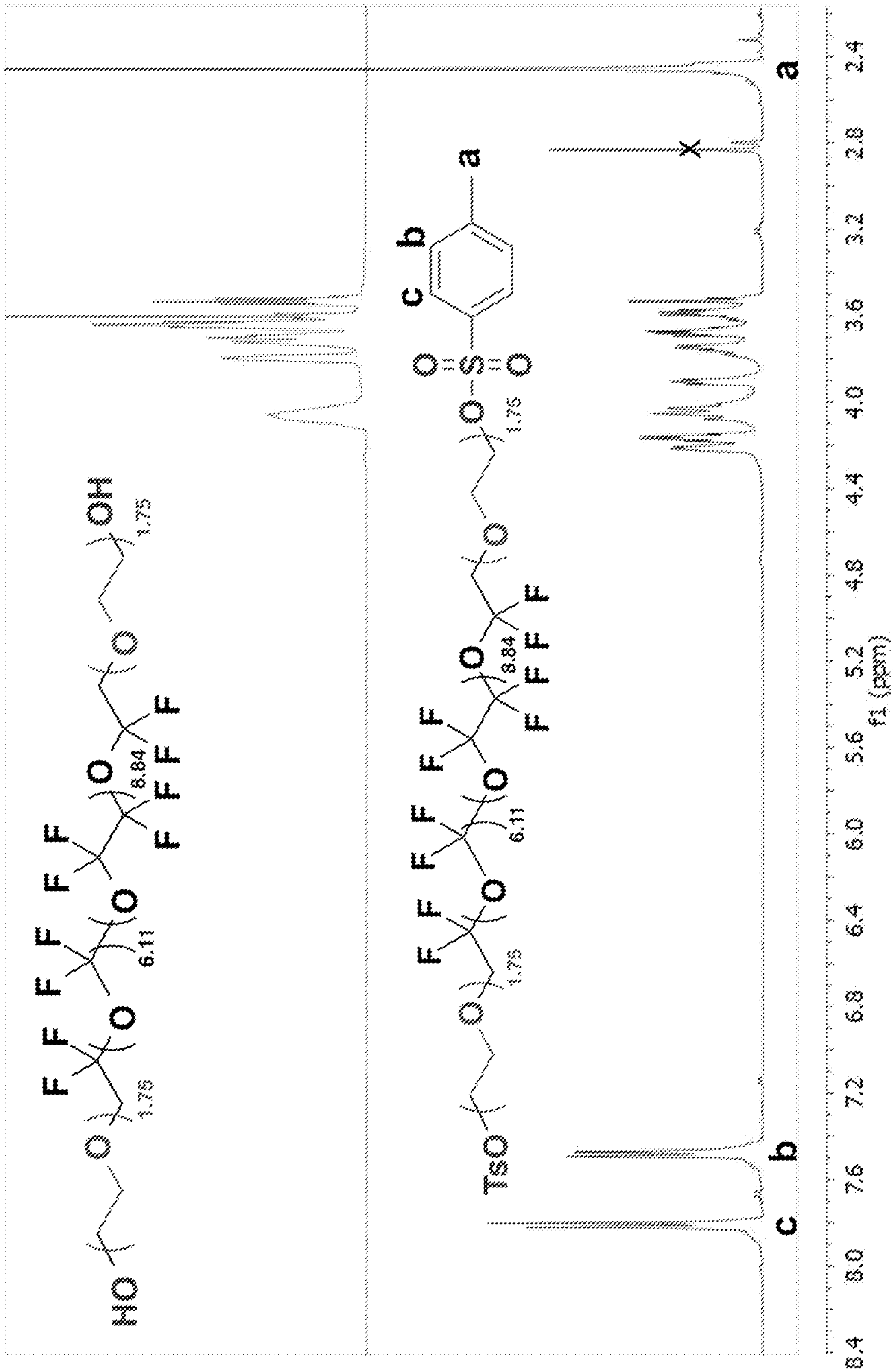

FIG. 22

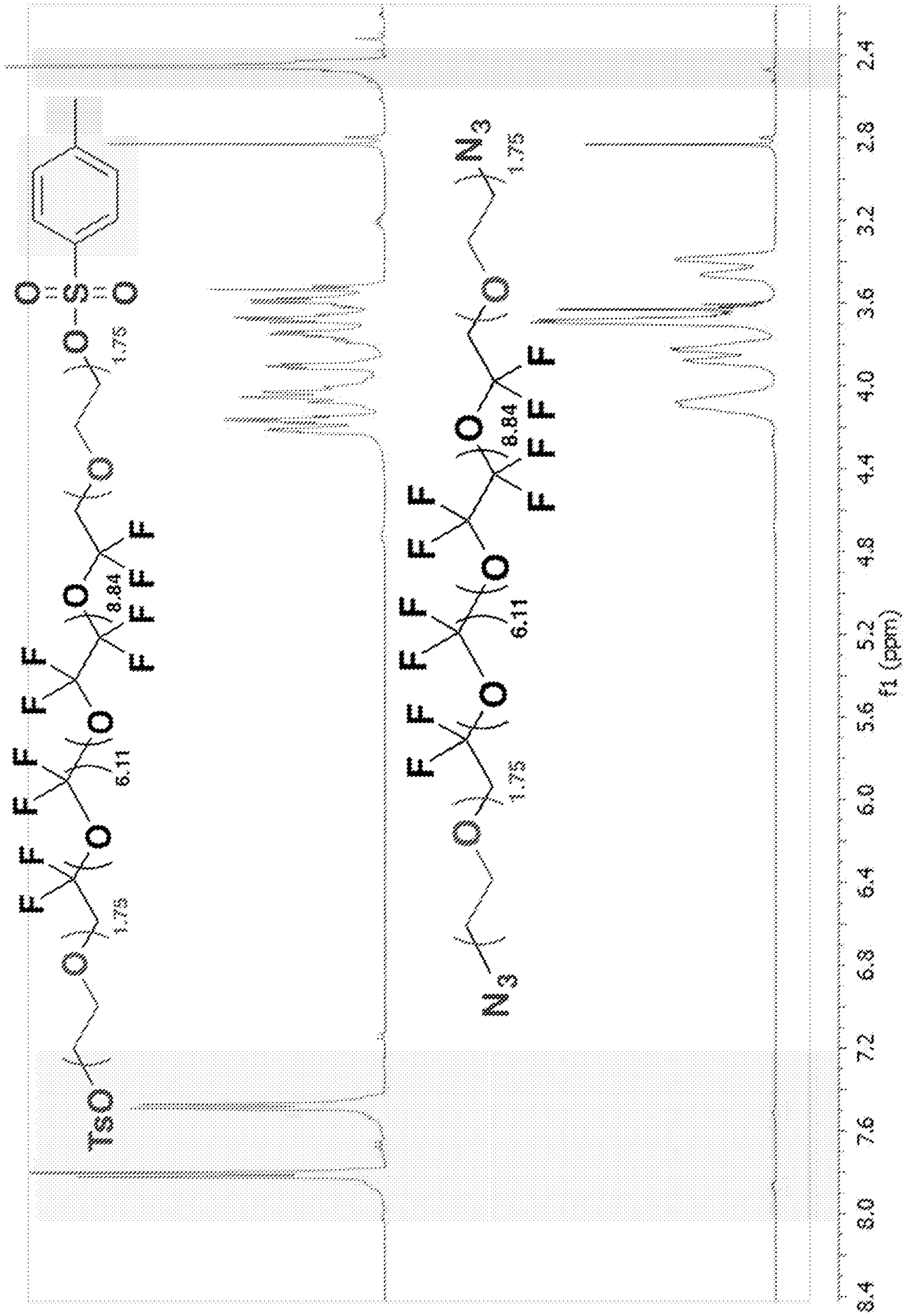

FIG. 23

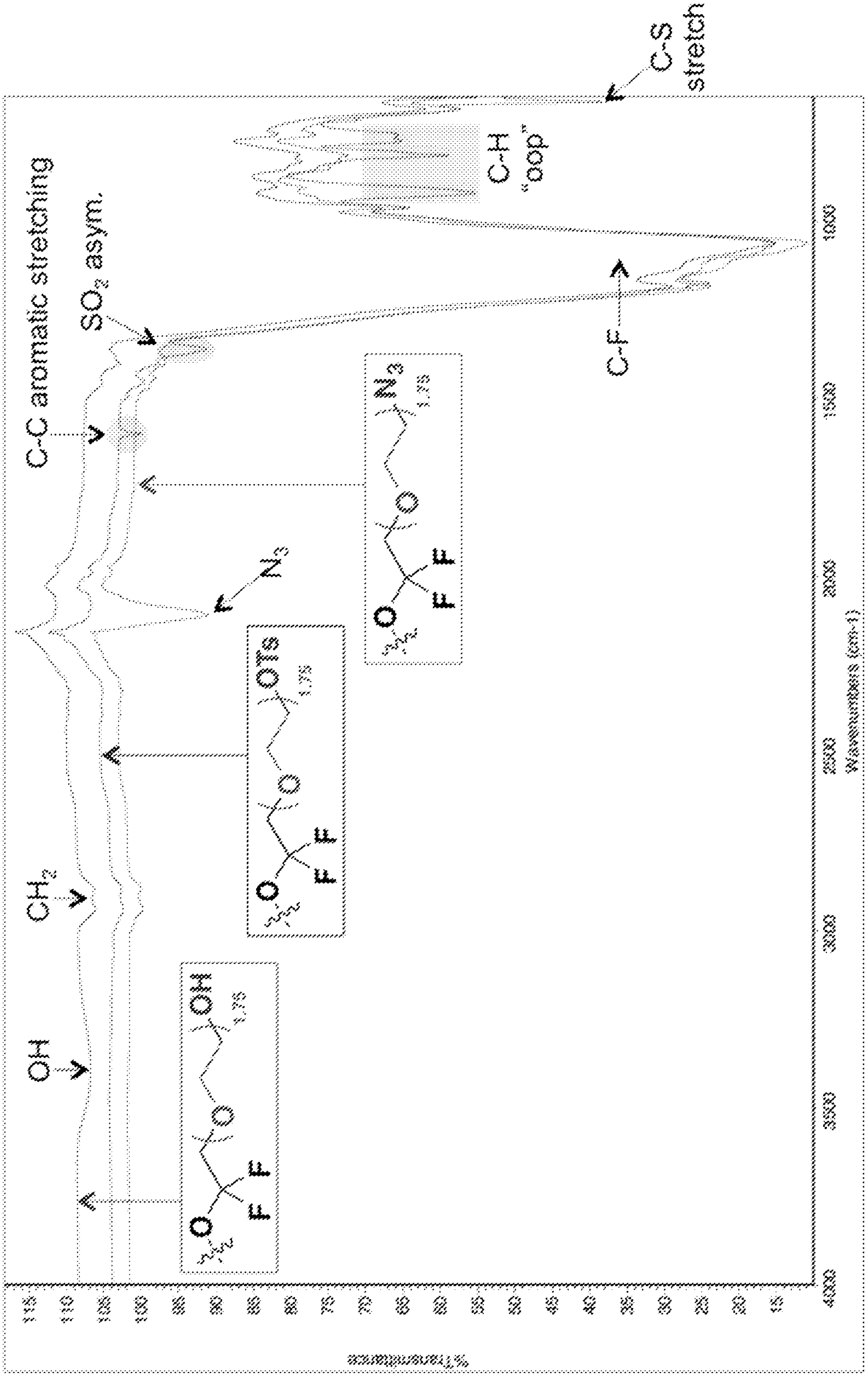
C-C aromatic stretching
SO2 asym.
N3
CH2
OH
C-H "oop"
C-F
C-S stretch
Wavenumbers (cm-1)
%Transmittance

FIG. 24

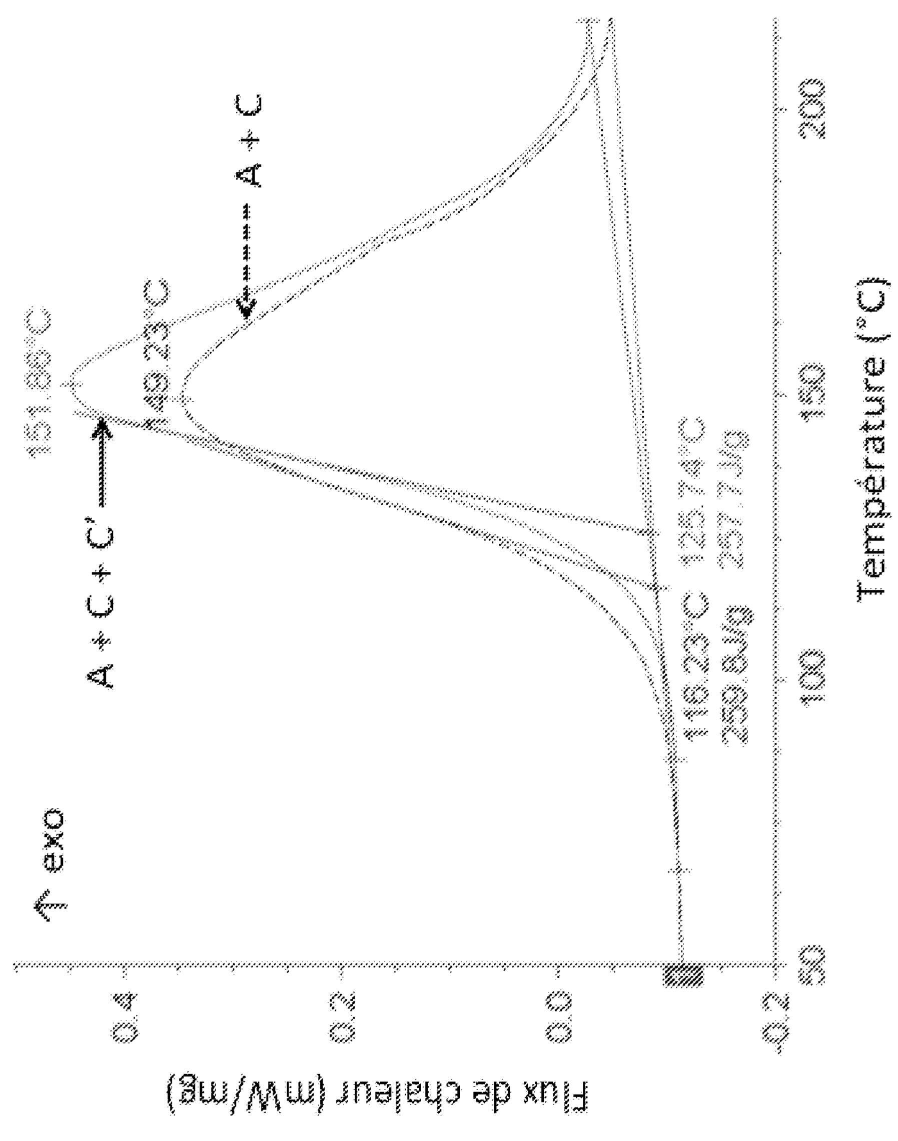
↑ exo
A + C + C'
A + C
151.86°C
149.23°C
116.23°C
259.8J/g
125.74°C
257.7J/g
Température (°C)
Flux de chaleur (mW/mg)
0.4
0.2
0.0
-0.2
50
100
150
200

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2014055406 A
- WO 2010115855 A
- US 2010324234 A
- WO 2012131278 A


### Littérature non-brevet citée dans la description


- **YANG et al.** *Journal of Materials Chemistry,* Novembre 2011, vol. 22 (3
- **KARIS et al.** *J. Fluorine Chem.,* 2002, vol. 118, 81-94
- **YANG et al.** *J. Mater. Chem.,* 2012, vol. 22, 1100-1106
- *Chem. Rev.,* 2006, vol. 106, 3936-3962
- *Macromolecules,* 2010, vol. 43, 3652-3663
- *Macromolecules,* 2012, vol. 45, 7375-7387
- *Dalton Trans.,* 2012, vol. 41, 4335
- *Biomaterials,* 2014, vol. 35, 2322
- *Chem. Commun.,* 2007, 380